(19) Europäisches Patentamt
European Patent Office
Office européen des brevets

(11) EP 4 810 052 A2

(12) EUROPEAN PATENT APPLICATION

(43) Date of publication:
**23.09.2026 Bulletin 2026/39**

(21) Application number: **26195541.3**

(22) Date of filing: **15.11.2024**

(51) International Patent Classification (IPC):
***A61K 38/00*** *(2006.01)*

(52) Cooperative Patent Classification (CPC):
**C07K 14/72;** A61K 38/00

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **17.11.2023 EP 23210715**

(62) Document number(s) of the earlier application(s) in accordance with Art. 76 EPC:
**24808348.7 / 4 788 699**

(71) Applicant: **Gubra A/S**
**2970 Hørsholm (DK)**

(72) Inventors:
- **HJØRRINGGAARD, Claudia**
  **DK-2970 Hørsholm (DK)**
- **Nielsen, Jens Christian**
  **DK-2970 Hørsholm (DK)**
- **Lars, BAUMANN**
  **42096 Wuppertal (DE)**
- **NYGAARD, Mads Mørup**
  **DK-2970 Hørsholm (DK)**
- **Donald, BIERER**
  **42096 Wuppertal (DE)**
- **Norbert, TENNAGELS**
  **42096 Wuppertal (DE)**
- **DALBØGE, Louise Schjellerup**
  **DK-2970 Hørsholm (DK)**

(74) Representative: **COPA Copenhagen Patents**
**Rosenvængets Allé 25**
**2100 Copenhagen Ø (DK)**

Remarks:
•The complete document including Reference Table(s) and the Sequence Listing(s) can be downloaded from the EPO website
•This application was filed on 30-07-2026 as a divisional application to the application mentioned under INID code 62.

(54) **HCRHR2 SELECTIVE POLYPEPTIDES**

(57) The present invention relates to UCN2 analogues with high solubility, improved potency, high chemical stability and/or improved selectivity for CRHR2 over CRHR1.

105
100
95
90
85
80
75
Body weight (%)
1
6
11
16
21
26
31
Study day
*
***
***
***
Vehicle
SEQ ID NO: 16 10 nmol/kg
SEQ ID NO: 16 30 nmol/kg
SEQ ID NO: 215 10 nmol/kg
SEQ ID NO: 215 30 nmol/kg

**FIG. 5**

EP 4 810 052 A2

## Description

FIELD OF THE INVENTION

The present invention relates to UCN2 analogues. In particular, the present invention relates to UCN2 analogues with improved potency, improved chemical stability, and/or high selectivity.

BACKGROUND OF THE INVENTION

Urocortins (UCNs) are endogenous peptides belonging to the corticotropin-releasing factor (CRF) family. This family also includes corticotropin-releasing hormone (CRH), also referred to as corticotropin-releasing factor (CRF), urotensins, and sauvagine. The corticotropin-releasing factor (CRF) family of peptides acts through corticotropin-releasing hormone (CRH) receptors, namely corticotropin-releasing hormone receptor 1 (hCRHR1) and corticotropin-releasing hormone receptor 2 (hCRHR2), which are encoded by the hCRHR1 gene and the hCRHR2 gene, respectively. hCRHR1 is expressed in the central nervous system (CNS), whereas hCRHR2 is expressed in the CNS as well as in several peripheral tissues. The highest concentration of CRH neurons is found in the hypothalamic paraventricular nucleus (PVN).

There are three known endogenous urocortins named urocortin-I (UCN1), urocortin-II (UCN2), and urocortin-III (UCN3). Despite a high degree of sequence homology of urocortins, the binding of these peptides to hCRHR1 and hCRHR2 is different. UCN1 (and CRH) non-selectively activates hCRHR1 and hCRHR2, whereas UCN2 and UCN3 show higher selectively towards hCRHR2. UCN2 is a 38 amino acid neuropeptide with the amino acid sequence IVLSLDV-PIGLLQILLEQARARAAREQATTNARILARV (SEQ ID NO: 1).

The CRH family of peptides modulates the neuroendocrine stress response via the hypothalamic-pituitary adrenal axis (HPA). These effects are, however, believed to be mediated exclusively by activation of the hCRHR1. In line with this and unlike CRH, peripheral UCN2 or UCN3 administration does not increase corticosterone secretion. Furthermore, activation of hCRHR2 by UCN2 reduces food intake without provoking behavioural arousal or anxiogenic effects as observed for the anorexigenic action induced by the activation of the brain hCRHR1 signalling pathways. This collectively, emphasizes that the stress response and the beneficial cardiometabolic effects mediated by the CRH family of peptides can be separated by using selective hCRHR2 agonists.

Urocortins acting at hCRHR2 have shown marked and beneficial hemodynamic, hormonal, and renal effects in experimental models of heart failure, such as reductions in cardiac preload and afterload, increases in cardiac output and contractility, decreased blood pressure, increased vasodilation and improved kidney function (i.e. increased creatinine clearance). In clinical studies, UCN2 and UCN3 have been shown to have direct vasodilator actions in healthy volunteers and in patients with heart failure. These findings support that urocortins may be useful in the treatment of cardiovascular or kidney diseases.

Furthermore, urocortins have recently gained significant interest in the field of metabolic disorders. Obesity and associated metabolic disorders such as insulin resistance and Type 2 Diabetes (T2D) are major sources of morbidity and mortality that are reaching epidemic proportions. Diet and exercise have proven to be effective in combating these afflictions; however, adherence to these interventions is generally low, and it is clear that additional treatments are needed to alleviate metabolic dysfunction.

Intracerebroventricular injection of UCN2 and UCN3 have shown to suppress food intake. In addition, chronic subcutaneous delivery of pegylated UCN2 has been shown to reduce body weight, inhibit food intake, improve glucose tolerance, and increase glucose uptake in skeletal muscle while improving body composition towards a healthier phenotype, i.e. reduced fat mass and increased/preserved lean mass. Besides the beneficial effects on food intake and body weight regulation, UCN2 has also been reported to improve whole body glucose tolerance by improving skeletal muscle insulin sensitivity and increase insulin-stimulated glucose uptake into skeletal muscle. These findings suggest that urocortins may be useful not only in the treatment of cardiovascular diseases but also in the treatment of metabolic disorders, such as diabetes and obesity, where they have potential to promote a healthy and sustained weight loss by reducing the adipose tissue mass while preserving/increasing lean body mass by selectively targeting hCRHR2 to avoid potential side effects induced by HPA-axis activation by hCRHR1.

The emerging evidence of potential benefits of targeting hCRHR2 has led to the development of a number of analogues of UCN2. One such example is WO 2022/038179, which discloses analogues of UCN2 and their effects on systolic blood pressure, body mass and body fat content. Other examples of UCN2 analogues may be found in e.g. WO 2023/285334, WO2018/013803 and WO2023/285347.

Despite the emerging of novel UCN2 analogues, there is still a need in the art for improved UCN2 analogues, in particular UCN2 analogues with high selectivity for hCRHR2 over hCRHR1, high potency, high solubility, and high physical and chemical stability at physiologic pH.

The present invention sets forth to provide peptides acting as selective hCRHR2 agonists, which show high

selectivity for hCRHR2 over hCRHR1, high solubility, increased chemical stability, and/or improved hCRHR2 potency.

SUMMARY OF THE INVENTION

The present invention relates to UCN2 analogues with high solubility, improved potency, high chemical stability, and improved selectivity for hCRHR2 over hCRHR1. The invention is set forth in the claims.

BRIEF DESCRIPTION OF THE FIGURES

Fig. 1 shows the effect on hCRHR2 and hCRHR1 potency by introducing glutamate (E) residues into positions $X^{21}$, $X^{22}$, $X^{23}$, $X^{24}$, $X^{25}$, $X^{26}$, $X^{27}$, $X^{28}$, $X^{33}$, $X^{35}$, $X^{36}$, $X^{37}$, $X^{39}$, $X^{40}$, or $X^{41}$, respectively. A positive SHAP value indicates an increased $pEC_{50}$ on hCRHR2 or hCRHR1. A negative SHAP value indicates a decreased $pEC_{50}$ on hCRHR2 or hCRHR1.

Fig. 2A shows the average hCRHR1-$pEC_{50}$ and hCRHR2-$pEC_{50}$ obtained by introducing a lipidated lysine (K) residue into each of the positions $X^4$-$X^{41}$ in the UCN2 backbone having $X^{36}$=E and $X^{40}$=E (SEQ ID NO: 228).

Fig. 2B shows the selectivity ratio (hCRHR1-$EC_{50}$/hCRHR2-$EC_{50}$) obtained by introducing a lipidated lysine (K) residue into each of the positions $X^4$-$X^{41}$ in the UCN2 backbone having $X^{36}$=E and $X^{40}$=E (SEQ ID NO: 228).

Fig. 3 shows the mean SHAP values in substitution of different amino acids residues into the lipidated UCN2 analogue SEQ ID NO: 3 (reference 1).

Fig. 4 shows the improvement in selectivity obtained by substitution of different amino acids residues into position $X^{20}$ of reference 1 (SEQ ID NO: 205) or reference 2 (SEQ ID NO: 124).

Fig. 5 shows that selected peptides (SEQ ID NO: 16, and 215) reduce body weight in DIO mice (n = 9, mean ± SEM). Relative body weight to first day of dosing (study day 1). *: $p < 0.05$, ***: $p < 0.001$ compared to Vehicle.

Fig. 6 shows mean arterial blood pressure measured by radiotelemetry in conscious, free moving, healthy mice treated with (SEQ ID NO: 3) (n = 4-6/group, mean ± SEM).

Fig. 7 shows the heart rates measured by radiotelemetry in conscious, free moving, healthy mice treated with (SEQ ID NO: 3) (n = 4-6/group, mean ± SEM).

Fig. 8 shows hemodynamic effects in conscious, free moving Spontaneously Hypertensive Rats mice treated with (SEQ ID NO: 3). A) Mean arterial blood pressure. B) Heart rate. (n = 8/group, mean ± SEM).

Figs. 9A-F show hemodynamic and safety properties of SEQ ID NO: 3 after subcutaneous bolus application in an ascending dose regime (0.03, 0.1, 0.3 and 1.0 mg/kg) as measured in conscious, free moving, healthy pigs using radiotelemetry. A) Heart rate. B) Mean arterial blood pressure. C) Contractility. D) Relaxation. E) PR-Interval, ECG. F) QT-Interval. Data are mean of n= 4-8 animals per group.

Figs. 10A-F show hemodynamic and safety properties of SEQ ID NO: 3 after once weekly subcutaneous bolus application (0.3 mg/kg) as measured in conscious, free moving, healthy pigs using radiotelemetry. A) Heart rate. B) Mean arterial blood pressure. C) Contractility. D) Relaxation. E) PR-Interval, ECG. F) QT-Interval. Data are mean of n= 6-8 animals per group ± SEM.

Figs. 11A-D show hemodynamic effects of SEQ ID NO: 215 administration. A) Contractility (n =4-12, mean ± SEM). B) Coronary blood flow (n =1-6, mean ± SEM). C) Rate pressure product (n =1-10), mean ± SEM). D) Rate pressure product versus contractility.

Embodiments of the invention will be described in more detail in the following with regard to the accompanying figures.

In the present context, lipidation refers to the covalent attachment of a lipid optionally through a linker/spacer to a UCN2 agonist according to the present invention. The lipid may be a C18DA (octadecanedioic acid), or C20DA

(icosanedioic acid) optionally connected through a linker/spacer consisting of one or more covalently connected units commonly used in the art. Linkers/spacers commonly used may be, but is not limited to, e.g. [vE], [OEG], [eLys], [ACHC] or [AHX] as illustrated below.

yE OEG Ahx eLys ACHC

Lipidation is typically performed to improve the pharmacokinetic profile of a polypeptide by e.g. improving metabolic stability, reducing enzymatic degradation, lowering excretion and metabolism, all in all resulting in a prolonged in vivo half-life ($t_{1/2}$). The UCN2 agonists according to the present invention may be lipidated using various lipids (and linkers/spacers) commonly used in the art depending on the desired half-life. The polypeptides may be lipidated, e.g. at a lysine (K) residue as exemplified herein. Preferably, the lipidation is performed at a lysine residue (K) in position $X^{20}$, $X^{25}$, $X^{28}$, $X^{29}$, $X^{32}$, $X^{33}$ or $X^{40}$, most preferably position $X^{32}$ or $X^{33}$. Preferably, the lipid (and linker/spacer) is selected from the list consisting of C16DA (hexadecanedioic acid), C18DA, C20DA, C18DA[γE]-, C18DA[vE][vE]-, C18DA[γE][OEG]-, C18DA [γE][OEG][OEG]-, C18DA[γE][γE][OEG][OEG]-, C18DA[vE][AHX]-, C18DA[vE][vE][AHX]-, C20DA[γE]-, C20DA[γE][γE]-, C20DA[γE][OEG]-, C20DA[γE][OEG][OEG]-, C20DA[γE][γE][OEG][OEG]-, C20DA[γE][AHX]-, or C20DA[γE][γE][AHX]-. More preferably, the lipid (and linker) is selected from the list consisting of C18DA[vE]-, C18DA[vE][vE]-, C18DA[γE] [OEG]-, C18DA[γE][OEG][OEG]-, C18DA[vE][vE][OEG][OEG]-, C20DA[γE]-, C20DA[γE][γE]-, C20DA[γE][OEG]-, C20DA[γE][OEG][OEG]-, or C20DA[γE][γE][OEG][OEG]-. Most preferably, the lipidation is C20DA[γE][γE][OEG][OEG]-.

The amino acids herein refer to the natural amino acids (i.e. L amino acids) unless otherwise stated. The abbreviation Aib refers to 2-aminoisobutyric acid. The abbreviation Nle refers to L-norleucine ((2S)-2-Aminohexanoic acid). MeI refers to N-methyl-L-isoleucine. Hyp refers to L-hydroxyproline ((2S,4R)-4-Hydroxyproline). Cle refers to cycloleucine (1-Aminocyclopentane-1-carboxylic acid). c4NHPro refers to cis-4-amino-L-proline ((2S,4S)-L-Pro(4-NH2)-OH). c4FPro refers to cis-4-fluoro-L-proline ((2S,4S)-L-Pro(4-F)-OH). In the present context, a substitution in a derivative is preferably a conservative substitution to a conservative amino acid. The groups of conservative amino acids may be defined as: G, A, V, L, I, P (aliphatic or cyclic); S, C, T, M (hydroxyl or sulphur containing); F, Y, W (aromatic); H, K, R (basic); D, E, N, Q (acidic or amide).

In the present context, it should be understood that all peptides according to the invention has a C-terminal carboxamide ($-CONH_2$). In the present context, it should be understood that the polypeptides may have a free amine ($-NH_2$) at the N-terminal, be N-acylated (-NHCOR) with e.g. an acetyl (Ac) at the N-terminal, be N-methylated ($-NHCH_3$ or $-N(CH_3)_2$) at the N-terminus or be deaminated at the N-terminus. In the most preferred embodiment, the polypeptides are mono-N-methylated ($-NHCH_3$) at the N-terminal.

$EC_{50}$ values are used as a measure of agonist potency at the corticotropin-releasing hormone receptor 1 (hCRHR1) and corticotropin-releasing hormone receptor 2 (hCRHR2). An $EC_{50}$ value is a measure of the concentration of a compound required to achieve half of that compound's maximal activity in a particular assay. In the present context, a selective hCRHR2 agonist should be understood as an agonist having a hCRHR1-$EC_{50}$/hCRHR2-$EC_{50}$ ratio of at least 500. Preferably, the selectivity ratio is higher than native UCN2 (selectivity ratio of 917, see Table 2). Thus, preferably, the hCRHR1-$EC_{50}$/hCRHR2-$EC_{50}$ ratio is at least 1000, more preferably, the hCRHR1-$EC_{50}$/hCRHR2-$EC_{50}$ ratio is at least 1500, even more preferably, the hCRHR1-$EC_{50}$/hCRHR2-$EC_{50}$ ratio is at least 2000, yet even more preferably, the hCRHR1-$EC_{50}$/hCRHR2-$EC_{50}$ ratio is at least 2500, most preferably, the hCRHR1-$EC_{50}$/hCRHR2-$EC_{50}$ ratio is at least 3000, when measured using the assay and conditions as described herein for purified peptides. As $EC_{50}$ values may depend on the type of assay performed or the specific assay conditions, $EC_{50}$ for UCN2 has been given herein as an internal standard for comparison between different assay runs or even different assays. As demonstrated herein, the hCRHR1-$EC_{50}$/hCRHR2-$EC_{50}$ ratio may be >5000. The hCRHR1-$EC_{50}$/hCRHR2-$EC_{50}$ ratio is also referred to interchangeably as selectivity.

Also, the selective hCRHR2 agonists of the invention have an hCRHR1 potency relative to native UCN2 [(hCRHR1-$EC_{50}$ peptide of invention)/(hCRHR1-$EC_{50}$ UCN2) of at least 10, such as at least 15, such as at least 20, when measured using the assay and conditions as described herein for purified peptides.

The UCN2 agonists according to the present invention may be in the form of a pharmaceutically acceptable salt and/or solvate. Thus, pharmaceutically acceptable salts are intended to include any salts that are commonly used in formulations of peptides. Such salts include both acid addition salts and basic salts, and examples may be found e.g. in Remington's Pharmaceutical Sciences, 17th edition.

DETAILED DESCRIPTION OF THE INVENTION

The present invention relates to UCN2 analogues with high solubility, improved potency, high chemical stability, and improved selectivity for hCRHR2 over hCRHR1. More particularly, the present invention relates to the finding that UCN2 analogues with high solubility can be obtained by incorporating at least two glutamate residues (E) into the position $X^{21}$, $X^{22}$, $X^{24}$, $X^{25}$, $X^{26}$, $X^{27}$, $X^{28}$, $X^{33}$, $X^{33}$, $X^{36}$, $X^{39}$, or $X^{40}$, without major adverse effects on hCRHR2 potency and selectivity (see Example 1). The present invention further relates to the finding that lipidation in position $X^{20}$, $X^{25}$, $X^{27}$, $X^{28}$, $X^{29}$, $X^{32}$, $X^{33}$ or $X^{40}$ provided the highest selectivity ratio for hCRHR2 over hCRHR1 (see Example 2). The present invention further relates to the finding that UCN2 analogues with improved chemical stability can be obtained by certain amino acid residues in position $X^{33}$ or $X^{35}$ (see Example 3). Furthermore, the present invention relates to the finding that UCN2 analogues with improved hCRHR2 potency can be obtained by introduction of certain amino acids in position $X^6$, $X^{10}$, $X^{11}$, $X^{12}$, and/or $X^{38}$, in particular a norleucine (Nle) residue in position $X^{38}$ (see Example 4). Finally, the present invention relates to the finding that certain amino acids in position $X^{20}$, $X^{21}$, $X^{26}$, $X^{30}$ and/or $X^{41}$ can improve the selectivity ratio, in particular, in the presence of a potency enhancing the Nle residue in position $X^{38}$.

*UCN2 analogues with high solubility and high selectivity for hCRHR2*

As shown in Example 1 herein, amino acid positions $X^{21}$, $X^{22}$, $X^{24}$, $X^{25}$, $X^{26}$, $X^{27}$, $X^{28}$, $X^{33}$, $X^{35}$, $X^{36}$, $X^{39}$, and $X^{40}$ were identified as suitable positions to incorporate a glutamate residue (E) to increase the solubility of the peptides. Furthermore, as shown in Example 2 herein, the amino acid positions $X^{20}$, $X^{22}$, $X^{28}$, $X^{29}$, $X^{32}$, $X^{33}$ and $X^{40}$ were identified as suitable positions for introducing a lipidated lysine (K) residue to alter the PK properties of the peptides while providing the highest selectivity ratio for hCRHR2 over hCRHR1.

Thus, in a first aspect, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-$X^{22}$-R-$X^{24}$-$X^{25}$-$X^{26}$-$X^{27}$-$X^{28}$-$X^{29}$-$X^{30}$-A-$X^{32}$-$X^{33}$-N-$X^{35}$-$X^{36}$-I-$X^{38}$-$X^{39}$-$X^{40}$-$X^{41}$-$NH_2$ (I

) wherein $X^4$ is selected as I, MeI or P; $X^6$ is selected as L or T; $X^{10}$ is selected as Cle or V; $X^{11}$ is selected as P or Hyp; $X^{12}$ is selected as I or L; $X^{20}$ is selected as E, A, F, G, H, I, K, L, N, Q, R, S, T, D, P, W, Y or V; $X^{21}$ is selected as Q, E or L; $X^{22}$ is selected as A or E; $X^{24}$ is selected as A, or E; $X^{25}$ is selected as R, E or K; $X^{26}$ is selected as A, E or L; $X^{27}$ is selected as A, E, or K; $X^{28}$ is selected as R, E, or K; $X^{29}$ is selected as E or K; $X^{30}$ is selected as Q or R; $X^{32}$ is selected as T or K; $X^{33}$ is selected as T, E, K, V, Y, W, S, P, F, L, I, H, G, Q, A or Aib; $X^{35}$ is selected as A, E, W, T, S, F, K, L, H, G, Q, D, N, R, Aib, L, I, or V; $X^{36}$ is selected as R or E; $X^{38}$ is selected as L or Nle; $X^{39}$ is selected as A or E; $X^{40}$ is selected as R, E or K; $X^{41}$ is V, or I; and wherein only one of $X^{20}$, $X^{25}$, $X^{27}$, $X^{28}$, $X^{29}$, $X^{32}$, $X^{33}$, or $X^{40}$, is selected as K, and wherein the K is lipidated optionally through a linker/spacer; and further wherein at least two of $X^{21}$, $X^{22}$, $X^{24}$, $X^{25}$, $X^{26}$, $X^{27}$, $X^{28}$, $X^{33}$, $X^{35}$, $X^{36}$, $X^{39}$, and $X^{40}$ are selected as E.

It is highly preferred that at least two of $X^{22}$, $X^{33}$, $X^{36}$, and $X^{40}$ are selected as E to improve the solubility of the peptides.

Thus, in a preferred embodiment of the first aspect, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-$X^{22}$-R-A-$X^{25}$-$X^{26}$-$X^{27}$-$X^{28}$-$X^{29}$-$X^{30}$-A-$X^{32}$-$X^{33}$-N-$X^{35}$-$X^{36}$-I-$X^{38}$-$X^{39}$-$X^{40}$-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as I or L; $X^{20}$ is selected as E, A, F, G, H, I, K, L, N, Q, R, S, T, D, P, W, Y or V; $X^{21}$ is selected as Q, or L; $X^{22}$ is selected as A or E; $X^{25}$ is selected as R, or K; $X^{26}$ is selected as A, or L; $X^{27}$ is selected as A, or K; $X^{28}$ is selected as R, or K; $X^{29}$ is selected as E or K; $X^{38}$ is selected as Q or R; $X^{32}$ is selected as T or K; $X^{33}$ is selected as T, E, K, V, Y, W, S, P, F, L, I, H, G, Q, A or Aib; $X^{35}$ is selected as A, E, W, T, S, F, K, L, H, G, Q, D, N, R, Aib, L, I, or V; $X^{36}$ is selected as R or E; $X^{38}$ is selected as L or Nle; $X^{40}$ is selected as R, E or K; $X^{41}$ is V, or I; and wherein only one of $X^{20}$, $X^{25}$, $X^{27}$, $X^{28}$, $X^{29}$, $X^{32}$, $X^{33}$, or $X^{40}$ is selected as K, and wherein the K is lipidated optionally through a linker/spacer; and further wherein at least two of $X^{22}$, $X^{33}$, $X^{36}$, and $X^{40}$ are selected as E.

In a preferred embodiment of the first aspect, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-$X^{22}$-R-A-R-$X^{26}$-A-R-$X^{29}$-$X^{30}$-A-$X^{32}$-$X^{33}$-N-$X^{35}$-$X^{36}$-I-$X^{38}$-A-$X^{40}$-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as I or L; $X^{20}$ is selected as E, A, F, G, H, I, K, L, N, Q, R, S, T, D, P, W, Y or V; $X^{21}$ is selected as Q or L; $X^{22}$ is selected as A or E; $X^{26}$ is selected as A, or L; $X^{29}$ is selected as E or K; $X^{30}$ is selected as Q or R; $X^{32}$ is selected as T or K; $X^{33}$ is selected as T, E, K, V, Y, W, S, P, F, L, I, H, G, Q, A or Aib; $X^{35}$ is selected as A, E, W, T, S, F, K, L, H, G, Q, D, N, R, Aib, L, I, or V; $X^{36}$ is selected as R or E; $X^{38}$ is selected as L or Nle; $X^{40}$ is selected as R, E or K; $X^{41}$ is V, or I; and wherein only one of $X^{20}$, $X^{29}$, $X^{32}$, $X^{33}$, or $X^{40}$ is selected as K, and wherein the K is lipidated optionally through a linker/spacer; and further wherein at least two of $X^{22}$, $X^{33}$, $X^{36}$, and $X^{40}$ are selected as E.

In the most preferred embodiment, $X^{36}$ and $X^{40}$ are selected as E to increase the solubility of the peptides.

Thus, in a more preferred embodiment of the first aspect, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-A-R-A-R-$X^{26}$-A-R-$X^{29}$-$X^{30}$-A-$X^{32}$-$X^{33}$-N-$X^{35}$-E-I-$X^{38}$-A-E-$X^{40}$-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as I or L; $X^{20}$ is selected as E, A, F, G, H, I, K, L, N, Q, R, S, T, D, P, W, Y or V; $X^{21}$ is selected as Q or L; $X^{26}$ is selected as A, or L; $X^{29}$ is selected as E or K; $X^{30}$ is selected as Q or R; $X^{32}$ is selected as T or K; $X^{33}$ is selected as T, E, K, V, Y, W, S, P, F, L, I, H, G, Q, A or Aib; $X^{35}$ is selected as A, E, W, T, S, F, K, L, H, G, Q, D, N, R, Aib, L, I, or V; $X^{38}$ is selected as L or Nle; $X^{41}$ is V, or I; and wherein only one of $X^{20}$, $X^{29}$, $X^{32}$, or $X^{33}$ is selected as K, and wherein the K is lipidated optionally through a linker/spacer.

All the amino acid positions $X^{20}$, $X^{25}$, $X^{28}$, $X^{29}$, $X^{32}$, $X^{33}$ and $X^{40}$ were identified as suitable positions for introducing a lipidated lysine in order to alter the PK properties of the peptides while providing high selectivity for hCRHR2. However, it is most preferred that $X^{32}$ or $X^{33}$ is used as lipidation site.

Thus, in an even more preferred embodiment of the first aspect, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-A-R-A-R-$X^{26}$-A-R-E-$X^{30}$-A-$X^{32}$-$X^{33}$-N-$X^{35}$-E-I-$X^{38}$-A-E-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as I or L; $X^{20}$ is selected as E, A, F, G, H, I, K, L, N, Q, R, S, T, D, P, W, Y or V; $X^{21}$ is selected as Q or L; $X^{26}$ is selected as A, or L; $X^{30}$ is selected as Q or R; $X^{32}$ is selected as T or K; $X^{33}$ is selected as T, E, K, V, Y, W, S, P, F, L, I, H, G, Q, A or Aib; $X^{35}$ is selected as A, E, W, T, S, F, K, L, H, G, Q, D, N, R, Aib, L, I, or V; $X^{38}$ is selected as L or Nle; $X^{41}$ is V, or I; and wherein only one of $X^{32}$ or $X^{33}$ is selected as K, and wherein the K is lipidated optionally through a linker/spacer.

In yet an even more preferred embodiment of the first aspect, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-A-R-A-R-A-A-R-E-Q-A-$X^{32}$-$X^{33}$-N-$X^{35}$-E-I-$X^{38}$-A-E-$X^{41}$-$NH_2$ (I)

wherein $X^4$ is selected as I, MeI or P; $X^{20}$ is selected as E, A, F, G, H, I, K, L, N, Q, R, S, T, or V; $X^{21}$ is selected as Q or L; $X^{32}$ is selected as T or K; $X^{33}$ is selected as T, E, K, V, Y, W, S, P, F, L, I, H, G, Q, A or Aib; $X^{35}$ is selected as A, E, W, T, S, F, K, L, H, G, Q, D, N, R, Aib, L, I, or V; $X^{38}$ is selected as L or Nle; $X^{41}$ is V, or I; and wherein only one of $X^{32}$ or $X^{33}$ is selected as K, and wherein the K is lipidated optionally through a linker/spacer.

In a highly preferred embodiment of the first aspect, the present invention relates to a polypeptide or a pharmaceutically acceptable salt thereof comprising the structure of Formula (I)

$X^4$-V-L-S-L-D-V-P-I-G-L-L-Q-I-L-L-$X^{20}$-$X^{21}$-A-R-A-R-A-A-R-E-Q-A-$X^{32}$-$X^{33}$-N-$X^{35}$-E-I-$X^{38}$-A-E-$X^{41}$-$NH_2$ (I)

wherein $X^4$ is selected as I, MeI or P; $X^{20}$ is selected as E, A, F, G, H, I, K, L, N, Q, R, S, T, or V; $X^{21}$ is selected as Q or L; $X^{32}$ is selected as T or K; $X^{33}$ is selected as T, E, K, or Aib; $X^{35}$ is selected as A, L, I, or V; $X^{38}$ is selected as L or Nle; $X^{41}$ is V, or I; and $X^{32}$ is selected as K, and wherein the K is lipidated optionally through a linker/spacer.

*UCN2 analogues with improved chemical stability*

Position $X^{34}$ was identified as a chemical labile residue in the UCN2 backbone. As shown in Example 3 herein,

substitution of the amino acid alanine (A) in position $X^{35}$ with the amino acids Aib, L, I, or V or substitution of the amino acid threonine (T) in position $X^{33}$ with the amino acid E, K(Lip), or Aib resulted in a significant improvement in chemical stability with no impact on potency or only a minor sacrifice of potency.

Thus, in a second aspect, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-$X^{22}$-R-$X^{24}$-$X^{25}$-$X^{26}$-$X^{27}$-$X^{28}$-$X^{29}$-$X^{30}$-A-$X^{32}$-$X^{33}$-N-$X^{35}$-$X^{36}$-I-$X^{38}$-$X^{39}$-$X^{40}$-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as I or L; $X^{20}$ is selected as E, A, F, G, H, I, K, L, N, Q, R, S, T, D, P, W, Y or V; $X^{21}$ is selected as Q, E or L; $X^{22}$ is selected as A or E; $X^{24}$ is selected as A, or E; $X^{25}$ is selected as R, E or K; $X^{26}$ is selected as A, E or L; $X^{27}$ is selected as A, E, or K; $X^{28}$ is selected as R, E, or K; $X^{29}$ is selected as E or K; $X^{30}$ is selected as Q or R; $X^{32}$ is selected as T or K; $X^{33}$ is selected as T, E, K, or Aib; $X^{35}$ is selected as A, E, Aib, L, I, or V; $X^{36}$ is selected as R or E; $X^{38}$ is selected as L or Nle; $X^{39}$ is selected as A or E; $X^{40}$ is selected as R, E or K; $X^{41}$ is V, or I; and wherein only one of $X^{20}$, $X^{25}$, $X^{27}$, $X^{28}$, $X^{29}$, $X^{32}$, $X^{33}$, or $X^{40}$ is selected as K, and wherein the K is lipidated optionally through a linker/spacer; and further wherein at least two of $X^{21}$, $X^{22}$, $X^{24}$, $X^{25}$, $X^{26}$, $X^{27}$, $X^{28}$, $X^{33}$, $X^{35}$, $X^{36}$, $X^{39}$, and $X^{40}$ are selected as E; and further, wherein $X^{35}$ is selected as Aib, L, I, or V when $X^{33}$ is T, or wherein $X^{33}$ is selected as E, K, or Aib, wherein the K is lipidated optionally through a linker/spacer when $X^{35}$ is A.

In a preferred embodiment, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-A-R-A-R-$X^{26}$-A-R-$X^{29}$-$X^{30}$-A-$X^{32}$-$X^{33}$-N-$X^{35}$-E-I-$X^{38}$-A-E-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as I or L; $X^{20}$ is selected as E, A, F, G, H, I, K, L, N, Q, R, S, T, D, P, W, Y or V; $X^{21}$ is selected as Q or L; $X^{26}$ is selected as A, or L; $X^{29}$ is selected as E or K; $X^{30}$ is selected as Q or R; $X^{32}$ is selected as T or K; $X^{33}$ is selected as T, E, K, or Aib; $X^{35}$ is selected as A, Aib, L, I, or V; $X^{38}$ is selected as L or Nle; $X^{41}$ is V, or I; and wherein only one of $X^{20}$, $X^{29}$, $X^{32}$, or $X^{33}$ is selected as K, and wherein the K is lipidated optionally through a linker/spacer; and further wherein $X^{35}$ is selected as Aib, L, I, or V when $X^{33}$ is T, or wherein $X^{33}$ is selected as E, K, or Aib, wherein the K is lipidated optionally through a linker/spacer when $X^{35}$ is A.

In an even more preferred embodiment, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-A-R-A-R-$X^{26}$-A-R-E-$X^{30}$-A-$X^{32}$-$X^{33}$-N-$X^{35}$-E-I-$X^{38}$-A-E-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as I or L; $X^{20}$ is selected as E, A, F, G, H, I, K, L, N, Q, R, S, T, D, P, W, Y or V; $X^{21}$ is selected as Q or L; $X^{26}$ is selected as A, or L; $X^{30}$ is selected as Q or R; $X^{32}$ is selected as T or K; $X^{33}$ is selected as T, E, K, or Aib; $X^{35}$ is selected as A, Aib, L, I, or V; $X^{38}$ is selected as L or Nle; $X^{41}$ is V, or I; and wherein only one of $X^{32}$ or $X^{33}$ is selected as K, and wherein the K is lipidated optionally through a linker/spacer; and further wherein $X^{35}$ is selected as Aib, L, I, or V when $X^{33}$ is T, or wherein $X^{33}$ is selected as E, K, or Aib, wherein the K is lipidated optionally through a linker/spacer when $X^{35}$ is A.

In an even more preferred embodiment, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-A-R-A-R-$X^{26}$-A-R-E-$X^{30}$-A-$X^{32}$-T-N-$X^{35}$-E-I-$X^{38}$-A-E-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as I or L; $X^{20}$ is selected as E, A, F, G, H, I, K, L, N, Q, R, S, T, D, P, W, Y or V; $X^{21}$ is selected as Q or L; $X^{26}$ is selected as A, or L; $X^{30}$ is selected as Q or R; $X^{35}$ is selected as Aib, L, I, or V; $X^{38}$ is selected as L or Nle; $X^{41}$ is V, or I; and wherein $X^{32}$ is selected as K, and wherein the K is lipidated optionally through a linker/spacer.

In yet an even more preferred embodiment, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-A-R-A-R-$X^{26}$-A-R-E-$X^{30}$-A-$X^{32}$-$X^{33}$-N-A-E-I-$X^{38}$-A-E-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as I or L; $X^{20}$ is selected as E, A, F, G, H, I, K, L, N, Q, R, S, T, D, P, W, Y or V; $X^{21}$ is selected as Q or L; $X^{26}$ is selected as A, or L; $X^{30}$ is selected as Q or R; $X^{32}$ is selected as T or K; $X^{33}$ is selected as E, K, or Aib; $X^{38}$ is selected as L or Nle; $X^{41}$ is V, or I; and wherein only one of $X^{32}$ or $X^{33}$ is selected as K, and wherein the K is lipidated optionally through a linker/spacer.

In another even more preferred embodiment, the present invention relates to a polypeptide or a pharmaceutically acceptable salt thereof comprising the structure of Formula (I)

$X^4$-V-L-S-L-D-P-I-G-L-L-Q-I-L-L-$X^{20}$-$X^{21}$-A-R-A-R-A-A-R-E-Q-A-$X^{32}$-T-N-$X^{35}$-E-I-$X^{38}$-A-E-$X^{41}$-$NH_2$ (I)

wherein $X^4$ is selected as I, MeI or P; $X^{20}$ is selected as E, A, F, G, H, I, K, L, N, Q, R, S, T, or V; $X^{21}$ is selected as Q or L; $X^{35}$ is selected as Aib, L, I, or V; $X^{38}$ is selected as L or Nle; $X^{41}$ is V, or I; and wherein $X^{32}$ is selected as K, and wherein the K is lipidated optionally through a linker/spacer.

In yet another even more preferred embodiment of the first aspect, the present invention relates to a polypeptide or a pharmaceutically acceptable salt thereof comprising the structure of Formula (I)

$X^4$-V-L-S-L-D-P-I-G-L-L-Q-I-L-L-$X^{20}$-$X^{21}$-A-R-A-R-A-A-R-E-Q-A-$X^{32}$-$X^{33}$-N-A-E-I-$X^{38}$-A-E-$X^{41}$-$NH_2$ (I)

wherein $X^4$ is selected as I, MeI or P; $X^{20}$ is selected as E, A, F, G, H, I, K, L, N, Q, R, S, T, or V; $X^{21}$ is selected as Q or L; $X^{32}$ is selected as T or K; $X^{33}$ is selected as E, K, or Aib; $X^{38}$ is selected as L or Nle; $X^{41}$ is V, or I; and wherein only one of $X^{32}$ or $X^{33}$, is selected as K, and wherein the K is lipidated optionally through a linker/spacer.

It is most preferred that $X^{33}$ = T and $X^{35}$ = Aib, L, I, or V, most preferably Aib, to ensure high chemical stability.

*UCN2 analogues with improved potency*

As shown in Example 4 herein, certain amino acid residues in position(s) $X^6$, $X^{10}$, $X^{11}$, $X^{12}$, and/or $X^{38}$ had a positive effect (i.e. increased) hCRHR2 potency. In particularly, a norleucine (Nle) residue in position $X^{38}$ resulted in improved hCRHR2 potency.

Thus, in a third aspect, the present invention relates to a polypeptide or a pharmaceutically acceptable salt thereof with improved hCRHR2 potency comprising the structure of Formula (I)

$X^4$-V-$X^6$-S-L-D-$X^{10}$-$X^{11}$-$X^{12}$-G-L-L-Q-I-L-L-$X^{20}$-$X^{21}$-$X^{22}$-R-$X^{24}$-$X^{25}$-$X^{26}$-$X^{27}$-$X^{28}$-$X^{29}$-$X^{30}$-A-$X^{32}$-$X^{33}$-N-$X^{35}$-$X^{36}$-I-[Nle]-$X^{39}$-$X^{40}$-$X^{41}$-($NH_2$) (I)

wherein $X^4$ is selected as I, MeI or P; $X^6$ is selected as L or T; $X^{10}$ is selected as Cle or V; $X^{11}$ is selected as P or Hyp; $X^{12}$ is selected as I or L; $X^{20}$ is selected as E, A, F, G, H, I, K, L, N, Q, R, S, T, D, P, W, Y or V; $X^{21}$ is selected as Q, E or L; $X^{22}$ is selected as A or E; $X^{24}$ is selected as A, or E; $X^{25}$ is selected as R, E or K; $X^{26}$ is selected as A, E or L; $X^{27}$ is selected as A, E, or K; $X^{28}$ is selected as R, E, or K; $X^{29}$ is selected as E or K; $X^{30}$ is selected as Q or R; $X^{32}$ is selected as T or K; $X^{33}$ is selected as T, E, K, V, Y, W, S, P, F, L, I, H, G, Q, A or Aib; $X^{35}$ is selected as A, E, W, T, S, F, K, L, H, G, Q, D, N, R, Aib, L, I, or V; $X^{36}$ is selected as R or E; $X^{39}$ is selected as A or E; $X^{40}$ is selected as R, E or K; $X^{41}$ is V, or I; and wherein only one of $X^{20}$, $X^{25}$, $X^{27}$, $X^{28}$, $X^{29}$, $X^{32}$, $X^{33}$, or $X^{40}$, is selected as K, and wherein the K is lipidated optionally through a linker/spacer; and further wherein at least two of $X^{21}$, $X^{22}$, $X^{24}$, $X^{25}$, $X^{26}$, $X^{27}$, $X^{28}$, $X^{33}$, $X^{35}$, $X^{36}$, $X^{39}$, and $X^{40}$ are selected as E.

In a preferred embodiment, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-A-R-A-R-$X^{26}$-A-R-E-$X^{30}$-A-$X^{32}$-$X^{33}$-N-$X^{35}$-E-I-[Nle]-A-E-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as L or I; $X^{20}$ is selected as E, A, F, G, H, I, K, L, N, Q, R, S, T, D, P, W, Y or V; $X^{21}$ is selected as Q or L; $X^{26}$ is selected as A, or L; $X^{30}$ is selected as Q or R; $X^{32}$ is selected as T or K; $X^{33}$ is selected as T, E, K, V, Y, W, S, P, F, L, I, H, G, Q, A or Aib; $X^{35}$ is selected as A, E, W, T, S, F, K, L, H, G, Q, D, N, R, Aib, L, I, or V; $X^{41}$ is V, or I; and wherein only one of $X^{32}$ or $X^{33}$ is selected as K, and wherein the K is lipidated optionally through a linker/spacer.

In a more preferred embodiment, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-A-R-A-R-$X^{26}$-A-R-E-$X^{30}$-A-$X^{32}$-$X^{33}$-N-$X^{35}$-E-I-[Nle]-A-E-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as L or I; $X^{20}$ is selected as E, A, F, G, H, I, K, L, N, Q, R, S, T, D, P, W, Y or V; $X^{21}$ is selected as Q or L; $X^{26}$ is selected as A, or L; $X^{30}$ is selected as Q or R; $X^{32}$ is selected as T or K; $X^{33}$ is selected as T, E, K, or Aib; $X^{35}$ is selected as A, Aib, L, I, or V; $X^{41}$ is V, or I; and wherein only one of $X^{32}$ or $X^{33}$ is selected as K, and wherein the K is lipidated optionally through a linker/spacer; and further wherein $X^{35}$ is selected as Aib, L, I, or V when $X^{33}$ is T, or wherein $X^{33}$ is selected as E, K, or Aib, wherein the K is lipidated optionally through a linker/spacer when $X^{35}$ is A.

In some highly preferred embodiments wherein $X^{38}$ is selected as Nle, potency may be further improved by selecting $X^6$ as T; $X^{10}$ as Cle; $X^{11}$ as Hyp; and/or $X^{12}$ as I or L.

In another preferred embodiment, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-A-R-A-R-$X^{26}$-A-R-E-$X^{30}$-A-$X^{32}$-$X^{33}$-N-$X^{35}$-E-I-[Nle]-A-E-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as L or I; $X^{20}$ is selected as E, A, F, G, H, I, K, L, N, Q, R, S, T, D, P, W, Y or V; $X^{21}$ is selected as Q or L; $X^{26}$ is selected as A, or L; $X^{30}$ is selected as Q or R; $X^{32}$ is selected as T or K; $X^{33}$ is selected as T, E, K, V, Y, W, S, P, F, L, I, H, G, Q, A or Aib; $X^{35}$ is selected as A, E, W, T, S, F, K, L, H, G, Q, D, N, R, Aib, L, I, or V; $X^{41}$ is V, or I; and wherein only one of $X^{32}$ or $X^{33}$ is selected as K, and wherein the K is lipidated optionally through a linker/spacer.

*UCN2 analogues with improved potency and high selectivity*

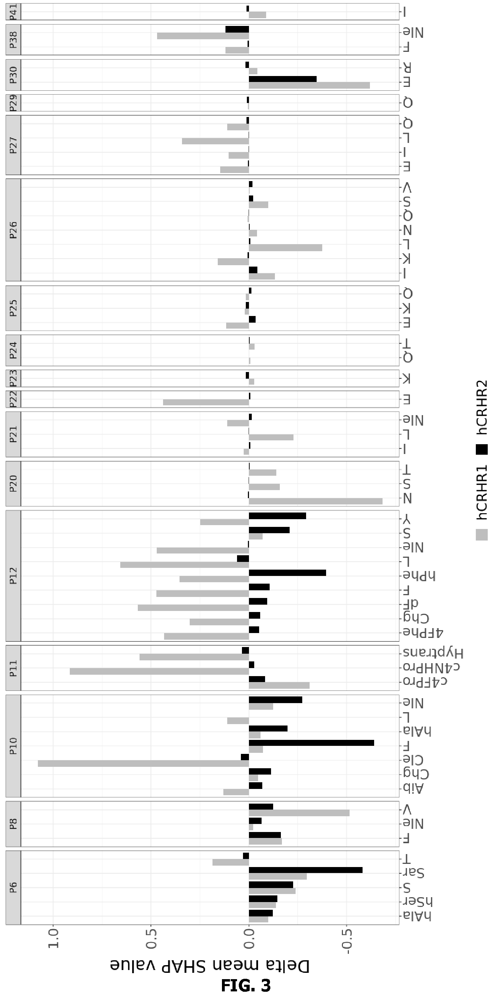

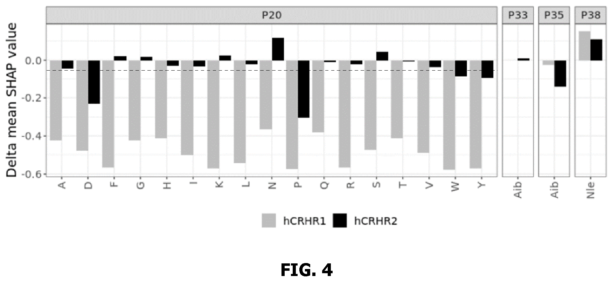

As shown herein, a norleucine (Nle) residue in position $X^{38}$ resulted in improved hCRHR2 potency, but with a simultaneously drop in selectivity for hCRHR2 (see Example 4, Table 3). As shown in Fig. 3, certain amino acid residues in position(s) $X^{20}$, $X^{21}$, $X^{26}$, $X^{30}$ and/or $X^{41}$ were found to decrease hCRHR1 potency while hCRHR2 potency was unchanged. These positions (i.e. $X^{20}$, $X^{21}$, $X^{26}$, $X^{30}$ and/or $X^{41}$) may be used to regain high selectivity when potency-improving substitutions are introduced, e.g. Nle in position $X^{38}$. As shown in Fig. 4 for position $X^{20}$, the amino acid residue A, F, G, H, I, K, L, N, Q, R, S, T, or V were all found to decrease hCRHR1 potency without compromising hCRHR2 potency (see Example 5, Fig. 4). As shown in Fig. 3 for position $X^{21}$, leucine (L); for position $X^{26}$, leucine (L); for position $X^{30}$, arginine (R), and for position $X^{41}$, isoleucine (I), were also found to decrease hCRHR1 potency without compromising hCRHR2 potency.

In a highly preferred embodiment, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-A-R-A-R-$X^{26}$-A-R-E-$X^{30}$-A-$X^{32}$-$X^{33}$-N-$X^{35}$-E-I-[Nle]-A-E-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as L or I; $X^{20}$ is selected as E, A, F, G, H, I, K, L, N, Q, R, S, T, D, P, W, Y or V; $X^{21}$ is selected as Q or L; $X^{26}$ is selected as A, or L; $X^{30}$ is selected as Q or R; $X^{32}$ is selected as T or K; $X^{33}$ is selected as T, E, K, V, Y, W, S, P, F, L, I, H, G, Q, A or Aib; $X^{35}$ is selected as A, E, W, T, S, F, K, L, H, G, Q, D, N, R, Aib, L, I, or V; $X^{41}$ is V, or I; and wherein only one of $X^{32}$ or $X^{33}$ is selected as K, and wherein the K is lipidated optionally through a linker/spacer, and further wherein at least one of $X^{20}$, $X^{21}$, $X^{26}$, $X^{30}$ or $X^{41}$ is selected as follows, $X^{20}$ is selected as A, F, G, H, I, K, L, N, Q, R, S, T, or V; $X^{21}$ is selected as L; $X^{26}$ is selected as L; $X^{30}$ is selected as R; or $X^{41}$ is selected as I.

In yet a highly preferred embodiment, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-A-R-A-R-$X^{26}$-A-R-E-$X^{30}$-A-$X^{32}$-$X^{33}$-N-$X^{35}$-E-I-[Nle]-A-E-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as L or I; $X^{20}$ is selected as E, A, F, G, H, I, K, L, N, Q, R, S, T, D, P, W, Y or V; $X^{21}$ is selected as Q or L; $X^{26}$ is selected as A, or L; $X^{30}$ is selected as Q or R; $X^{32}$ is selected as T or K; $X^{33}$ is selected as T, E, K, or Aib; $X^{35}$ is selected as A, Aib, L, I, or V; $X^{41}$ is V, or I; and wherein only one of $X^{32}$ or $X^{33}$ is selected as K, and wherein the K is lipidated optionally through a linker/spacer; and further wherein $X^{35}$ is selected as Aib, L, I, or V when $X^{33}$ is T, or wherein $X^{33}$ is selected as E, K, or Aib,

wherein the K is lipidated optionally through a linker/spacer when $X^{35}$ is A; and further wherein at least one of $X^{20}$, $X^{21}$, $X^{26}$, $X^{30}$ or $X^{41}$ is selected as follows, $X^{20}$ is selected as A, F, G, H, I, K, L, N, Q, R, S, T, or V; $X^{21}$ is selected as L; $X^{26}$ is selected as L; $X^{30}$ is selected as R; or $X^{41}$ is selected as I.

More preferably, position $X^{20}$ or $X^{41}$ is used to improve selectivity, and $X^{21}$, $X^{26}$, and $X^{30}$ are selected as the native amino acid in UCN2.

Thus, in a more highly preferred embodiment, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-Q-A-R-A-R-A-A-R-E-Q-A-$X^{32}$-$X^{33}$-N-$X^{35}$-E-I-[Nle]-A-E-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as L or I; $X^{20}$ is selected as E, A, F, G, H, I, K, L, N, Q, R, S, T, D, P, W, Y or V; $X^{32}$ is selected as T or K; $X^{33}$ is selected as T, E, K, V, Y, W, S, P, F, L, I, H, G, Q, A or Aib; $X^{35}$ is selected as A, E, W, T, S, F, K, L, H, G, Q, D, N, R, Aib, L, I, or V; $X^{41}$ is V, or I; and wherein only one of $X^{32}$ or $X^{33}$ is selected as K, and wherein the K is lipidated optionally through a linker/spacer, and further wherein one of $X^{20}$ or $X^{41}$ is selected as follows; $X^{20}$ is selected A, F, G, H, I, K, L, N, Q, R, S, T, or V; or $X^{41}$ is selected as I.

In yet a more highly preferred embodiment, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-Q-A-R-A-R-A-A-R-E-Q-A-$X^{32}$-$X^{33}$-N-$X^{35}$-E-I-[Nle]-A-E-$X^{41}$-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as L or I; $X^{20}$ is selected as E, A, F, G, H, I, K, L, N, Q, R, S, T, D, P, W, Y or V; $X^{32}$ is selected as T or K; $X^{33}$ is selected as T, E, K, or Aib; $X^{35}$ is selected as A, Aib, L, I, or V; $X^{41}$ is V, or I; and wherein only one of $X^{32}$ or $X^{33}$ is selected as K, and wherein the K is lipidated optionally through a linker/spacer; and further wherein $X^{35}$ is selected as Aib, L, I, or V when $X^{33}$ is T, or wherein $X^{33}$ is selected as E, K, or Aib, wherein the K is lipidated optionally through a linker/spacer when $X^{35}$ is A; and further wherein one of $X^{20}$ or $X^{41}$ is selected as follows; $X^{20}$ is selected A, F, G, H, I, K, L, N, Q, R, S, T, or V; or $X^{41}$ is selected as I.

Most preferably, position $X^{20}$ is used to regain selectivity in the presence of a potency enhancing Nle in position $X^{38}$, and most preferably, $X^{41}$ is selected as V.

Hence, in another preferred embodiment, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-$X^{21}$-A-R-A-R-$X^{26}$-A-R-E-$X^{30}$-A-$X^{32}$-$X^{33}$-N-$X^{35}$-E-I-[Nle]-A-E-V-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as L or I, $X^{20}$ is selected as A, F, G, H, I, K, L, N, Q, R, S, T, or V; $X^{21}$ is selected as Q or L; $X^{26}$ is selected as A, or L; $X^{30}$ is selected as Q or R; $X^{32}$ is selected as T or K; $X^{33}$ is selected as T, E, K, V, Y, W, S, P, F, L, I, H, G, Q, A or Aib; $X^{35}$ is selected as A, E, W, T, S, F, K, L, H, G, Q, D, N, R, Aib, L, I, or V; and wherein only one of $X^{32}$ or $X^{33}$ is selected as K, and wherein the K is lipidated optionally through a linker/spacer.

In an even more highly preferred embodiment, the present invention relates to a polypeptide or a pharmaceutically acceptable salt thereof comprising the structure of Formula (I)

$X^4$-V-$X^6$-S-L-D-$X^{10}$-$X^{11}$-$X^{12}$-G-L-L-Q-I-L-L-$X^{20}$-Q-A-R-A-R-A-A-R-E-Q-A-$X^{32}$-$X^{33}$-N-$X^{35}$-E-I-[Nle]-A-E-V-$NH_2$ (I)

wherein $X^4$ is selected as I, MeI or P; $X^6$ is selected as L or T; $X^{10}$ is selected as Cle or V; $X^{11}$ is selected as P or Hyp; $X^{12}$ is selected as L or I; $X^{20}$ is selected as A, F, G, H, I, K, L, N, Q, R, S, T, or V; $X^{32}$ is selected as T or K; $X^{33}$ is selected as T, E, K, or Aib; $X^{35}$ is selected as A, Aib, L, I, or V; and wherein only one of $X^{32}$ or $X^{33}$ is selected as K, and wherein the K is lipidated optionally through a linker/spacer; and further wherein $X^{35}$ is selected as Aib, L, I, or V when $X^{33}$ is T, or wherein $X^{33}$ is selected as E, K, or Aib, wherein the K is lipidated optionally through a linker/spacer when $X^{35}$ is A.

In any of the above aspects and embodiments, if not already stated or shown, it is most preferred that $X^4$ is selected as MeI or P, most preferably $X^4$ is MeI. In any of the above aspects and embodiments, if not already shown, it is most preferred that $X^6$ is selected as L. In any of the above aspects and embodiments, if not already shown, it is most preferred that $X^{10}$ is selected as V. In any of the above embodiments, if not already shown, it is most preferred that $X^{11}$ is

selected as P. In any of the above embodiments, if not already shown, it is most preferred that $X^{12}$ is selected as I. In any of the above aspects and embodiments, if not already shown, it is preferred that $X^{20}$ is selected as N, F, G, K, Q, S, or T, when $X^{38}$ = Nle; most preferably $X^{20}$ is selected as S, when $X^{38}$ = Nle. When $X^{38}$ is L, it is most preferred that $X^{20}$ is selected as E, N, F, G, K, Q, S, or T; most preferably $X^{20}$ is selected as E or S. In any of the above aspects and embodiments, if not already shown, it is most preferred that $X^{21}$ is selected as Q. In any of the above aspects and embodiments, if not already shown, it is most preferred that $X^{22}$ is selected as A. In any of the above aspects and embodiments, if not already shown, it is most preferred that $X^{24}$ is selected as A. In any of the above aspects and embodiments, if not already shown, it is most preferred that $X^{25}$ is selected as R. In any of the above aspects and embodiments, if not already shown, it is most preferred that $X^{26}$ is selected as A. In any of the above aspects and embodiments, if not already shown, it is most preferred that $X^{27}$ is selected as A. In any of the above aspects and embodiments, if not already shown, it is most preferred that $X^{28}$ is selected as R. In any of the above aspects and embodiments, if not already shown, it is most preferred that $X^{29}$ is selected as E. In any of the above aspects and embodiments, if not already shown, it is most preferred that $X^{30}$ is selected as Q. In any of the above aspects and embodiments, if not already shown, it is most preferred that $X^{38}$ is selected as Nle. In any of the above aspects and embodiments, if not already shown, it is most preferred that $X^{35}$ is selected as Aib, when $X^{33}$ is T. In any of the above aspects and embodiments, if not already shown, it is most preferred that $X^{33}$ is selected as Aib or K, wherein the K is lipidated optionally through a linker/spacer when $X^{35}$ is A. In any of the above aspects and embodiments, if not already shown, it is most preferred that $X^{39}$ is selected as A. In any of the above aspects and embodiments, if not already shown, it is most preferred that $X^{41}$ is selected as V. In any of the above aspects and embodiments, if not already shown, it is most preferred that $X^{36}$ and $X^{40}$ are selected as E.

Hence, in a particularly highly preferred embodiment, the present invention relates to a polypeptide or a pharmaceutically acceptable salt thereof comprising the structure of Formula (I)

$X^4$-V-L-S-L-D-V-P-I-G-L-L-Q-I-L-L-$X^{20}$-Q-A-R-A-R-A-A-R-E-Q-A-$X^{32}$-$X^{33}$-N-$X^{35}$-E-I-[NLe]-A-E-V-$NH_2$ (I)

wherein $X^4$ is selected as MeI or P; $X^{20}$ is selected as N, F, G, K, Q, S, or T, most preferably $X^{20}$ is selected as S; $X^{32}$ is selected as T or K; $X^{33}$ is selected as T, E, K, or Aib; $X^{35}$ is selected as A, Aib, L, I, or V; and wherein only one of $X^{32}$ or $X^{33}$ is selected as K, and wherein the K is lipidated optionally through a linker/spacer; and further wherein $X^{35}$ is selected as Aib, L, I, or V, when $X^{33}$ is T, or wherein $X^{33}$ is selected as E, K, or Aib, wherein the K is lipidated optionally through a linker/spacer when $X^{35}$ is A.

In another particularly highly preferred embodiment, the present invention relates to a polypeptide or a pharmaceutically acceptable salt thereof comprising the structure of Formula (I)

$X^4$-V-L-S-L-D-V-P-I-G-L-L-Q-I-L-L-$X^{20}$-Q-A-R-A-R-A-A-R-E-Q-A-[K*]-$X^{33}$-N-$X^{35}$-E-I-[Nle]-A-E-V-$NH_2$ (I)

wherein $X^4$ is selected as MeI or P; $X^{20}$ is selected as N, F, G, K, Q, S, or T, most preferably $X^{20}$ is selected as S ; $X^{33}$ is selected as T, or Aib; $X^{35}$ is selected as A, or Aib; and further wherein $X^{35}$ is selected as Aib, if $X^{33}$ is T, or wherein $X^{33}$ is selected as Aib, if $X^{35}$ is A; * denotes a covalent attachment of a lipid, optionally through a linker/spacer, to the ε-amino group of the lysine sidechain.

In an even more particularly highly preferred embodiment, the peptide is selected from the list consisting of:

[MeI]VLSLDVPIGLLQILLSQARARAAREQA[K*]TN[Aib]EI[Nle]AEV($NH_2$) (SEQ ID NO: 215),
PVLSLDVPIGLLQILLSQARARAAREQA[K*][Aib]NAEI[Nle]AEV($NH_2$) (SEQ ID NO: 238),
[MeI]VLSLDVPIGLLQILLEQARARAAREQAT[K*]NAEILAEV($NH_2$) (SEQ ID NO: 6), or
[MeI]VLSLDVPIGLLQILLEQARARAAREQA[K*]TN[Aib]EILAEV($NH_2$) (SEQ ID NO: 16),

wherein the * denotes a covalent attachment of a lipid, optionally through a linker/spacer, to the ε-amino group of the lysine sidechain.

The lipid (and linker/spacer) may be selected from any lipid (and linker/spacer) known in the art. Most preferably, the lipid (and linker/spacer) is C20DA[γE][γE][OEG][OEG]-.

In a most preferred embodiment, the peptide is [MeI]VLSLDVPIGLLQILLSQARARAAREQA[K*]TN[Aib]EI[Nle]AEV($NH_2$) (SEQ ID NO: 215), wherein the * denotes a covalent attachment of a lipid, to the ε-amino group of the lysine sidechain, wherein the lipid (and linker/spacer) is C20DA[γE][γE][OEG][OEG]-.

It will be apparent for those skilled in the art that any of the findings described herein regarding optimal positions to improve solubility without compromising potency or selectivity (i.e. at least two glutamate residues (E) into position $X^{21}$, $X^{22}$, $X^{24}$, $X^{25}$, $X^{26}$, $X^{27}$, $X^{28}$, $X^{33}$, $X^{35}$, $X^{36}$, $X^{39}$, or $X^{40}$); optimal lipidation sites to alter PK while ensuring the highest possible selectivity for hCRHR2 over hCRHR1 (i.e. a lipidated lysine in one of the positions $X^{20}$, $X^{25}$, $X^{27}$, $X^{28}$, $X^{29}$, $X^{32}$, $X^{33}$ or $X^{40}$);

positions to improve chemical stability (i.e. specific amino acids in position $X^{33}$ or $X^{35}$); positions to improve hCRHR2 potency (i.e. specific amino acid residues in position(s) $X^{6}$, $X^{10}$, $X^{11}$, $X^{12}$, and/or $X^{38}$, in particular a norleucine (Nle) residue in position $X^{38}$); and positions to improve the selectivity ratio (i.e. specific amino acid residues in position $X^{20}$, $X^{21}$, $X^{26}$, $X^{30}$ and/or $X^{41}$, in particularly, in the presence of a potency enhancing Nle residue in position $X^{38}$) are preferably combined to solve more than one problem and provide the best polypeptides. Likewise, these findings can also be applied to improve the peptides UCN2 analogues in the prior art such as described in WO 2023/285334, WO2018/013803 and WO2023/285347.

## EXPERIMENTAL SECTION

*General procedure for solid phase peptide synthesis*

The peptides were synthesized using a SyroII fully automated parallel peptide synthesizer (MultiSynTech GmbH, Germany), equipped with heating block, on Tentagel S RAM with a loading of 0.23-0.25 mmol/g (Rapp polymer GmbH, Germany). $N^{\alpha}$-Fmoc deprotection was performed in two stages by treating the resin with 40% piperidine/DMF (0.2 M HOBt (1-hydroxybenzotriazole)) for 3 min at 45°C followed by 20% piperidine/DMF (0.1 M HOBt) for 7 min at 75°C. Except Asp, Cys and His residues which were $N^{\alpha}$-Fmoc deprotections at room temperature, i.e. 40% piperidine/DMF (0.2 M HOBt) for 3 min followed by 20% piperidine/DMF (0.1 M HOBt) for 15 min. The coupling chemistry was DIC (N,N'-diisopropyl-carbodiimide)/Oxyma (ethyl cyano(hydroxyimino)acetate) in DMF using amino acid solutions of 0.5 M in DMF and a molar excess of 5-fold. Standard Fmoc protected amino acids were used. Coupling conditions were s single or double couplings for 15 min at 75°C. Except His and Cys residues, which were double coupled for 15 min at 50°C. Also, amino acids coupled after Aib were double coupled. The Fmoc-amino acids were dissolved at 0.5 M in DMF containing 0.5 M Oxyma, except His which was dissolved in NMP. The resin was washed 5x with DMF after $N^{\alpha}$-Fmoc deprotection and 3x after couplings.

For N-terminal lipidated examples, the lipidation was conducted on-resin as the last step in the peptide synthesis. The N-terminal lipidated peptides optionally contained linker residue(s), such as [γE], [OEG], [OEG]-[OEG] etc. The linker residues were introduced by coupling Fmoc-OEG-OH, Fmoc-OEG-OEG and/or Fmoc-Glu-OtBu to the N-terminal amino acid elongating the peptide prior to coupling the fatty diacid, such as tert-butyl protected fatty diacids, such as tBu-C18-diacid, tBu-C20-diacid etc. The linker residues were double- or triple coupled using standard conditions. The fatty acid was double coupled using 2 eq. building block.

For other lipidated examples, Boc-protected amino acid was incorporated as the N-terminal residue, and the lipidation position was incorporated as orthogonal protected Lysine, here Lys(Mtt). The Mtt group (4-methyl-trityl) was removed by treating the resin with 75% HFIP (1,1,1,3,3,3-hexafluoro-propan-2-ol) in DCM(dichloromethane) plus 5% TIPS for 10 minutes. Procedure repeated 3 times. The resin was washed with 10% DIPEA in DCM, followed by 3x DMF wash.

The lipidated peptides optionally contain linker residue(s). The linker residue(s) was/were coupled to the epsilon-amino group of the deprotected lysine prior to coupling the fatty acid, such as tert-butyl protected fatty diacids, such as tBu-C18-diacid, tBu-C20-diacid etc. The linker residues were double- or triple-coupled using standard conditions. The fatty acid was double coupled using 2 eq. building block.

*For library peptide synthesis:*

After synthesis, the resin was washed with DCM and dried, and the polypeptide was cleaved from the resin by a 45-60 min treatment with TFA (trifluoroacetic acid)/TES (triethylsilane)/DODT(2,2'-(ethylenedioxy)diethanethiol)/water (93/2.5/2.5/2.0) at 40°C, followed by precipitation with 3 volumes of cold diethyl ether, further washing with diethyl ether and left to dry. The peptides were characterized by LC-MS (Waters, Denmark) and quantified by LC-CAD (ThermoFisher scientific, Denmark). Finally, the peptides were freeze dried using a Telstar benchtop freeze drier.

*For peptides to be purified:*

After synthesis, the resin was washed with DCM and dried, and the polypeptide was cleaved from the resin by a 120 min treatment with TFA (trifluoroacetic acid)/TES (triethylsilane)/DODT(2,2'-(ethylenedioxy)diethanethiol)/water (93/2.5/2.5/2.0) at 40°C, followed by precipitation with cold diethyl ether, further washing with diethyl ether and left to dry. The peptides were dissolved in acetonitrile/water and purified by reverse phase HPLC using a Waters preparative HPLC with C18 column (Reprosil Gold 200 Å, 5μm, 40 mm × 250 mm), preparative pumps (waters 2545), UV/VIS detector (Waters 2489) and a Waters fraction collector III. The mobile phase was run with a gradient of buffer A (0.1% TFA in H2O) and buffer B (0.1% TFA in acetonitrile at a flow rate of 40 mL/min at room temperature. Relevant fractions were analysed, pooled, and lyophilized. Finally, the peptides were freeze dried using a Telstar benchtop freeze drier.

Peptide purity and mass were determined by analytical RP-HPLC-MS on a ACQUITY UPLC Peptide CSH C18

column (Waters, ACQUITY UPLC Peptide CSH, C18, 130 Å, 1.7 μm, 2.1 mm x 100 mm) using a Waters Acquity HPLC System equipped with 3100 Mass Detector. Analysis was performed by gradient elution with buffer A (0.3% TFA in $H_2O$) and buffer B (0.3% TFA in acetonitrile) at a temperature of 40 °C (gradients used 40-60%B over 14 min, or 50-70%B over 14 min).

*General procedure for determination of hCRHR1 potency*

HTRF (CisBio) cAMP assay: The assay technology has thoroughly been described in the CisBio HTRF cAMP assay Kit manual (# 62AM4PEC). Briefly, time resolved fluorescence technic was applied to measure cAMP. This technic is based on a competitive immunoassay using cryptate-labelled anti-cAMP antibody and d2-labeled cAMP. In the absence of cellular cAMP, the anti-cAMP cryptate conjugate may get into proximity to cAMP-d2 conjugate and energy (FRET) can be transferred from cryptate to d2.

CHO-K1 cells stably expressing the human corticotropin-releasing hormone receptor 1 (Eurofins/DiscoverX Cat #95-0047C2 hCRHR1 stable monoclonal cell lines) were used as cells in suspension, brought to life from frozen cell stocks immediately before assay performance. 384-Well (Corning, #4513) assay formats with a total assay volume of 20 μl were applied and cells (2.000 cells/well) were incubated with peptide agonists for 30 minutes at room temperature using DPBS (Sigma, #D8537) containing 0.5mM IBMX (Sigma, # I5879) and 0.05% casein (Sigma, # C4765-10ml) as stimulation buffer. After the addition of HTRF® detection reagents and incubation for 1 hr on a plate shaker (2400 rpm), signals at 620 and 665nm (raw counts: ratio of 665/620) were detected at a ClarioStar (BMG Labtech, Ortenberg, Germany) plate reader.

*For peptide libraries:*

Concentration-response evaluation of compounds was performed with 5 concentrations of agonist peptides and EC50 values were calculated by nonlinear regression using sigmoid concentration-response with variable slope.

*For purified peptides:*

Concentration-response evaluation of compounds was performed with 11 concentrations of agonist peptides and EC50 values were calculated by nonlinear regression using sigmoid concentration-response with variable slope.

*General procedure for determination of hCRHR2 potency.*

HTRF (CisBio) cAMP assay: The assay technology has thoroughly been described in the CisBio HTRF cAMP assay Kit manual (# 62AM4PEC). Briefly, time resolved fluorescence technic was applied to measure cAMP. This technic is based on a competitive immunoassay using cryptate-labelled anti-cAMP antibody and d2-labeled cAMP. In the absence of cellular cAMP, the anti-cAMP cryptate conjugate may get into proximity to cAMP-d2 conjugate and energy (FRET) can be transferred from cryptate to d2.

CHO-K1 cells stably expressing the human corticotropin-releasing hormone receptor 2 (Eurofins/DiscoverX, Cat #95-0048C2 hCRHR2 stable monoclonal cell lines) were used as cells in suspension, brought to life from frozen cell stocks immediately before assay performance. 384-Well (Corning, #4513) assay formats with a total assay volume of 20 μl were applied and cells (2.000 cells/well) were incubated with peptide agonists for 30 minutes at room temperature using DPBS (Sigma, #D8537) containing 0.5mM IBMX (Sigma, # I5879) and 0.05% casein (Sigma, # C4765-10ml) as stimulation buffer. After the addition of HTRF® detection reagents and incubation for 1 hr on a plate shaker (2400 rpm), signals at 620 and 665nm (raw counts: ratio of 665/620) were detected at a ClarioStar (BMG Labtech, Ortenberg, Germany) plate reader.

*For peptide libraries:*

Concentration-response evaluation of compounds was performed with 5 concentrations of agonist peptides and EC50 values were calculated by nonlinear regression using sigmoid concentration-response with variable slope.

*For purified peptides:*

Concentration-response evaluation of compounds was performed with 11 concentrations of agonist peptides and EC50 values were calculated by nonlinear regression using sigmoid concentration-response with variable slope.

*General procedure for determination of turbidity and fibril formation of library peptides*

Peptides were dissolved in buffers (50 mM sodium phosphate at pH 7.5) to 267μM and incubated for 1-2 hours at

room temperature. The samples were then divided into two replicates of 80 µl in a black 384 well plate (µ-clear, Greiner Bio-One) and mixed with Thioflavin T (ThT) to a final concentration of 4 µM. The plate was centrifuged for 2 min at 2000 rpm to remove air bubbles, sealed, and placed in a plate reader (CLARIOstar, BMG). Firstly, turbidity of the samples was measured as the absorbance at 600 nm. Secondly, the plate reader temperature was set to 40° C and the fluorescence was measured every 10 min for 72 hours by exciting the ThT at 450 nm and measuring the emission at 480 nm. Samples were stressed by shaking the plate at 700 rpm (linear) for five minutes before every measurement, and fibril formation was determined as the average emission for each sample.

*General procedure for determination of fibril formation of purified peptides*

Thioflavin T fibril formation assay: Peptides were dissolved as 267 µM in 50 mM phosphate buffer pH 7.5 for 2 h at room temperature on a rocking table. The samples were then divided into three replicates of 80 µl/well and mixed with 2 µl/well Thioflavin T (ThT)to a final concentration of 4 µM ThT in a black 384 well microplate with a clear bottom (Greiner #781096). The plate was inserted in a CLARIOstar Plus microplate reader (BMG Labtech) and fibril formation was measured as an increase in fluorescence emission at 480 nm (excitation at 450 nm) during 96 h at 40°C with cycles of 5 min rest and 5 min linearshaking at700 rpm.

*General procedure for determination of solubility of purified peptides.*

Solubility was tested in the following vehicles: 100 mM phosphate buffer facilitating a target pH of 6.5, 7.0, and 7.5. Samples were directly dissolved in Mini-UniPrep Syringeless Filter 0.45 µm unit (Whatman). For the desired concentration, such as a concentration of 4000 µM (approximately 20 mg/mL), 1600 nmol peptide was dissolved in 400 µL vehicle. Samples were incubated for at least 1 h at room temperature on a rocking table. The pH was measured and adjusted. Samples were left on the rocking table for an additional hour before a second pH measurement and adjustment. Visual inspection was performed and noted before the filter was pushed. The concentration of peptide in the filtrate was determined using CAD (Charged Aerosol Detection), double determination was done for each sample. The measured concentration was reported in µM. Furthermore, the pH of the filtrate was measured and reported. Peptides with a measured concentration within +/-20% of target concentration are considered fully soluble.

*General procedure for determination of chemical stability of library peptides*

Chemical stability was tested at a concentration of 267µM peptide in the following vehicles: 50 mM phosphate buffer pH 7.5 and 8.0. Dissolved samples were incubated for at least 2 h at room temperature on a rocking table. The main peak purity at time zero (T0) was determined using reversed phase chromatography coupled to high resolution mass spectrometry. The samples were incubated at 40°C for 7 days (T7) and 14 days (T14). The samples were analyzed with reversed phase chromatography coupled to high resolution mass spectrometry.

Reversed phase chromatography coupled to mass spectrometry was performed on a Thermo Vanquish Tandem UHPLC system equipped with a Acquity BEH column (1.7 µm, C18, 130 Å, 50 x 2.1 mm (Waters, 186002350)) coupled to a Thermo Exploris 120 high resolution mass spectrometer. Analysis was performed by gradient elution with buffer A (0.1% formic acid in $H_2O$) and buffer B (0.1% formic acid in acetonitrile) at a column temperature of 40 °C with a gradient from 5-70% B in 7.5 min at a flowrate of 0.4 mL/min. The mass spectrometer was operated in data-dependent acquisition mode at a MS1 resolution of 60.000, and a MS2 resolution of 30.000 in positive mode (Top4). Peptides were fragmented with a normalized collision energy of 25%. Peptides were quantified based on the extracted ion chromatogram (EIC) of their highest isotopic peak. The intact peptide was determined from the integrated chromatogram as the area under the main peak relative to the total peak area. The purity loss was calculated as the difference between the T0 and T7 time point and/or as the difference between the T0 and T14 time point.

*General procedure for determination of chemical stability of purified peptides*

Chemical stability was tested at a concentration of approximately of 1.0 mg/ml peptide in the following vehicles: 50 mM phosphate buffer pH 7.5. Dissolved samples were incubated for at least 2 h at room temperature on a rocking table. The main peak purity at time zero (T0) was determined using reversed phase chromatography. The samples were incubated at 40°C for 14 days (T14) and 28 days (T28). The samples were analyzed with reversed phase chromatography and size exclusion chromatography.

Reversed Phase chromatography was performed on a Thermo Dionex Ultimate UHPLC system with UV detection at 215 nm) and equipped with a Kinetex column (1.7 µm, C8, 100 Å, 150 x 2.1 mm (00F-4499-AN)). Analysis was performed by gradient elution with buffer A (0.1% TFA in 95:5 $H_2O$:acetonitrile) and buffer B (0.1% TFA in 5:95 $H_2O$: acetonitrile) at a column temperature of 50 °C (gradient used 25-55%B over 40 min. Flowrate was 0.5 mL/min. The main

peak purity was determined from the integrated chromatogram as the area under the main peak relative to the total peak area. The purity loss was calculated as the difference between the T0 and T14 time point and/or as the difference between the T0 and T28 time point.

Size exclusion chromatography (SEC) was used as an analysis for the formation of high molecular weight products (HMWP, covalent dimers, trimers etc.,). SEC analyses was performed on Thermo Dionex Ultimate UHPLC system with UV detection at 215 nm) equipped with a SEC column (Waters BEH 125Å, 1.7 μm, 300mm x 4.6). Analysis was performed by isocratic elution using 60% buffer B (0.1% TFA in 5:95 $H_2O$: acetonitrile) in buffer A (0.1% TFA in 95:5 $H_2O$: acetonitrile) at a column temperature of 60° C over 20 min. Flowrate was 0.3 mL/min.

Chromatograms were integrated as HMWP (all peaks eluting before the main peak), main peak (assuming this is monomeric peptide) and if relevant LMWP (low molecular weight products, all peaks eluting after main peak). The amount of HMWP was reported as area relative to total area in percent.

*Dynamic Light Scattering (DLS) studies of peptide oligomerisation/particle formation*

Samples were prepared by dissolving 800 nmol of each peptide in 400 μl 50 mM phosphate pH 8.0 to a concentration of 2000 μM (ca 10 mg/ml). After fully dissolution for 2 hours on a rocking table, samples were adjusted to pH 7.5 and filtrated through a 0.2 μm Whatman Anotop filter. Comparators were commercially available liraglutide drug product (Victoza®, Novo Nordisk) pH 8.2, and an aliquot of this drug product adjusted to pH 6.7. These samples were filtrated through a 0.02 μm Whatman Anotop filter.

Samples were pipetted as triplicate of 30 μl to an Aurora 384 well microtiter plate, which was centrifuged for 2 minutes at 2200 rpm and then sealed with a Thermo sealing tape 235307. DLS measurements were done in a Wyatt DynaPro platereader III with the following parameters:

| Parameter | Value |
|---|---|
| DLS acquisition time | 10 sec |
| DLS acquisitions per measurement | 10 |
| Measurements per well within a scan | 1 |
| Number of scans | 1 |
| Start temperature | 25°C |
| Plate sealant | Sealing tape |
| Set temperature | 25°C |
| Laser on | Yes |

The microtiter plate was incubated in a Grant-bio Thermo Shaker PHMP-4 at 40°C with 700 rpm continuous shaking before subsequent DLS measurements. Data analysis was done using Wyatt Dynamics software ver. 7.10.1.21.

*PK in mice*

Lean male NMRI/RjHan mice were obtained from JanVier (JanVier Labs, France) at 6 weeks of age. The animals were single housed under a 12/12 h dark-light cycle, light off at 3 PM. Room temperature was controlled to 22°C ± 1°C, with 60 % ± 20% humidity. Animals had ad libitum access to regular rodent chow (Altromin 1324, Brogaarden, Denmark) and tap water.

After 2 weeks of acclimatization, the animals were randomized into treatment groups (n=9 per group) based on body weight. Animals were dosed IV with one peptide and SC with another peptide (15 or 50 nmol/kg) Plasma samples were collected for PK analysis at times 0.17, 1, 3, 6, 24, 30, 48, 72 and 96 hours post dosing in a sparse sampling design with 3 mice per group per timepoint. Plasma samples were analyzed using LC-MSMS and PK parameters were estimated by Non-Compartmental Analysis (NCA).

*PK in rats*

Lean male Sprague Dawley rats (RJHan:SD) were obtained from JanVier (JanVier Labs, France) at 7 weeks of age. The animals were pair housed until randomization under a 12/12 h dark-light cycle, light off at 3 PM. Room temperature was controlled to 22°C ± 1°C, with 60 % ± 20% humidity.

Animals had ad libitum access to regular rodent chow (Altromin 1324, Brogaarden, Denmark) and tap water.

After 2 weeks of acclimatization, the animals were single housed and randomized into treatment groups (n = 3 per group) based on body weight on study day -3. On study day 1, animals were dosed 50 nmol/kg IV and SC in combination of two peptides. Plasma samples were collected for PK analysis at times 0.17, 1, 3, 6, 24, 30, 48, 72 and 96 hours post dosing. Plasma samples were analyzed using LC-MSMS and PK parameters were estimated by Non-Compartmental Analysis (NCA).

*PK in minipigs*

Pharmacokinetics studies were determined in male Göttingen minipigs from Ellegaard Göttingen Minipigs A/S. Animals were acclimatised for at least 7 days before placed on study. The minipigs had prior to study been surgically implanted with central venous catheters inserted which were used for blood sampling. Animals were housed under a 12/12 h dark-light cycle with ad libitum access to domestic water and animals were fed a commercially standard maintenance diet twice daily.

Test compounds where administrated either as a cassette or as a single compound. Test compounds were administrated by a single intravenous injection or short infusion (5-10 min) through the implanted catheter or a single subcutaneously injection. The subcutaneously injection was given in the mid neck between ear and scapula with a stopper on the needle, allowing 0.5 cm of the needle to be inserted.Plasma concentration-time profiles employing 12-16 sampling points were obtained from each animal. As an example, blood samples were collected on the following times: 0 (predose), 0.17, 0.33, 0.5, 1, 2, 5, 6, 24, 48, 72, 92, 168, 240 and 336 hrs post administration. Approximately 2 mL blood was drawn from the central catheter from each animal at each sampling point. Blood samples were taken in K2 or K3 EDTA tubes. Blood was kept on ice for maximum 30 min before centrifugation (10 min, 4°C, 2000 x g). Approximately 200 μL plasma samples was transferred to a Micronic tube and stored at -20° or lower.

Pharmacokinetics parameters were calculated by non-compartmental analysis (NCA) of the individual plasma concentrations-time profile from the animals. The log-linear trapezoidal method was used for estimation of AUC and AUMC. The terminal T½ was determined as $\ln(2)/\lambda z$ where $\lambda z$ is the first order rate constant as determined by the terminal log-linear part of the curve.

Plasma concentrations were measured by LC-MSMS using electrospray ionization and multiple reaction monitoring. Calibration standards and quality control (QC) samples were prepared in specie match matrix. 15 μL calibration standards, QC and study samples were extracted by protein precipitation using 60 μL methanol followed by addition of 45 μL milliQ water. Samples were shaken (800 rpm) at room temperature for 5 min before centrifugation (2570×g, 40 min, 4°C) and the supernatant was transferred to a LoBind PCR plate. Samples were analysed on a Thermo Triscend UHPLC system coupled to a Sciex API 6500+ mass spectrometer. Samples were subject to online SPE clean-up on a HLB column (1×50 mm, Waters) before loaded onto a Aeris Peptide XB C18 column (3.6 μm, 100 Å, 2×50 mm, Phenomenex) analytical column. The mobile phases consisted of acetonitrile and milliQ H2O both containing 0.1% V/V formic acid. The flow rate was 0.60 mL/min and the column was kept at room temperature.

*Effect on food intake*

Male NMRI mice were obtained from JanVier (JanVier Labs, France) at 5 weeks of age. The animals were group-housed 4 mice pr. cage under a 12/12 h dark-light cycle, light off at 1 PM. Room temperature was controlled to 22°C ± 1°C, with 60 % ± 20% humidity. Animals had ad libitum access to regular rodent chow (Altromin 1324, Brogaarden, Denmark) and tap water.

Animals were transferred 5-7 days before the start of the study to a real-time food intake monitoring system, HM-2 system (MBRose, Denmark) to allow acclimatization to experimental conditions. As the animals were uniquely identified with microchips, each individual animal was identified by its microchip upon entry and exit from the food channel. Randomization of the mice for each study group (n=7-8) was based on body weight measured the day before the start of the study. A vehicle-treated group was included in each experiment. Six hours before the start of the dark phase, animals were fasted. One hour before the dark phase, animals were dosed once subcutaneously with test peptide. Food intake was reported hourly for a period of 72 hours. The percentage of food intake reduction was normalized to average food intake from the vehicle group. Statistical significance was evaluated using One-way analysis of variance with Dunnett's multiple comparison test. $P < 0.05$ was considered statistically significant.

*Effect on body mass and body fat content in a mouse model of diet-induced obesity (DIO)*

SEQ ID NO: 3, SEQ ID NO: 6, SEQ ID NO: 238, SEQ ID NO: 16 and SEQ ID NO: 215 were assessed for their ability to reduce body mass and body fat content in a mouse model of diet-induced obesity (DIO).

Male C57BL/6JRj mice were obtained from JanVier (JanVier Labs, France) at 5 weeks of age. The animals were group-housed during obesity induction and then single housed 2 weeks prior to study start. They were housed under a

12/12 h dark-light cycle, light off at 3 PM. Room temperature was controlled to 22°C ± 1°C, with 60 % ± 20% humidity. For 22-23 weeks prior to treatment start and throughout the study, the animals had ad libitum access to high-fat diet (60% kcal fat, D12492, Research Diets) and tap water.

The animals were randomized according to lean mass (EchoMRI) and body weight. Animals were dosed subcutaneously once for 30-32 days. In study 1, the animals were dosed with vehicle or SEQ ID NO: 3 (10, 30, 60 or 100 nmol/kg). In study 2, the animals were dosed with vehicle or SEQ ID NO: 6 (10 or 30 nmol/kg), SEQ ID NO: 238 (10 or 30 nmol/kg), SEQ ID NO: 16 (10 or 30 nmol/kg), SEQ ID NO: 215 (10 or 30 nmol/kg) or SEQ ID NO: 3 (30 nmol/kg).

Body weight and food intake were recorded daily until day 28 and body composition evaluated on day 26 using EchoMRI. At termination, blood for HbA1c analyses were collected and M. gastrocnemius and M. soleus were weighed.

Statistical significance was evaluated using One-way analysis of variance with Dunnett's multiple comparison test. $P < 0.05$ was considered statistically significant.

*Telemetry in mice*

Blood pressure and heart frequency were monitored in freely moving conscious mice by radiotelemetry (PA-C10, Data Sciences). Briefly, analgesia with metamizole (100 mg / kg sc) was performed. For implantation of the telemetry transmitter, the animals were anesthetized with isoflurane with 5% induction and 2% maintenance. The neck was opened in the area of the hyoid bone up to the sternum in the median line. A subcutaneous pocket for the transmitter housing was prepared. The dextra carotid artery was then bluntly exposed and clamped. The sender was pushed caudally into the carotid artery through an incision. The catheter was fixed with a monofilament prolene thread, which also closed the incision. The vessel was also ligated cranially, and the clamp was then removed. The transmitter housing was pushed subcutaneously into a prepared skin pocket on the side opposite the catheter. Finally, the skin wound was closed. Postoperatively, an antibiotic (Ursocyclin® 10, Serumwerk Bernburg AG, Germany; 400 mg/l drinking water for 10 days) and an analgesic (Rimadyl®, 4 mg/kg sc for 3 days) were administered postoperatively.

For arterial blood pressure measurements, the animals were kept in a 12h: 12h day night cycle in a suitable room for at least one day before the first measurement. Animals were administrated s.c once daily in the morning for 7 consecutive days with either 1) Placebo, 2) SEQ ID NO: 3, 10 nmol/kg, 3) SEQ ID NO: 3, 30 nmol/kg or 4) SEQ ID NO: 3, 100 nmol/kg in a cross over design with 2 animals per group (n = 6-8) per week and with 1 week washout between treatments. The telemetric measurement took place over 24 hours. The averaged data (1 min to 10 min grid) were displayed graphically.

For statistical analysis, 5 hours means were calculated starting from one hour after dosing and one hour after lights off. All data were evaluated using a 2-way ANOVA with Dunnett's test.

*Telemetry in rats*

Blood pressure and heart rate were monitored in freely moving conscious animals by radiometry (DSI Data Science International, MN, USA). Female adult hypertensive rats (SHR) were implanted with transmitters. After shaving the abdominal wall, a midline abdominal incision was made, and the fluid-filled sensor catheter was inserted upstream into the exposed descending aorta between the iliac bifurcation and the renal arteries. According to the DSI guidelines the tip of the telemetric catheter was located just caudal to the renal arteries and secured by tissue adhesive. The transmitter body was affixed to the inner peritoneal - 3 - wall before closure of abdomen. A two-layer closure of the abdominal incision was used, with individual suturing of the peritoneum and the muscle wall followed by closure of the outer skin. Surgery was performed under aseptic conditions. For postsurgical protection against infections and pain a single dosage of an antibiotic (Ursocyclin 10% pro inj., Serumwerk, s.c.) and analgesic were injected (Rimadyl®, 4 mg/kg s.c., Pfizer, Germany). All animals were single-housed at 22-24°C ambient temperature and maintained on a 12/12 h dark-light cycle with free access to standard laboratory rat chow and water ad libitum.

All animals were treated with daily s.c. doses of vehicle or SEQ ID NO: 3, (10 or 30 nmol/kg) for 7 days (1 ml/kg). Drug administration took place at 9.00 a.m. (= 0 hours). For analysis, data were grouped to provide half-hourly averages.

For statistical analysis, 24 hours means were calculated starting from time of dosing. All data were evaluated using a 2-way ANOVA with Dunnett's test.

*Telemetry in pigs*

PK/PD/safety properties of SEQ ID NO: 3 were evaluated in freely moving conscious healthy pigs by radiometry.

In study part A, the effects of an ascending dose regime of SEQ ID NO: 3, (0.03, 0.1, 0.3 and 1.0 mg/kg) was compared to placebo. Primary endpoints were blood pressure, heart rate and ECG measurements.

In study part B, the effect of repeated dosing (once weekly, for 4 weeks) with a single dose of SEQ ID NO: 3 (0.3 mg/kg) was compared to placebo. Primary endpoints were blood pressure, heart rate and ECG measurements.

*Acute effects of selected peptide on hemodynamics in anesthetized pigs*

Göttingen minipigs were anesthetized and instrumented to continuous monitor hemodynamics during baseline measurement (0.5h), vehicle treatment (0.5h) and ascending doses of SEQ ID NO: 215 each 0.5 hours (10, 30, 100 and 300 μg/kg/min) and compared to a similar setup using Dobutamine (1, 3, 10 and 20 μg/kg/min) and vehicle (NaCl).

*Myocardial ischemia model in rats*

Wistar male rats were obtained from JanVier (Janvier Labs, France) at 6 weeks of age. The animals were pair-housed. During the study, animals had free access to food (RM1, SDS Dietex) and drinking water ad libitum.

Myocardial infarction (MI) was induced by chronic left anterior descending coronary artery (LAD) ligation performed at day zero. Sham operated animals were subjected to the same protocol; after the left lateral thoracotomy exposing the heart, the rib cage was closed without passing the suture thread around LAD.

Treatments started at 1-month post-MI, in order to assess the potential beneficial effect of the candidate compound to prevent the progression of the pathology.

Rats underwent transthoracic echocardiographic (ECG) examination in order to assess cardiac morphology and function in a noninvasively way. Echocardiography was performed by using a digital ultrasound system (Vivid S60, GE Medical Systems) equipped with a 12 MHz phased-array and an 18 MHz linear-array transducer. Standard B-mode (Brightness-mode) and M-mode (Motion-mode) images of the heart were obtained in the two-dimensional (2D) parasternal long axis view (PSLA). LV parameters were measured and calculated as the mean of 3 consecutive cardiac cycles by a single blinded trained operator.

A total of four ECG exams were performed for all the animals. The first examination took place 5-7 days after surgery and was used as a control of the surgery. The second ECG was performed 1 month after LAD ligation. Based on the second ECG, MI rats included were randomized in 4 homogeneous groups based on the left ventricular internal diameter in diastole (LVIDd), telediastolic (TeleD) volume, ejection fraction (EF) and fractional shortening (FS): Group 1 (Sham): n=10, group 2 (MI/Vehicle): n=19, group 3 (MI/Atenolol-Lisinopril-Spironolactone (A+L+S) 1-1-10 mg/kg): n=19, group 4 (MI/test peptide 30 nmol/kg): n=19 and group 5 (MI/test peptide 100 nmol/kg): n=19. Vehicle and test peptide were administrated daily SC and A+L+S were dosed through drinking water. The third and fourth ECG were done 2 and 3 months after surgery, and were used to assess the cardiac remodelling and function following MI. At the fourth ECG isovolumic relaxation time (IVRT) and cardiac output (CO) were also assessed by Doppler-echocardiography.

Prior to termination, hemodynamic measurements were performed under anaesthesia using a fluid-filled catheter (BLPR and TBM4m, World Precision Instrument). At termination, left soleus was dissected and weighed.

Statistical analyses were performed with the Graphpad 9 software. If values were normally distributed, a parametric analysis was performed. If samples were drawn from non-normal populations, a non-parametric analysis was performed. First, differences were assessed between Sham and MI/Vehicle using a t-test. Then, the treated MI groups were compared to MI/Vehicle using a one-way ANOVA followed by the appropriate post-hoc test.

*Subcronich IRI*

Male C57BL/6jRj mice were obtained from JanVier (JanVier Labs, France) at 10 weeks of age. The animals were single-housed in Individually Ventilated Cages (IVC from Tecniplast, Typ I SL cage) with free access to water, food and encrichments. Animals were allowed to acclimatize for 7 days prior to study start. After acclimatization, animals were randomized into groups based on body weight; 1) Sham, 2) uIRI - Vehicle, 3) uIRI -SEQ ID NO: 3, 30 nmol/kg and 4) uIRI -SEQ ID NO: 3, 100 nmol/kg.

On study day 0, unilateral ischemia-reperfusion injury (uIRI, 25 min of occlusion followed by reperfusion) was performed on study group 2 - 4 (followed by uninephrectomy (UNx) on study day 6. Animals were subcutaneously dosed once daily starting prior to IRI surgery and until study day 7, where animals were terminated. Here plasma was collected, and the remaining kidney weighed and sampled for histological analysis. Kidneys were stained, analyzed and quantified using the following markers: collagen type-1 (col1a1), F4/80 and KIM-1.

**Example 1: Glutamate scan - Exploration of solubility and hCRHR2/hCRHR1 potency**

To identify the optimal sites for improvement of the solubility, without adversely affecting hCRHR2 potency and selectivity, a library of 190 peptides were synthesised, wherein 1-6 glutamate residues were introduced compared to a lipidated version of native UCN2 (SEQ ID NO: 2).

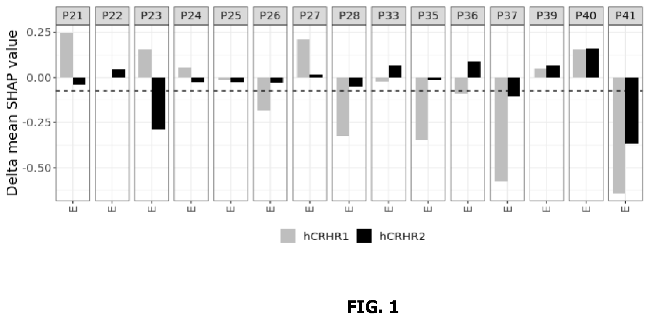

The $EC_{50}$ values on hCRHR2 and hCRHR1 were determined and SHAP values calculated from a random forest model, where $pEC_{50}$ values were fitted to the peptide amino acid sequences. Delta mean SHAP values were used to determine the level of contribution of each glutamate substitution relative to the native UCN2 residue on hCRHR2 and

hCRHR1 potency (Breiman, L. (2001), Random Forests, Machine Learning 45(1), 5-32.; Lundberg, S. M., & Lee, S. I. (2017). A unified approach to interpreting model predictions. Advances in neural information processing systems, 30.). Substitutions with positive delta mean SHAP values increased the end-point relative to the native UCN2 residue, while negative delta mean SHAP values decreased the end-point relative to the native UCN2 residue. Fig. 1 summarizes the results. Positions $X^{21}$, $X^{22}$, $X^{24}$, $X^{25}$, $X^{26}$, $X^{27}$, $X^{28}$, $X^{33}$, $X^{35}$,$X^{36}$, $X^{39}$, and $X^{40}$ were identified as suitable positions to incorporate glutamate residues (E) to increase the solubility without major adverse effects on hCRHR2 potency and selectivity. In particular, the positions $X^{22}$, $X^{33}$, $X^{36}$, $X^{39}$ and $X^{40}$ were identified as positions to incorporate glutamate residues to increase solubility while preserving or improving hCRHR2 potency and selectivity. Positions $X^{36}$ and $X^{40}$ were identified as the best positions to incorporate glutamate residues to increase solubility with a concomitant improvement of hCRHR2 potency.

**Example 2: Lipidation scan - Exploration of lipidation site and hCRHR2/hCRHR1 potency**

To identify the optimal lipidation sites, a lipidation scan was conducted on the UCN2 backbone having $X^{36}$=E and $X^{40}$=E (SEQ ID NO: 228), by introducing various lipidated lysine (K) residues in each of the positions $X^4$-$X^{41}$. The following lipidation strategies were used: C18DA-yGlu, C18DA-γGlu-OEG-OEG, C18DA-γGlu-γGlu-OEG-OEG, C20DA-gGlu, C20DA-γGlu-OEG-OEG, or C20DA-γGlu-γGlu-OEG-OEG.

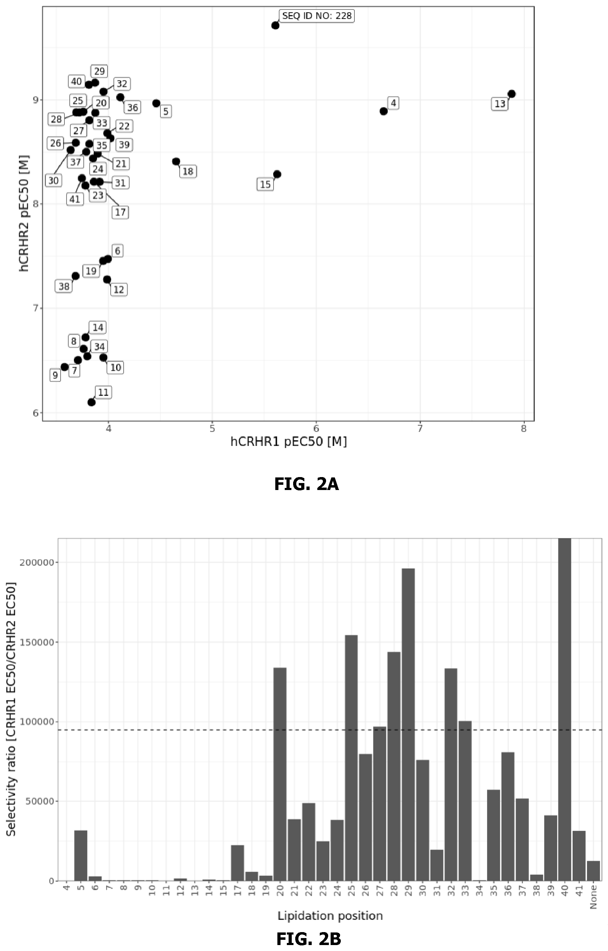

Fig. 2A shows the hCRHR1 and hCRHR2 potency for each lipidation site averaged across different lipidation strategies. The lipidation sites clustered in the upper left corner of Fig. 2A provides the highest hCRHR2 potency and the lowest hCRHR1 potency. Fig. 2B depicts the selectivity ratio for each lipidation site for hCRHR2 over hCRHR1 (i.e. hCRHR1 $EC_{50}$/hCRHR2 $EC_{50}$) averaged across lipidation strategies. Fig. 2B shows that positions $X^{20}$, $X^{25}$, $X^{27}$, $X^{28}$, $X^{29}$, $X^{32}$, $X^{33}$ and $X^{40}$ were identified as the most optimal sites for lipidation providing the highest selectivity ratio for hCRHR2 over hCRHR1. Most preferably, $X^{32}$ or $X^{33}$ is used as lipidation site.

Table 1 illustrates that lipidation in position $X^{32}$ or $X^{33}$ maintains high hCRHR2 potency and provides superior selectivity for hCRHR2 compared to native UCN2. Table 1 further demonstrates that glutamates (E), for increased solubility, in the most preferred positions $X^{36}$ and $X^{40}$ maintain high hCRHR2 potency and high selectivity for hCRHR2 over hCRHR1. N-terminal mono-methylation of $X^4$ or the substitution of $X^4$ = P were found to have minor impact on hCRHR2 potency, with N-terminal mono-methylation of $X^4$, i.e. $X^4$ = MeI, impacting hCRHR2 potency the least. Further, data confirms that desirable high solubility may be achieved by incorporating at least two glutamates into lipidated UCN2. All peptides were found not to fibrillate. Interestingly, the peptides SEQ ID NO: 3 and SEQ ID NO: 4 possessed a much higher selectivity ratio compared to the prior art peptides SEQ ID NO: 9, SEQ ID NO: 10 and SEQ ID NO: 11 disclosed in WO 2018/013803 A1 and WO 2023/285334 A1. This illustrates that two glutamates in positions $X^{36}$ and $X^{40}$, to improve solubility, with a lipidation in position $X^{32}$ or $X^{33}$, to alter PK properties, provide superior selectivity compared to prior art peptides.

Table 1 further shows that the lipidation in position $X^{15}$ (SEQ ID NO: 261 in Table 1) as used in WO 2022/038179 A1 (here position $X^{12}$) provided poor hCRHR2 potency.

**Table 1**

| SEQ ID NO: | Substitutions relative to native UCN2 | hCRHR2 $EC_{50}$ (nM) | hCRHR1 $EC_{50}$ (nM) | Selectivity ratio (hCRHR1/ hCRHR2) | Chemical stability at pH 7.5 after 14 days/28 days at 40°C (% degradation ) | Fibrillation (pH 7.5) | Solubility pH 7.5, target conc. =5 mg/mL (measured mg/mL) | Solubility pH 7.5, target conc. =10 mg/mL (measured mg/mL) |
|---|---|---|---|---|---|---|---|---|
| (native UCN2) 1 | - | 0.24 | 220 | 917 | ND | No | ND | ND |
| 2 | $X^{32}$=K(lip) | 1.8 | >5000 | >2778 | ND | No | 0.016 | ND |
| 3 | $X^{32}$=K(lip); $X^{36}$=E; $X^{40}$=E | 1.1 | >5000 | >4546 | -6.1 / -15 | No | 4.5 | 10 |
| 4 | $X^{33}$=K(lip); $X^{36}$=E; $X^{40}$=E | 1.3 | >5000 | >3846 | -1.3 / -3.9 | No | ND | 9.1 |
| 5 | $X^4$=MeI; $X^{32}$=K(lip); $X^{36}$=E; $X^{40}$=E | 1.2 | >5000 | >4167 | - 5.4 / ND | No | ND | 11 |

(continued)

Table 1

| SEQ ID NO: | Substitutions relative to native UCN2 | hCRHR2 $EC_{50}$ (nM) | hCRHR1 $EC_{50}$ (nM) | Selectivity ratio (hCRHR1/ hCRHR2) | Chemical stability at pH 7.5 after 14 days/28 days at 40°C (% degradation ) | Fibrillation (pH 7.5) | Solubility pH 7.5, target conc. =5 mg/mL (measured mg/mL) | Solubility pH 7.5, target conc. =10 mg/mL (measured mg/mL) |
|---|---|---|---|---|---|---|---|---|
| 6 | $X^{4}$=MeI; $X^{33}$=K(lip); $X^{36}$=E; $X^{40}$=E | 2.7 | >5000 | >1852 | -1.0 / -3.5 | No | ND | 11 |
| 7 | $X^{4}$=P; $X^{32}$=K(lip); $X^{36}$=E; $X^{40}$=E | 2.5 | >5000 | >2000 | - 11 / -23 | No | ND | 9.8 |
| 8 | $X^{4}$=AcI; $X^{32}$=K(lip); $X^{36}$=E; $X^{40}$=E | 1.4 | 560 | 400 | ND | ND | ND | ND |
| 9 | $X^{4}$=MeI; $X^{22}$=E; $X^{23}$=K; $X^{24}$=Q; $X^{25}$=E; $X^{26}$= K; $X^{27}$=E; $X^{28}$=K; $X^{29}$=Q; $X^{32}$=K(lip); $X^{36}$=Q; $X^{40}$=Q | 1.3 | 510 | 392 | ND | ND | ND | ND |
| 10 | $X^{4}$=MeI; $X^{6}$=T; $X^{22}$=E; $X^{23}$=K; $X^{24}$=Q; $X^{25}$=E; $X^{26}$=K; $X^{27}$=E; $X^{28}$=K; $X^{29}$=Q; $X^{32}$=K(lip); $X^{36}$=E; $X^{40}$=Q | 0.49 | 68 | 139 | ND | ND | ND | ND |
| 11 | $X^{3}$=K(lip); $X^{36}$=E; $X^{40}$=E | 1.6 | 670 | 419 | ND | ND | ND | ND |
| 260 | N.term.= lip; $X^{36}$=E; $X^{40}$=E | 1.4 | 110 | 79 | ND | ND | ND | ND |
| 261 | $X^{15}$=K(lip); $X^{36}$=E; $X^{40}$=E | 24 | >5000 | >208 | ND | ND | ND | ND |

**Example 3: Chemical stability - Identification of sites and substitutions to improve chemical stability**

Position $X^{34}$ was identified as a chemical labile residue in the UCN2 backbone. In order to improve the chemical stability of the peptides, compounds were synthesized, wherein the amino acid threonine (T), present in UCN2, in position $X^{33}$ or the amino acid alanine (A), present in UCN2, in position $X^{35}$ was substituted (see Table 2). The effect of each substitution is shown in relation to reference 1 (i.e. UCN2 with $X^{32}$ = K(Lip); $X^{36}$ and $X^{40}$ = E).

| Table 2 | | | | | | | |
|---|---|---|---|---|---|---|---|
| SEQ ID NO: | Substitutions relative to reference 1 | hCRHR2 $EC_{50}$ (nM) | hCRHR1 $EC_{50}$ (nM) | Selectivity ratio (hCRHR1/ hCRHR2) | Chemical stability at pH 7.5 after 14 days/28 days at 40°C (% degradation) | Fibrillation (pH 7.5) | Solubility pH 7.5, target conc. =10 mg/mL (measured mg/mL) |
| 3 (Lipidated UCN2 with $X^{36}$ = E; $X^{40}$ = E) (reference 1) | - | 1.1 | >5000 | >4546 | -6.1 / -15 | No | 10 |
| 12 | $X^{35}$ = L | 4.4 | >5000 | >1136 | -0.6 / -1.2 | No | 9.1 |
| 13 | $X^{35}$ = I | 5.4 | >5000 | >926 | -0.3 / -1.6 | No | 8.9 |
| 14 | $X^{35}$ = V | 5.0 | >5000 | > 1000 | -0.3 / -1.2 | No | 9.2 |
| 15 | $X^{35}$ = Aib | 2.3 | >5000 | >2174 | -1.0 / -1.2 | No | 9.8 |
| 16 | $X^4$=MeI; $X^{35}$=Aib | 2.7 | >5000 | >1852 | -0.6 / | No | 10 |
| 17 | $X^{33}$ = E | 2.1 | >5000 | >2381 | -1.6 / -2.6 | No | 9.2 |
| 18 | $X^4$=MeI; $X^{33}$ = E | 1.4 | >5000 | >3571 | -2.2 / ND | No | 11 |
| 19 | $X^{33}$ = Aib | 1.6 | >5000 | >3125 | -1.5 / -2.6 | No | 9.6 |
| 20 | $X^4$=MeI; $X^{33}$ = Aib | 1.2 | >5000 | >4167 | -1.4 / ND | No | 10 |
| 6 | $X^{32}$=T, $X^{33}$=K(Lip) | 2.7 | >5000 | >1852 | -1.0 / -3.5 | No | 9.8 |

As shown in Table 2, substitution of the amino acid alanine (A) in position $X^{35}$ with the amino acid L, I, V, or Aib or substitution of the amino acid threonine (T) in position $X^{33}$ with E, Aib or K(Lip) resulted in a significant improvement in chemical stability with no impact on potency or only a minor sacrifice of potency compared to reference 1. Furthermore, all peptides maintained high selectivity ratios superior to native UCN2. Also, the data demonstrates that high physical stability (i.e. no fibrillation) and solubility is preserved when substituting position $X^{33}$ or position $X^{35}$.

Thus, it is highly preferred that $X^{35}$ is selected as L, I, V, or Aib to provide UCN2 analogues with improved chemical stability. Alternatively, it is highly preferred that $X^{33}$ is selected as E, Aib or K(Lip) to provide UCN2 analogues with improved chemical stability.

**Example 4: Improvement of hCRHR2 potency**

To identify the sites for improvement of hCRHR2 potency, a library of 1140 peptides was designed. The library was based on the lipidated UCN2 analogue, reference 1, SEQ ID NO: 3, and the aim was to improve potency by optimizing either (1) the hCRHR2 binding pocket interactions with different amino acid residues in positions $X^6$, $X^{10}$, $X^{11}$ and $X^{12}$, (2) the bend region in UCN2 in position $X^{20-27}$, or (3) the hCRHR2-ECD:UCN2 interface by introducing different amino acid residues in positions $X^{25-27}$, $X^{29-30}$, $X^{38}$ and $X^{41}$. The tested amino acid residues investigated in each position are summarized in Fig. 3.

The $EC_{50}$ values on hCRHR2 and hCRHR1 were determined for each library and SHAP values calculated from a random forest model where $pEC_{50}$ values were fitted to the peptide amino acid sequences. Delta mean SHAP values were used to determine the level of contribution of each amino acid substitution relative to the corresponding native UCN2 residue on hCRHR2 and hCRHR1 potency (Breiman, L. (2001), Random Forests, Machine Learning 45(1), 5-32.; Lundberg, S. M., & Lee, S. I. (2017). A unified approach to interpreting model predictions. Advances in neural information processing systems, 30.). Substitutions with positive delta mean SHAP values increased the end-point relative to the

native UCN2 residue, while negative delta mean SHAP values decreased the end-point relative to the native UCN2 residue. The contribution of each amino acid is summarized in Fig. 3.

Fig. 3 shows that the majority of amino acids tested resulted in decreased or unchanged hCRHR2 potency relative to the native UCN2 residue, with the exception of amino acid positions $X^6$, $X^{10}$, $X^{11}$, $X^{12}$, and $X^{38}$, where threonine (T), cycloleucine (Cle), hydroxyproline (Hyp), leucine (L), and norleucine (Nle), respectively, had a positive effect (i.e. increased) hCRHR2 $pEC_{50}$. However, these substitutions were also found to increase hCRHR1 $pEC_{50}$ to a greater extent than hCRHR2 $pEC_{50}$, thereby decreasing overall selectivity.

However, as shown in Fig. 3, some substitutions in positions $X^{20}$, $X^{21}$, $X^{26}$, $X^{30}$ and $X^{41}$ were found to decrease hCRHR1 $pEC_{50}$ while hCRHR2 $pEC_{50}$ was unchanged. For position $X^{20}$, asparagine (N), serine (S) and threonine (T) were all found to decrease hCRHR1 $pEC_{50}$ without compromising hCRHR2 $pEC_{50}$. For position $X^{21}$, leucine (L); for position $X^{26}$, leucine (L); for position $X^{30}$, arginine (R), and for position $X^{41}$, isoleucine(I), were found to decrease hCRHR1 $pEC_{50}$ without compromising hCRHR2 $pEC_{50}$. Thus, positions $X^{20}$, $X^{21}$, $X^{26}$ and/or $X^{41}$ may be used to regain high selectivity when potency improving substitutions are introduced e.g. Nle in position $X^{38}$ (see Example 5).

Table 3 shows matched molecular pairs and the improved potency obtained by substituting the leucine (L) present in position $X^{38}$ of UCN2 with the amino acid norleucine (Nle). As can be seen, the substitution of L with Nle improved hCRHR2 potency but decreased the selectivity when positions $X^{20}$ and $X^{41}$ were the natural amino acid present in UCN2 (i.e. $X^{20}$ = E and $X^{41}$ = V).

Table 3

| | Amino acid positions | | | | | | | | | | | | | | | | | | | | | | | | | | | | | | | | | | | | | | Potency | |
|---|---|---|---|---|---|---|---|---|---|---|---|---|---|---|---|---|---|---|---|---|---|---|---|---|---|---|---|---|---|---|---|---|---|---|---|---|---|---|---|---|
| SEQ ID NO: | $X^4$ | $X^5$ | $X^6$ | $X^7$ | $X^8$ | $X^9$ | $X^{10}$ | $X^{11}$ | $X^{12}$ | $X^{13}$ | $X^{14}$ | $X^{15}$ | $X^{16}$ | $X^{17}$ | $X^{18}$ | $X^{19}$ | $X^{20}$ | $X^{21}$ | $X^{22}$ | $X^{23}$ | $X^{24}$ | $X^{25}$ | $X^{26}$ | $X^{27}$ | $X^{28}$ | $X^{29}$ | $X^{10}$ | $X^{31}$ | $X^{32}$ | $X^{33}$ | $X^{34}$ | $X^{35}$ | $X^{36}$ | $X^{37}$ | $X^{38}$ | $X^{39}$ | $X^{40}$ | $X^{41}$ | hCRHR2 $pEC_{50}$ | hCRHR1 $pEC_{50}$ |
| 21 | I | V | L | S | L | D | V | P | I | G | L | L | Q | I | L | L | E | Q | A | R | A | E | S | A | R | E | R | A | K* | T | N | A | E | I | L | A | E | I | 9.1 | 4.2 |
| 22 | I | V | L | S | L | D | V | P | I | G | L | L | Q | I | L | L | E | Q | A | R | A | E | S | A | R | E | R | A | K* | T | N | A | E | I | Nle | A | E | I | 9.8 | 5.3 |
| 23 | I | V | L | S | L | D | V | P | I | G | L | L | Q | I | L | L | E | Q | A | R | A | E | A | A | R | E | Q | A | K* | T | N | A | E | I | L | A | E | I | 9.3 | 4.4 |
| 24 | I | V | L | S | L | D | V | P | I | G | L | L | Q | I | L | L | E | Q | A | R | A | E | A | A | R | E | Q | A | K* | T | N | A | E | I | Nle | A | E | I | 9.4 | 6.0 |
| 25 | I | V | L | S | L | D | V | P | I | G | L | L | Q | I | L | L | E | Q | A | R | A | E | A | A | R | E | Q | A | K* | T | N | A | E | I | L | A | E | V | 9.1 | 5.2 |
| 26 | I | V | L | S | L | D | V | P | I | G | L | L | Q | I | L | L | E | Q | A | R | A | E | A | A | R | E | Q | A | K* | T | N | A | E | I | Nle | A | E | V | 9.5 | 6.6 |
| 27 | I | V | L | S | L | D | V | P | I | G | L | L | Q | I | L | L | E | Q | A | R | A | R | A | A | R | Q | Q | A | K* | T | N | A | E | I | L | A | E | I | 9.5 | 4.2 |
| 28 | I | V | L | S | L | D | V | P | I | G | L | L | Q | I | L | L | E | Q | A | R | A | R | A | A | R | Q | Q | A | K* | T | N | A | E | I | Nle | A | E | I | 9.8 | 5.9 |
| 29 | I | V | L | S | L | D | V | P | I | G | L | L | Q | I | L | L | E | Q | A | R | A | R | Q | A | R | E | Q | A | K* | T | N | A | E | I | L | A | E | I | 9.6 | 4.4 |
| 30 | I | V | L | S | L | D | V | P | I | G | L | L | Q | I | L | L | E | Q | A | R | A | R | Q | A | R | E | Q | A | K* | T | N | A | E | I | Nle | A | E | I | 9.9 | 5.8 |
| 31 | I | V | L | S | L | D | V | P | I | G | L | L | Q | I | L | L | E | Q | A | R | A | Q | A | Q | R | E | Q | A | K* | T | N | A | E | I | L | A | E | V | 9.1 | 5.4 |
| 32 | I | V | L | S | L | D | V | P | I | G | L | L | Q | I | L | L | E | Q | A | R | A | Q | A | Q | R | E | Q | A | K* | T | N | A | E | I | Nle | A | E | V | 9.5 | 7.6 |
| 33 | I | V | L | S | L | D | V | P | I | G | L | L | Q | I | L | L | E | Q | A | R | A | R | A | Q | R | E | E | A | K* | T | N | A | E | I | L | A | E | V | 8.9 | 3.9 |
| 34 | I | V | L | S | L | D | V | P | I | G | L | L | Q | I | L | L | E | Q | A | R | A | R | A | Q | R | E | E | A | K* | T | N | A | E | I | Nle | A | E | V | 9.6 | 4.0 |
| 35 | I | V | L | S | L | D | V | P | I | G | L | L | Q | I | L | L | E | Q | A | R | A | R | S | A | R | E | Q | A | K* | T | N | A | E | I | L | A | E | I | 9.3 | 4.1 |
| 36 | I | V | L | S | L | D | V | P | I | G | L | L | Q | I | L | L | E | Q | A | R | A | R | S | A | R | E | Q | A | K* | T | N | A | E | I | Nle | A | E | I | 9.8 | 5.3 |
| 37 | I | V | L | S | L | D | V | P | I | G | L | L | Q | I | L | L | E | Q | A | R | A | R | A | L | R | Q | E | A | K* | T | N | A | E | I | L | A | E | V | 8.5 | 3.8 |
| 38 | I | V | L | S | L | D | V | P | I | G | L | L | Q | I | L | L | E | Q | A | R | A | R | A | L | R | Q | E | A | K* | T | N | A | E | I | Nle | A | E | V | 9.4 | 4.2 |
| 39 | I | V | L | S | L | D | V | P | I | G | L | L | Q | I | L | L | E | Q | A | R | A | R | A | L | R | E | Q | A | K* | T | N | A | E | I | L | A | E | V | 9.3 | 6.1 |
| 40 | I | V | L | S | L | D | V | P | I | G | L | L | Q | I | L | L | E | Q | A | R | A | R | A | L | R | E | Q | A | K* | T | N | A | E | I | Nle | A | E | V | 9.4 | 7.6 |
| 41 | I | V | L | S | L | D | V | P | I | G | L | L | Q | I | L | L | E | Q | A | R | A | E | S | A | R | E | Q | A | K* | T | N | A | E | I | L | A | E | V | 9.2 | 4.8 |
| 42 | I | V | L | S | L | D | V | P | I | G | L | L | Q | I | L | L | E | Q | A | R | A | E | S | A | R | E | Q | A | K* | T | N | A | E | I | Nle | A | E | V | 9.7 | 6.6 |
| 43 | I | V | L | S | L | D | V | P | I | G | L | L | Q | I | L | L | E | Q | A | R | A | Q | A | A | R | E | Q | A | K* | T | N | A | E | I | L | A | E | I | 9.3 | 4.2 |
| 44 | I | V | L | S | L | D | V | P | I | G | L | L | Q | I | L | L | E | Q | A | R | A | Q | A | A | R | E | Q | A | K* | T | N | A | E | I | Nle | A | E | I | 10 | 5.9 |
| 3 | I | V | L | S | L | D | V | P | I | G | L | L | Q | I | L | L | E | Q | A | R | A | R | A | A | R | E | Q | A | K* | T | N | A | E | I | L | A | E | V | 9.3 | 4.6 |
| 45 | I | V | L | S | L | D | V | P | I | G | L | L | Q | I | L | L | E | Q | A | R | A | R | A | A | R | E | Q | A | K* | T | N | A | E | I | Nle | A | E | V | 9.6 | 6.7 |
| 46 | I | V | L | S | L | D | V | P | I | G | L | L | Q | I | L | L | E | Q | A | R | A | R | A | Q | R | Q | Q | A | K* | T | N | A | E | I | L | A | E | V | 9.5 | 5.6 |
| 47 | I | V | L | S | L | D | V | P | I | G | L | L | Q | I | L | L | E | Q | A | R | A | R | A | Q | R | Q | Q | A | K* | T | N | A | E | I | Nle | A | E | V | 9.7 | 7.2 |

**Table 3**

| | Amino acid positions | | | | | | | | | | | | | | | | | | | | | | | | | | | | | | | | | | | | | | Potency | |
|---|---|---|---|---|---|---|---|---|---|---|---|---|---|---|---|---|---|---|---|---|---|---|---|---|---|---|---|---|---|---|---|---|---|---|---|---|---|---|---|---|
| SEQ ID NO: | $X^{4}$ | $X^{5}$ | $X^{6}$ | $X^{7}$ | $X^{8}$ | $X^{9}$ | $X^{10}$ | $X^{11}$ | $X^{12}$ | $X^{13}$ | $X^{14}$ | $X^{15}$ | $X^{16}$ | $X^{17}$ | $X^{18}$ | $X^{19}$ | $X^{20}$ | $X^{21}$ | $X^{22}$ | $X^{23}$ | $X^{24}$ | $X^{25}$ | $X^{26}$ | $X^{27}$ | $X^{28}$ | $X^{29}$ | $X^{10}$ | $X^{31}$ | $X^{32}$ | $X^{33}$ | $X^{34}$ | $X^{35}$ | $X^{36}$ | $X^{37}$ | $X^{38}$ | $X^{39}$ | $X^{40}$ | $X^{41}$ | hCRHR2 $pEC_{50}$ | hCRHR1 $pEC_{50}$ |
| 48 | I | V | L | S | L | D | V | P | I | G | L | L | Q | I | L | L | E | Q | A | R | A | Q | A | A | R | E | R | A | K* | T | N | A | E | I | L | A | E | V | 9.4 | 4.7 |
| 49 | I | V | L | S | L | D | V | P | I | G | L | L | Q | I | L | L | E | Q | A | R | A | Q | A | A | R | E | R | A | K* | T | N | A | E | I | Nle | A | E | V | 9.9 | 5.5 |
| 50 | I | V | L | S | L | D | V | P | I | G | L | L | Q | I | L | L | E | Q | A | R | A | R | A | L | R | E | E | A | K* | T | N | A | E | I | L | A | E | V | 8.7 | 3.8 |
| 51 | I | V | L | S | L | D | V | P | I | G | L | L | Q | I | L | L | E | Q | A | R | A | R | A | L | R | E | E | A | K* | T | N | A | E | I | Nle | A | E | V | 9.1 | 3.9 |
| 52 | I | V | L | S | L | D | V | P | I | G | L | L | Q | I | L | L | E | Q | A | R | A | R | Q | A | R | E | Q | A | K* | T | N | A | E | I | L | A | E | V | 9.2 | 5.8 |
| 53 | I | V | L | S | L | D | V | P | I | G | L | L | Q | I | L | L | E | Q | A | R | A | R | Q | A | R | E | Q | A | K* | T | N | A | E | I | Nle | A | E | V | 9.5 | 6.6 |
| 54 | I | V | L | S | L | D | V | P | I | G | L | L | Q | I | L | L | E | Q | A | R | A | Q | A | A | R | Q | Q | A | K* | T | N | A | E | I | L | A | E | V | 9.4 | 4.7 |
| 55 | I | V | L | S | L | D | V | P | I | G | L | L | Q | I | L | L | E | Q | A | R | A | Q | A | A | R | Q | Q | A | K* | T | N | A | E | I | Nle | A | E | V | 9.9 | 6.6 |
| 56 | I | V | L | S | L | D | V | P | I | G | L | L | Q | I | L | L | E | Q | A | R | A | R | A | Q | R | E | Q | A | K* | T | N | A | E | I | L | A | E | V | 9.4 | 5.5 |
| 57 | I | V | L | S | L | D | V | P | I | G | L | L | Q | I | L | L | E | Q | A | R | A | R | A | Q | R | E | Q | A | K* | T | N | A | E | I | Nle | A | E | V | 9.9 | 7.2 |
| 58 | I | V | L | S | L | D | V | P | I | G | L | L | Q | I | L | L | E | Q | A | R | A | E | A | L | R | Q | Q | A | K* | T | N | A | E | I | L | A | E | V | 9.5 | 7.0 |
| 59 | I | V | L | S | L | D | V | P | I | G | L | L | Q | I | L | L | E | Q | A | R | A | E | A | L | R | Q | Q | A | K* | T | N | A | E | I | Nle | A | E | V | 9.8 | 8.3 |
| 60 | I | V | L | S | L | D | V | P | I | G | L | L | Q | I | L | L | E | Q | A | R | A | R | S | A | R | E | Q | A | K* | T | N | A | E | I | L | A | E | V | 9.3 | 4.6 |
| 61 | I | V | L | S | L | D | V | P | I | G | L | L | Q | I | L | L | E | Q | A | R | A | R | S | A | R | E | Q | A | K* | T | N | A | E | I | Nle | A | E | V | 10 | 5.9 |
| 62 | I | V | L | S | L | D | V | P | I | G | L | L | Q | I | L | L | E | Q | A | R | A | Q | A | A | R | E | Q | A | K* | T | N | A | E | I | L | A | E | V | 9.5 | 4.6 |
| 63 | I | V | L | S | L | D | V | P | I | G | L | L | Q | I | L | L | E | Q | A | R | A | Q | A | A | R | E | Q | A | K* | T | N | A | E | I | Nle | A | E | V | 9.6 | 6.3 |
| 64 | I | V | L | S | L | D | V | P | I | G | L | L | Q | I | L | L | E | Q | A | R | A | R | A | Q | R | E | Q | A | K* | T | N | A | E | I | L | A | E | I | 9.9 | 4.6 |
| 65 | I | V | L | S | L | D | V | P | I | G | L | L | Q | I | L | L | E | Q | A | R | A | R | A | Q | R | E | Q | A | K* | T | N | A | E | I | Nle | A | E | I | 10 | 6.8 |
| 66 | I | V | L | S | L | D | V | P | I | G | L | L | Q | I | L | L | E | Q | A | R | A | R | A | A | R | E | R | A | K* | T | N | A | E | I | L | A | E | V | 9.5 | 4.3 |
| 67 | I | V | L | S | L | D | V | P | I | G | L | L | Q | I | L | L | E | Q | A | R | A | R | A | A | R | E | R | A | K* | T | N | A | E | I | Nle | A | E | V | 9.9 | 5.7 |
| 68 | I | V | L | S | L | D | V | P | I | G | L | L | Q | I | L | L | E | Q | A | R | A | R | A | A | R | E | Q | A | K* | T | N | A | E | I | L | A | E | I | 9.6 | 4.2 |
| 69 | I | V | L | S | L | D | V | P | I | G | L | L | Q | I | L | L | E | Q | A | R | A | R | A | A | R | E | Q | A | K* | T | N | A | E | I | Nle | A | E | I | 9.7 | 5.4 |
| 70 | I | V | L | S | L | D | V | P | I | G | L | L | Q | I | L | L | E | Q | A | R | A | R | S | Q | R | E | E | A | K* | T | N | A | E | I | L | A | E | V | 8.9 | 3.8 |
| 71 | I | V | L | S | L | D | V | P | I | G | L | L | Q | I | L | L | E | Q | A | R | A | R | S | Q | R | E | E | A | K* | T | N | A | E | I | Nle | A | E | V | 9.2 | 4.0 |
| 72 | I | V | L | S | L | D | V | P | I | G | L | L | Q | I | L | L | E | Q | A | R | A | R | Q | L | R | E | Q | A | K* | T | N | A | E | I | L | A | E | V | 9.5 | 6.6 |
| 73 | I | V | L | S | L | D | V | P | I | G | L | L | Q | I | L | L | E | Q | A | R | A | R | Q | L | R | E | Q | A | K* | T | N | A | E | I | Nle | A | E | V | 9.7 | 8.4 |
| 74 | I | V | L | S | L | D | V | P | I | G | L | L | Q | I | L | L | E | Q | A | R | A | R | A | A | R | E | E | A | K* | T | N | A | E | I | L | A | E | I | 8.7 | 3.8 |
| 75 | I | V | L | S | L | D | V | P | I | G | L | L | Q | I | L | L | E | Q | A | R | A | R | A | A | R | E | E | A | K* | T | N | A | E | I | Nle | A | E | I | 9.1 | 3.8 |
| 76 | I | V | L | S | L | D | V | c4NHPro | I | G | L | L | Q | I | L | L | E | Q | A | R | A | R | V | A | R | E | Q | A | K* | T | N | A | E | I | L | A | E | V | 8.8 | 8.8 |
| 77 | I | V | L | S | L | D | V | c4NHPro | I | G | L | L | Q | I | L | L | E | Q | A | R | A | R | V | A | R | E | Q | A | K* | T | N | A | E | I | Nle | A | E | V | 9.3 | 8.5 |

**Table 3**

| | Amino acid positions | | | | | | | | | | | | | | | | | | | | | | | | | | | | | | | | | | | | | | Potency | |
|---|---|---|---|---|---|---|---|---|---|---|---|---|---|---|---|---|---|---|---|---|---|---|---|---|---|---|---|---|---|---|---|---|---|---|---|---|---|---|---|---|
| SEQ ID NO: | $X^{4}$ | $X^{5}$ | $X^{6}$ | $X^{7}$ | $X^{8}$ | $X^{9}$ | $X^{10}$ | $X^{11}$ | $X^{12}$ | $X^{13}$ | $X^{14}$ | $X^{15}$ | $X^{16}$ | $X^{17}$ | $X^{18}$ | $X^{19}$ | $X^{20}$ | $X^{21}$ | $X^{22}$ | $X^{23}$ | $X^{24}$ | $X^{25}$ | $X^{26}$ | $X^{27}$ | $X^{28}$ | $X^{29}$ | $X^{10}$ | $X^{31}$ | $X^{32}$ | $X^{33}$ | $X^{34}$ | $X^{35}$ | $X^{36}$ | $X^{37}$ | $X^{38}$ | $X^{39}$ | $X^{40}$ | $X^{41}$ | hCRHR2 $pEC_{50}$ | hCRHR1 $pEC_{50}$ |
| 78 | I | V | L | S | L | D | Cle | P | I | G | L | L | Q | I | L | L | E | Q | A | R | A | R | Q | A | R | E | Q | A | K* | T | N | A | E | I | L | A | E | V | 9.4 | 8.1 |
| 79 | I | V | L | S | L | D | Cle | P | I | G | L | L | Q | I | L | L | E | Q | A | R | A | R | Q | A | R | E | Q | A | K* | T | N | A | E | I | Nle | A | E | V | 9.6 | 8.7 |
| 80 | I | V | L | S | L | D | V | c4FPro | I | G | L | L | Q | I | L | L | T | Q | A | R | A | R | A | A | R | E | Q | A | K* | T | N | A | E | I | L | A | E | V | 9.0 | 4.6 |
| 81 | I | V | L | S | L | D | V | c4FPro | I | G | L | L | Q | I | L | L | T | Q | A | R | A | R | A | A | R | E | Q | A | K* | T | N | A | E | I | Nle | A | E | V | 9.7 | 3.4 |
| 82 | I | V | L | S | L | D | V | P | I | G | L | L | Q | I | L | L | S | Q | A | R | A | R | A | A | R | E | Q | A | K* | T | N | A | E | I | L | A | E | V | 9.2 | 3.8 |
| 83 | I | V | L | S | L | D | V | P | I | G | L | L | Q | I | L | L | S | Q | A | R | A | R | A | A | R | E | Q | A | K* | T | N | A | E | I | Nle | A | E | V | 9.5 | 5.1 |
| 84 | I | V | L | S | L | D | V | Hyp | I | G | L | L | Q | I | L | L | E | Q | A | R | A | R | V | A | R | E | Q | A | K* | T | N | A | E | I | L | A | E | V | 9.0 | 7.6 |
| 85 | I | V | L | S | L | D | V | Hyp | I | G | L | L | Q | I | L | L | E | Q | A | R | A | R | V | A | R | E | Q | A | K* | T | N | A | E | I | Nle | A | E | V | 9.6 | 8.7 |
| 86 | I | V | L | S | L | D | Cle | c4FPro | I | G | L | L | Q | I | L | L | E | Q | A | R | A | R | A | A | R | E | Q | A | K* | T | N | A | E | I | L | A | E | V | 9.2 | 8.5 |
| 87 | I | V | L | S | L | D | Cle | c4FPro | I | G | L | L | Q | I | L | L | E | Q | A | R | A | R | A | A | R | E | Q | A | K* | T | N | A | E | I | Nle | A | E | V | 9.5 | 8.6 |
| 88 | I | V | L | S | L | D | V | P | I | G | L | L | Q | I | L | L | S | Q | A | R | A | R | A | A | R | E | Q | A | K* | T | N | A | E | I | L | A | E | I | 9.2 | 4.4 |
| 89 | I | V | L | S | L | D | V | P | I | G | L | L | Q | I | L | L | S | Q | A | R | A | R | A | A | R | E | Q | A | K* | T | N | A | E | I | Nle | A | E | I | 9.4 | 3.4 |
| 90 | I | V | L | S | L | D | V | P | I | G | L | L | Q | I | L | L | E | Q | A | R | A | R | V | A | R | E | Q | A | K* | T | N | A | E | I | L | A | E | V | 9.0 | 4.7 |
| 91 | I | V | L | S | L | D | V | P | I | G | L | L | Q | I | L | L | E | Q | A | R | A | R | V | A | R | E | Q | A | K* | T | N | A | E | I | Nle | A | E | V | 9.4 | 8.8 |
| 92 | I | V | L | S | L | D | V | P | I | G | L | L | Q | I | L | L | T | Q | A | R | A | R | Q | A | R | E | Q | A | K | T | N | A | E | I | L | A | E | V | 9.4 | 4.0 |
| 93 | I | V | L | S | L | D | V | P | I | G | L | L | Q | I | L | L | T | Q | A | R | A | R | Q | A | R | E | Q | A | K | T | N | A | E | I | Nle | A | E | V | 9.4 | 3.8 |
| 94 | I | V | L | S | L | D | V | P | I | G | L | L | Q | I | L | L | S | Q | A | R | A | R | I | A | R | E | Q | A | K | T | N | A | E | I | L | A | E | V | 9.0 | 3.4 |
| 95 | I | V | L | S | L | D | V | P | I | G | L | L | Q | I | L | L | S | Q | A | R | A | R | I | A | R | E | Q | A | K | T | N | A | E | I | Nle | A | E | V | 9.3 | 3.8 |
| 96 | I | V | L | S | L | D | V | C4FPro | I | G | L | L | Q | I | L | L | E | Q | A | R | A | R | N | A | R | E | Q | A | K | T | N | A | E | I | L | A | E | V | 8.7 | 3.7 |
| 97 | I | V | L | S | L | D | V | C4FPro | I | G | L | L | Q | I | L | L | E | Q | A | R | A | R | N | A | R | E | Q | A | K | T | N | A | E | I | Nle | A | E | V | 9.2 | 8.6 |
| 98 | I | V | L | S | L | D | V | P | L | G | L | L | Q | I | L | L | E | Q | A | R | A | R | A | A | R | E | Q | A | K | T | N | A | E | I | L | A | E | V | 9.4 | 8.0 |
| 99 | I | V | L | S | L | D | V | P | L | G | L | L | Q | I | L | L | E | Q | A | R | A | R | A | A | R | E | Q | A | K | T | N | A | E | I | Nle | A | E | V | 9.5 | 8.5 |
| 102 | I | V | L | S | L | D | V | C4FPro | I | G | L | L | Q | I | L | L | E | Q | A | R | A | R | A | A | R | E | Q | A | K | T | N | A | E | I | L | A | E | V | 9.1 | 3.6 |
| 103 | I | V | L | S | L | D | V | C4FPro | I | G | L | L | Q | I | L | L | E | Q | A | R | A | R | A | A | R | E | Q | A | K | T | N | A | E | I | Nle | A | E | V | 9.4 | 8.7 |
| 104 | I | V | L | S | L | D | V | C4NHPro | I | G | L | L | Q | I | L | L | E | Q | A | R | A | R | I | A | R | E | Q | A | K | T | N | A | E | I | L | A | E | V | 9.0 | 8.8 |
| 105 | I | V | L | S | L | D | V | C4NHPro | I | G | L | L | Q | I | L | L | E | Q | A | R | A | R | I | A | R | E | Q | A | K | T | N | A | E | I | Nle | A | E | V | 9.1 | 8.3 |
| 106 | I | V | L | S | L | D | V | C4FPro | I | G | L | L | Q | I | L | L | E | Q | A | R | A | R | V | A | R | E | Q | A | K | T | N | A | E | I | L | A | E | V | 9.0 | 3.8 |
| 107 | I | V | L | S | L | D | V | C4FPro | I | G | L | L | Q | I | L | L | E | Q | A | R | A | R | V | A | R | E | Q | A | K | T | N | A | E | I | Nle | A | E | V | 9.2 | 3.6 |
| 108 | I | V | L | S | L | D | Cle | P | I | G | L | L | Q | I | L | L | E | Q | A | R | A | R | V | A | R | E | Q | A | K | T | N | A | E | I | L | A | E | V | 9.3 | 8.5 |
| 109 | I | V | L | S | L | D | Cle | P | I | G | L | L | Q | I | L | L | E | Q | A | R | A | R | V | A | R | E | Q | A | K | T | N | A | E | I | Nle | A | E | V | 9.4 | 8.6 |

| Table 3 | | | | | | | | | | | | | | | | | | | | | | | | | | | | | | | | | | | | | | | | |
|---|---|---|---|---|---|---|---|---|---|---|---|---|---|---|---|---|---|---|---|---|---|---|---|---|---|---|---|---|---|---|---|---|---|---|---|---|---|---|---|---|
| | Amino acid positions | | | | | | | | | | | | | | | | | | | | | | | | | | | | | | | | | | | | | | Potency | |
| SEQ ID NO: | $X^{4}$ | $X^{5}$ | $X^{6}$ | $X^{7}$ | $X^{8}$ | $X^{9}$ | $X^{10}$ | $X^{11}$ | $X^{12}$ | $X^{13}$ | $X^{14}$ | $X^{15}$ | $X^{16}$ | $X^{17}$ | $X^{18}$ | $X^{19}$ | $X^{20}$ | $X^{21}$ | $X^{22}$ | $X^{23}$ | $X^{24}$ | $X^{25}$ | $X^{26}$ | $X^{27}$ | $X^{28}$ | $X^{29}$ | $X^{10}$ | $X^{31}$ | $X^{32}$ | $X^{33}$ | $X^{34}$ | $X^{35}$ | $X^{36}$ | $X^{37}$ | $X^{38}$ | $X^{39}$ | $X^{40}$ | $X^{41}$ | hCRHR2 $pEC_{50}$ | hCRHR1 $pEC_{50}$ |
| 110 | I | V | L | S | L | D | Cle | P | I | G | L | L | Q | I | L | L | E | Q | A | R | A | R | A | A | R | E | Q | A | K | T | N | A | E | I | L | A | E | V | 9.2 | 8.7 |
| 111 | I | V | L | S | L | D | Cle | P | I | G | L | L | Q | I | L | L | E | Q | A | R | A | R | A | A | R | E | Q | A | K | T | N | A | E | I | Nle | A | E | V | 9.5 | 8.8 |
| 112 | I | V | L | S | L | D | V | P | I | G | L | L | Q | I | L | L | T | Q | A | R | A | R | N | A | R | E | Q | A | K | T | N | A | E | I | L | A | E | V | 9.2 | 3.5 |
| 113 | I | V | L | S | L | D | V | P | I | G | L | L | Q | I | L | L | T | Q | A | R | A | R | N | A | R | E | Q | A | K | T | N | A | E | I | Nle | A | E | V | 9.7 | 3.4 |
| 114 | I | V | L | S | L | D | V | P | I | G | L | L | Q | I | L | L | E | Q | A | R | A | R | N | A | R | E | Q | A | K | T | N | A | E | I | L | A | E | V | 9.2 | 4.3 |
| 115 | I | V | L | S | L | D | V | P | I | G | L | L | Q | I | L | L | E | Q | A | R | A | R | N | A | R | E | Q | A | K | T | N | A | E | I | Nle | A | E | V | 9.7 | 8.7 |
| 116 | I | V | L | S | L | D | V | C4NHPro | I | G | L | L | Q | I | L | L | E | Q | A | R | A | R | Q | A | R | E | Q | A | K | T | N | A | E | I | L | A | E | V | 9.1 | 9.1 |
| 117 | I | V | L | S | L | D | V | C4NHPro | I | G | L | L | Q | I | L | L | E | Q | A | R | A | R | Q | A | R | E | Q | A | K | T | N | A | E | I | Nle | A | E | V | 9.3 | 8.9 |
| 118 | MeI | V | L | S | L | D | V | P | I | G | L | L | Q | I | L | L | A | Q | A | R | A | R | A | A | R | E | Q | A | K* | Aib | N | A | E | I | L | A | E | V | 10.7 | 4.0 |
| 119 | MeI | V | L | S | L | D | V | P | I | G | L | L | Q | I | L | L | A | Q | A | R | A | R | A | A | R | E | Q | A | K* | Aib | N | A | E | I | Nle | A | E | V | 10.7 | 6.0 |
| 120 | MeI | V | L | S | L | D | V | P | I | G | L | L | Q | I | L | L | D | Q | A | R | A | R | A | A | R | E | Q | A | K* | Aib | N | A | E | I | L | A | E | V | 10.1 | 5.2 |
| 121 | MeI | V | L | S | L | D | V | P | I | G | L | L | Q | I | L | L | D | Q | A | R | A | R | A | A | R | E | Q | A | K* | Aib | N | A | E | I | Nle | A | E | V | 10.2 | 4.7 |
| 122 | MeI | V | L | S | L | D | V | P | I | G | L | L | Q | I | L | L | G | Q | A | R | A | R | A | A | R | E | Q | A | K* | Aib | N | A | E | I | L | A | E | V | 11.0 | 4.0 |
| 123 | MeI | V | L | S | L | D | V | P | I | G | L | L | Q | I | L | L | G | Q | A | R | A | R | A | A | R | E | Q | A | K* | Aib | N | A | E | I | Nle | A | E | V | 11.3 | 6.1 |
| 20 | MeI | V | L | S | L | D | V | P | I | G | L | L | Q | I | L | L | E | Q | A | R | A | R | A | A | R | E | Q | A | K* | Aib | N | A | E | I | L | A | E | V | 10.0 | 5.9 |
| 124 | MeI | V | L | S | L | D | V | P | I | G | L | L | Q | I | L | L | E | Q | A | R | A | R | A | A | R | E | Q | A | K* | Aib | N | A | E | I | Nle | A | E | V | 11.2 | 6.6 |
| 125 | MeI | V | L | S | L | D | V | P | I | G | L | L | Q | I | L | L | H | Q | A | R | A | R | A | A | R | E | Q | A | K* | Aib | N | A | E | I | L | A | E | V | 9.6 | 2.6 |
| 126 | MeI | V | L | S | L | D | V | P | I | G | L | L | Q | I | L | L | H | Q | A | R | A | R | A | A | R | E | Q | A | K* | Aib | N | A | E | I | Nle | A | E | V | 11.0 | 6.1 |
| 127 | MeI | V | L | S | L | D | V | P | I | G | L | L | Q | I | L | L | F | Q | A | R | A | R | A | A | R | E | Q | A | K* | Aib | N | A | E | I | L | A | E | V | 9.7 | 2.1 |
| 128 | MeI | V | L | S | L | D | V | P | I | G | L | L | Q | I | L | L | F | Q | A | R | A | R | A | A | R | E | Q | A | K* | Aib | N | A | E | I | Nle | A | E | V | 11.3 | 4.0 |
| 129 | MeI | V | L | S | L | D | V | P | I | G | L | L | Q | I | L | L | P | Q | A | R | A | R | A | A | R | E | Q | A | K* | Aib | N | A | E | I | L | A | E | V | 9.8 | 4.1 |
| 130 | MeI | V | L | S | L | D | V | P | I | G | L | L | Q | I | L | L | P | Q | A | R | A | R | A | A | R | E | Q | A | K* | Aib | N | A | E | I | Nle | A | E | V | 10.2 | 4.7 |
| 131 | MeI | V | L | S | L | D | V | P | I | G | L | L | Q | I | L | L | S | Q | A | R | A | R | A | A | R | E | Q | A | K* | Aib | N | A | E | I | L | A | E | V | 10.6 | 3.1 |
| 132 | MeI | V | L | S | L | D | V | P | I | G | L | L | Q | I | L | L | S | Q | A | R | A | R | A | A | R | E | Q | A | K* | Aib | N | A | E | I | Nle | A | E | V | 10.7 | 6.1 |
| 133 | MeI | V | L | S | L | D | V | P | I | G | L | L | Q | I | L | L | T | Q | A | R | A | R | A | A | R | E | Q | A | K* | Aib | N | A | E | I | L | A | E | V | 10.8 | 2.6 |
| 134 | MeI | V | L | S | L | D | V | P | I | G | L | L | Q | I | L | L | T | Q | A | R | A | R | A | A | R | E | Q | A | K* | Aib | N | A | E | I | Nle | A | E | V | 11.1 | 6.1 |
| 135 | MeI | V | L | S | L | D | V | P | I | G | L | L | Q | I | L | L | W | Q | A | R | A | R | A | A | R | E | Q | A | K* | Aib | N | A | E | I | L | A | E | V | 10.3 | 2.2 |
| 136 | MeI | V | L | S | L | D | V | P | I | G | L | L | Q | I | L | L | W | Q | A | R | A | R | A | A | R | E | Q | A | K* | Aib | N | A | E | I | Nle | A | E | V | 10.4 | 3.2 |
| 137 | MeI | V | L | S | L | D | V | P | I | G | L | L | Q | I | L | L | V | Q | A | R | A | R | A | A | R | E | Q | A | K* | Aib | N | A | E | I | L | A | E | V | 10.5 | 2.9 |
| 138 | MeI | V | L | S | L | D | V | P | I | G | L | L | Q | I | L | L | V | Q | A | R | A | R | A | A | R | E | Q | A | K* | Aib | N | A | E | I | Nle | A | E | V | 10.8 | 6.0 |
| 139 | MeI | V | L | S | L | D | V | P | I | G | L | L | Q | I | L | L | E | Q | A | R | A | R | A | A | R | E | Q | A | K* | Aib | N | A | E | I | L | A | E | I | 10.6 | 5.7 |
| 140 | MeI | V | L | S | L | D | V | P | I | G | L | L | Q | I | L | L | E | Q | A | R | A | R | A | A | R | E | Q | A | K* | Aib | N | A | E | I | Nle | A | E | I | 11.9 | 6.4 |
| 141 | MeI | V | L | S | L | D | V | P | I | G | L | L | Q | I | L | L | E | Q | A | R | A | R | A | A | R | E | Q | A | K* | Aib | N | A | E | I | L | A | E | L | 9.9 | 2.7 |
| 142 | MeI | V | L | S | L | D | V | P | I | G | L | L | Q | I | L | L | E | Q | A | R | A | R | A | A | R | E | Q | A | K* | Aib | N | A | E | I | Nle | A | E | L | 10.4 | 5.6 |
| 143 | MeI | V | L | S | L | D | V | P | I | G | L | L | Q | I | L | L | E | Q | A | R | A | R | A | A | R | E | Q | A | K* | Aib | N | A | E | I | L | A | E | Aib | 9.7 | 2.2 |
| 144 | MeI | V | L | S | L | D | V | P | I | G | L | L | Q | I | L | L | E | Q | A | R | A | R | A | A | R | E | Q | A | K* | Aib | N | A | E | I | Nle | A | E | Aib | 10.4 | 5.6 |
| 145 | MeI | V | L | S | L | D | V | P | I | G | L | L | Q | I | L | L | S | Q | A | R | A | R | A | A | R | E | Q | A | K* | A | N | A | E | I | L | A | E | V | 10.6 | 2.9 |
| 146 | MeI | V | L | S | L | D | V | P | I | G | L | L | Q | I | L | L | S | Q | A | R | A | R | A | A | R | E | Q | A | K* | A | N | A | E | I | Nle | A | E | V | 10.9 | 6.0 |
| 147 | MeI | V | L | S | L | D | V | P | I | G | L | L | Q | I | L | L | S | Q | A | R | A | R | A | A | R | E | Q | A | K* | Q | N | A | E | I | L | A | E | V | 10.7 | 3.2 |
| 148 | MeI | V | L | S | L | D | V | P | I | G | L | L | Q | I | L | L | S | Q | A | R | A | R | A | A | R | E | Q | A | K* | Q | N | A | E | I | Nle | A | E | V | 11.0 | 5.9 |
| 149 | MeI | V | L | S | L | D | V | P | I | G | L | L | Q | I | L | L | S | Q | A | R | A | R | A | A | R | E | Q | A | K* | G | N | A | E | I | L | A | E | V | 10.0 | 2.2 |
| 150 | MeI | V | L | S | L | D | V | P | I | G | L | L | Q | I | L | L | S | Q | A | R | A | R | A | A | R | E | Q | A | K* | G | N | A | E | I | Nle | A | E | V | 10.1 | 2.0 |
| 151 | MeI | V | L | S | L | D | V | P | I | G | L | L | Q | I | L | L | S | Q | A | R | A | R | A | A | R | E | Q | A | K* | H | N | A | E | I | L | A | E | V | 10.7 | 2.8 |
| 152 | MeI | V | L | S | L | D | V | P | I | G | L | L | Q | I | L | L | S | Q | A | R | A | R | A | A | R | E | Q | A | K* | H | N | A | E | I | Nle | A | E | V | 10.9 | 6.0 |
| 153 | MeI | V | L | S | L | D | V | P | I | G | L | L | Q | I | L | L | S | Q | A | R | A | R | A | A | R | E | Q | A | K* | I | N | A | E | I | L | A | E | V | 10.6 | 5.8 |
| 154 | MeI | V | L | S | L | D | V | P | I | G | L | L | Q | I | L | L | S | Q | A | R | A | R | A | A | R | E | Q | A | K* | I | N | A | E | I | Nle | A | E | V | 10.9 | 6.1 |
| 155 | MeI | V | L | S | L | D | V | P | I | G | L | L | Q | I | L | L | S | Q | A | R | A | R | A | A | R | E | Q | A | K* | L | N | A | E | I | L | A | E | V | 10.3 | 3.3 |
| 156 | MeI | V | L | S | L | D | V | P | I | G | L | L | Q | I | L | L | S | Q | A | R | A | R | A | A | R | E | Q | A | K* | L | N | A | E | I | Nle | A | E | V | 10.6 | 6.2 |
| 157 | MeI | V | L | S | L | D | V | P | I | G | L | L | Q | I | L | L | S | Q | A | R | A | R | A | A | R | E | Q | A | K* | F | N | A | E | I | L | A | E | V | 9.8 | 2.7 |
| 158 | MeI | V | L | S | L | D | V | P | I | G | L | L | Q | I | L | L | S | Q | A | R | A | R | A | A | R | E | Q | A | K* | F | N | A | E | I | Nle | A | E | V | 10.7 | 5.3 |
| 159 | MeI | V | L | S | L | D | V | P | I | G | L | L | Q | I | L | L | S | Q | A | R | A | R | A | A | R | E | Q | A | K* | P | N | A | E | I | L | A | E | V | 8.2 | 2.0 |
| 160 | MeI | V | L | S | L | D | V | P | I | G | L | L | Q | I | L | L | S | Q | A | R | A | R | A | A | R | E | Q | A | K* | P | N | A | E | I | Nle | A | E | V | 9.1 | 2.6 |
| 161 | MeI | V | L | S | L | D | V | P | I | G | L | L | Q | I | L | L | S | Q | A | R | A | R | A | A | R | E | Q | A | K* | S | N | A | E | I | L | A | E | V | 10.4 | 2.3 |
| 162 | MeI | V | L | S | L | D | V | P | I | G | L | L | Q | I | L | L | S | Q | A | R | A | R | A | A | R | E | Q | A | K* | S | N | A | E | I | Nle | A | E | V | 11.7 | 4.6 |
| 163 | MeI | V | L | S | L | D | V | P | I | G | L | L | Q | I | L | L | S | Q | A | R | A | R | A | A | R | E | Q | A | K* | T | N | A | E | I | L | A | E | V | 10.5 | 3.8 |
| 164 | MeI | V | L | S | L | D | V | P | I | G | L | L | Q | I | L | L | S | Q | A | R | A | R | A | A | R | E | Q | A | K* | T | N | A | E | I | Nle | A | E | V | 10.9 | 6.0 |
| 165 | MeI | V | L | S | L | D | V | P | I | G | L | L | Q | I | L | L | S | Q | A | R | A | R | A | A | R | E | Q | A | K* | W | N | A | E | I | L | A | E | V | 10.0 | 2.4 |
| 166 | MeI | V | L | S | L | D | V | P | I | G | L | L | Q | I | L | L | S | Q | A | R | A | R | A | A | R | E | Q | A | K* | W | N | A | E | I | Nle | A | E | V | 10.9 | 5.9 |
| 167 | MeI | V | L | S | L | D | V | P | I | G | L | L | Q | I | L | L | S | Q | A | R | A | R | A | A | R | E | Q | A | K* | Y | N | A | E | I | L | A | E | V | 10.5 | 2.5 |
| 168 | MeI | V | L | S | L | D | V | P | I | G | L | L | Q | I | L | L | S | Q | A | R | A | R | A | A | R | E | Q | A | K* | Y | N | A | E | I | Nle | A | E | V | 11.2 | 5.4 |

**Table 3**

| | Amino acid positions | | | | | | | | | | | | | | | | | | | | | | | | | | | | | | | | | | | | | | Potency | |
|---|---|---|---|---|---|---|---|---|---|---|---|---|---|---|---|---|---|---|---|---|---|---|---|---|---|---|---|---|---|---|---|---|---|---|---|---|---|---|---|---|
| SEQ ID NO: | $X^4$ | $X^5$ | $X^6$ | $X^7$ | $X^8$ | $X^9$ | $X^{10}$ | $X^{11}$ | $X^{12}$ | $X^{13}$ | $X^{14}$ | $X^{15}$ | $X^{16}$ | $X^{17}$ | $X^{18}$ | $X^{19}$ | $X^{20}$ | $X^{21}$ | $X^{22}$ | $X^{23}$ | $X^{24}$ | $X^{25}$ | $X^{26}$ | $X^{27}$ | $X^{28}$ | $X^{29}$ | $X^{10}$ | $X^{31}$ | $X^{32}$ | $X^{33}$ | $X^{34}$ | $X^{35}$ | $X^{36}$ | $X^{37}$ | $X^{38}$ | $X^{39}$ | $X^{40}$ | $X^{41}$ | hCRHR2 $pEC_{50}$ | hCRHR1 $pEC_{50}$ |
| 169 | MeI | V | L | S | L | D | V | P | I | G | L | L | Q | I | L | L | S | Q | A | R | A | R | A | A | R | E | Q | A | K* | V | N | A | E | I | L | A | E | V | 10.6 | 3.7 |
| 170 | MeI | V | L | S | L | D | V | P | I | G | L | L | Q | I | L | L | S | Q | A | R | A | R | A | A | R | E | Q | A | K* | V | N | A | E | I | Nle | A | E | V | 11.3 | 6.3 |
| 171 | MeI | V | L | S | L | D | V | P | I | G | L | L | Q | I | L | L | S | Q | A | R | A | R | A | A | R | E | Q | A | K* | T | N | R | E | I | L | A | E | V | 10.1 | 6.1 |
| 172 | MeI | V | L | S | L | D | V | P | I | G | L | L | Q | I | L | L | S | Q | A | R | A | R | A | A | R | E | Q | A | K* | T | N | R | E | I | Nle | A | E | V | 10.7 | 6.3 |
| 173 | MeI | V | L | S | L | D | V | P | I | G | L | L | Q | I | L | L | S | Q | A | R | A | R | A | A | R | E | Q | A | K* | T | N | N | E | I | L | A | E | V | 10.5 | 1.9 |
| 174 | MeI | V | L | S | L | D | V | P | I | G | L | L | Q | I | L | L | S | Q | A | R | A | R | A | A | R | E | Q | A | K* | T | N | N | E | I | Nle | A | E | V | 10.6 | 3.1 |
| 175 | MeI | V | L | S | L | D | V | P | I | G | L | L | Q | I | L | L | S | Q | A | R | A | R | A | A | R | E | Q | A | K* | T | N | D | E | I | L | A | E | V | 9.1 | 2.1 |
| 176 | MeI | V | L | S | L | D | V | P | I | G | L | L | Q | I | L | L | S | Q | A | R | A | R | A | A | R | E | Q | A | K* | T | N | D | E | I | Nle | A | E | V | 10.4 | 2.2 |
| 177 | MeI | V | L | S | L | D | V | P | I | G | L | L | Q | I | L | L | S | Q | A | R | A | R | A | A | R | E | Q | A | K* | T | N | Q | E | I | L | A | E | V | 10.4 | 2.5 |
| 178 | MeI | V | L | S | L | D | V | P | I | G | L | L | Q | I | L | L | S | Q | A | R | A | R | A | A | R | E | Q | A | K* | T | N | Q | E | I | Nle | A | E | V | 11.0 | 5.9 |
| 179 | MeI | V | L | S | L | D | V | P | I | G | L | L | Q | I | L | L | S | Q | A | R | A | R | A | A | R | E | Q | A | K* | T | N | G | E | I | L | A | E | V | 10.2 | 2.0 |
| 180 | MeI | V | L | S | L | D | V | P | I | G | L | L | Q | I | L | L | S | Q | A | R | A | R | A | A | R | E | Q | A | K* | T | N | G | E | I | Nle | A | E | V | 10.6 | 2.5 |
| 181 | MeI | V | L | S | L | D | V | P | I | G | L | L | Q | I | L | L | S | Q | A | R | A | R | A | A | R | E | Q | A | K* | T | N | H | E | I | L | A | E | V | 10.4 | 2.3 |
| 182 | MeI | V | L | S | L | D | V | P | I | G | L | L | Q | I | L | L | S | Q | A | R | A | R | A | A | R | E | Q | A | K* | T | N | H | E | I | Nle | A | E | V | 10.6 | 4.2 |
| 183 | MeI | V | L | S | L | D | V | P | I | G | L | L | Q | I | L | L | S | Q | A | R | A | R | A | A | R | E | Q | A | K* | T | N | L | E | I | L | A | E | V | 9.2 | 2.1 |
| 184 | MeI | V | L | S | L | D | V | P | I | G | L | L | Q | I | L | L | S | Q | A | R | A | R | A | A | R | E | Q | A | K* | T | N | L | E | I | Nle | A | E | V | 10.3 | 3.0 |
| 185 | MeI | V | L | S | L | D | V | P | I | G | L | L | Q | I | L | L | S | Q | A | R | A | R | A | A | R | E | Q | A | K* | T | N | K | E | I | L | A | E | V | 10.2 | 5.2 |
| 186 | MeI | V | L | S | L | D | V | P | I | G | L | L | Q | I | L | L | S | Q | A | R | A | R | A | A | R | E | Q | A | K* | T | N | K | E | I | Nle | A | E | V | 10.9 | 6.2 |
| 187 | MeI | V | L | S | L | D | V | P | I | G | L | L | Q | I | L | L | S | Q | A | R | A | R | A | A | R | E | Q | A | K* | T | N | F | E | I | L | A | E | V | 9.2 | 1.9 |
| 188 | MeI | V | L | S | L | D | V | P | I | G | L | L | Q | I | L | L | S | Q | A | R | A | R | A | A | R | E | Q | A | K* | T | N | F | E | I | Nle | A | E | V | 10.6 | 4.4 |
| 189 | MeI | V | L | S | L | D | V | P | I | G | L | L | Q | I | L | L | S | Q | A | R | A | R | A | A | R | E | Q | A | K* | T | N | S | E | I | L | A | E | V | 10.5 | 1.6 |
| 190 | MeI | V | L | S | L | D | V | P | I | G | L | L | Q | I | L | L | S | Q | A | R | A | R | A | A | R | E | Q | A | K* | T | N | S | E | I | Nle | A | E | V | 11.1 | 4.5 |
| 191 | MeI | V | L | S | L | D | V | P | I | G | L | L | Q | I | L | L | S | Q | A | R | A | R | A | A | R | E | Q | A | K* | T | N | T | E | I | L | A | E | V | 10.5 | 2.2 |
| 192 | MeI | V | L | S | L | D | V | P | I | G | L | L | Q | I | L | L | S | Q | A | R | A | R | A | A | R | E | Q | A | K* | T | N | T | E | I | Nle | A | E | V | 12.1 | 2.6 |
| 193 | MeI | V | L | S | L | D | V | P | I | G | L | L | Q | I | L | L | S | Q | A | R | A | R | A | A | R | E | Q | A | K* | T | N | W | E | I | L | A | E | V | 10.1 | 2.4 |
| 194 | MeI | V | L | S | L | D | V | P | I | G | L | L | Q | I | L | L | S | Q | A | R | A | R | A | A | R | E | Q | A | K* | T | N | W | E | I | Nle | A | E | V | 10.5 | 3.3 |
| 195 | MeI | V | L | S | L | D | V | P | I | G | L | L | Q | I | L | L | A | Q | A | R | A | R | A | A | R | E | Q | A | K* | T | N | Aib | E | I | L | A | E | V | 10.0 | 2.4 |
| 196 | MeI | V | L | S | L | D | V | P | I | G | L | L | Q | I | L | L | A | Q | A | R | A | R | A | A | R | E | Q | A | K* | T | N | Aib | E | I | Nle | A | E | V | 10.5 | 6.0 |
| 197 | MeI | V | L | S | L | D | V | P | I | G | L | L | Q | I | L | L | R | Q | A | R | A | R | A | A | R | E | Q | A | K* | T | N | Aib | E | I | L | A | E | V | 9.9 | 2.3 |
| 198 | MeI | V | L | S | L | D | V | P | I | G | L | L | Q | I | L | L | R | Q | A | R | A | R | A | A | R | E | Q | A | K* | T | N | Aib | E | I | Nle | A | E | V | 10.5 | 4.2 |

**Table 3**

| | Amino acid positions | | | | | | | | | | | | | | | | | | | | | | | | | | | | | | | | | | | | | | Potency | |
|---|---|---|---|---|---|---|---|---|---|---|---|---|---|---|---|---|---|---|---|---|---|---|---|---|---|---|---|---|---|---|---|---|---|---|---|---|---|---|---|---|
| SEQ ID NO: | $X^4$ | $X^5$ | $X^6$ | $X^7$ | $X^8$ | $X^9$ | $X^{10}$ | $X^{11}$ | $X^{12}$ | $X^{13}$ | $X^{14}$ | $X^{15}$ | $X^{16}$ | $X^{17}$ | $X^{18}$ | $X^{19}$ | $X^{20}$ | $X^{21}$ | $X^{22}$ | $X^{23}$ | $X^{24}$ | $X^{25}$ | $X^{26}$ | $X^{27}$ | $X^{28}$ | $X^{29}$ | $X^{10}$ | $X^{31}$ | $X^{32}$ | $X^{33}$ | $X^{34}$ | $X^{35}$ | $X^{36}$ | $X^{37}$ | $X^{38}$ | $X^{39}$ | $X^{40}$ | $X^{41}$ | hCRHR2 $pEC_{50}$ | hCRHR1 $pEC_{50}$ |
| 199 | MeI | V | L | S | L | D | V | P | I | G | L | L | Q | I | L | L | D | Q | A | R | A | R | A | A | R | E | Q | A | K* | T | N | Aib | E | I | L | A | E | V | 9.7 | 2.6 |
| 200 | MeI | V | L | S | L | D | V | P | I | G | L | L | Q | I | L | L | D | Q | A | R | A | R | A | A | R | E | Q | A | K* | T | N | Aib | E | I | Nle | A | E | V | 10.0 | 5.9 |
| 201 | MeI | V | L | S | L | D | V | P | I | G | L | L | Q | I | L | L | Q | Q | A | R | A | R | A | A | R | E | Q | A | K* | T | N | Aib | E | I | L | A | E | V | 10.2 | 2.7 |
| 202 | MeI | V | L | S | L | D | V | P | I | G | L | L | Q | I | L | L | Q | Q | A | R | A | R | A | A | R | E | Q | A | K* | T | N | Aib | E | I | Nle | A | E | V | 10.6 | 6.1 |
| 203 | MeI | V | L | S | L | D | V | P | I | G | L | L | Q | I | L | L | G | Q | A | R | A | R | A | A | R | E | Q | A | K* | T | N | Aib | E | I | L | A | E | V | 9.5 | 2.5 |
| 204 | MeI | V | L | S | L | D | V | P | I | G | L | L | Q | I | L | L | G | Q | A | R | A | R | A | A | R | E | Q | A | K* | T | N | Aib | E | I | Nle | A | E | V | 10.6 | 6.0 |
| 16 | MeI | V | L | S | L | D | V | P | I | G | L | L | Q | I | L | L | E | Q | A | R | A | R | A | A | R | E | Q | A | K* | T | N | Aib | E | I | L | A | E | V | 10.2 | 5.9 |
| 205 | MeI | V | L | S | L | D | V | P | I | G | L | L | Q | I | L | L | E | Q | A | R | A | R | A | A | R | E | Q | A | K* | T | N | Aib | E | I | Nle | A | E | V | 10.2 | 5.9 |
| 206 | MeI | V | L | S | L | D | V | P | I | G | L | L | Q | I | L | L | H | Q | A | R | A | R | A | A | R | E | Q | A | K* | T | N | Aib | E | I | L | A | E | V | 10.3 | 4.0 |
| 207 | MeI | V | L | S | L | D | V | P | I | G | L | L | Q | I | L | L | H | Q | A | R | A | R | A | A | R | E | Q | A | K* | T | N | Aib | E | I | Nle | A | E | V | 10.4 | 6.0 |
| 208 | MeI | V | L | S | L | D | V | P | I | G | L | L | Q | I | L | L | I | Q | A | R | A | R | A | A | R | E | Q | A | K* | T | N | Aib | E | I | L | A | E | V | 9.7 | 2.3 |
| 209 | MeI | V | L | S | L | D | V | P | I | G | L | L | Q | I | L | L | I | Q | A | R | A | R | A | A | R | E | Q | A | K* | T | N | Aib | E | I | Nle | A | E | V | 10.7 | 5.5 |
| 210 | MeI | V | L | S | L | D | V | P | I | G | L | L | Q | I | L | L | F | Q | A | R | A | R | A | A | R | E | Q | A | K* | T | N | Aib | E | I | L | A | E | V | 9.7 | 2.0 |
| 211 | MeI | V | L | S | L | D | V | P | I | G | L | L | Q | I | L | L | F | Q | A | R | A | R | A | A | R | E | Q | A | K* | T | N | Aib | E | I | Nle | A | E | V | 10.6 | 4.1 |
| 212 | MeI | V | L | S | L | D | V | P | I | G | L | L | Q | I | L | L | P | Q | A | R | A | R | A | A | R | E | Q | A | K* | T | N | Aib | E | I | L | A | E | V | 9.3 | 2.2 |
| 213 | MeI | V | L | S | L | D | V | P | I | G | L | L | Q | I | L | L | P | Q | A | R | A | R | A | A | R | E | Q | A | K* | T | N | Aib | E | I | Nle | A | E | V | 9.7 | 4.3 |
| 214 | MeI | V | L | S | L | D | V | P | I | G | L | L | Q | I | L | L | S | Q | A | R | A | R | A | A | R | E | Q | A | K* | T | N | Aib | E | I | L | A | E | V | 10.1 | 2.7 |
| 215 | MeI | V | L | S | L | D | V | P | I | G | L | L | Q | I | L | L | S | Q | A | R | A | R | A | A | R | E | Q | A | K* | T | N | Aib | E | I | Nle | A | E | V | 10.6 | 5.5 |
| 216 | MeI | V | L | S | L | D | V | P | I | G | L | L | Q | I | L | L | T | Q | A | R | A | R | A | A | R | E | Q | A | K* | T | N | Aib | E | I | L | A | E | V | 9.8 | 3.3 |
| 217 | MeI | V | L | S | L | D | V | P | I | G | L | L | Q | I | L | L | T | Q | A | R | A | R | A | A | R | E | Q | A | K* | T | N | Aib | E | I | Nle | A | E | V | 10.4 | 6.0 |
| 218 | MeI | V | L | S | L | D | V | P | I | G | L | L | Q | I | L | L | W | Q | A | R | A | R | A | A | R | E | Q | A | K* | T | N | Aib | E | I | L | A | E | V | 10.0 | 2.3 |
| 219 | MeI | V | L | S | L | D | V | P | I | G | L | L | Q | I | L | L | W | Q | A | R | A | R | A | A | R | E | Q | A | K* | T | N | Aib | E | I | Nle | A | E | V | 10.1 | 2.6 |
| 220 | MeI | V | L | S | L | D | V | P | I | G | L | L | Q | I | L | L | Y | Q | A | R | A | R | A | A | R | E | Q | A | K* | T | N | Aib | E | I | L | A | E | V | 9.6 | 2.6 |
| 221 | MeI | V | L | S | L | D | V | P | I | G | L | L | Q | I | L | L | Y | Q | A | R | A | R | A | A | R | E | Q | A | K* | T | N | Aib | E | I | Nle | A | E | V | 10.6 | 3.6 |
| 222 | MeI | V | L | S | L | D | V | P | I | G | L | L | Q | I | L | L | E | Q | A | R | A | R | A | A | R | E | Q | A | K* | T | N | Aib | E | I | L | A | E | I | 10.2 | 3.8 |
| 223 | MeI | V | L | S | L | D | V | P | I | G | L | L | Q | I | L | L | E | Q | A | R | A | R | A | A | R | E | Q | A | K* | T | N | Aib | E | I | Nle | A | E | I | 10.3 | 6.3 |
| 224 | MeI | V | L | S | L | D | V | P | I | G | L | L | Q | I | L | L | E | Q | A | R | A | R | A | A | R | E | Q | A | K* | T | N | Aib | E | I | L | A | E | L | 9.1 | 2.0 |
| 225 | MeI | V | L | S | L | D | V | P | I | G | L | L | Q | I | L | L | E | Q | A | R | A | R | A | A | R | E | Q | A | K* | T | N | Aib | E | I | Nle | A | E | L | 10.0 | 4.6 |
| 226 | MeI | V | L | S | L | D | V | P | I | G | L | L | Q | I | L | L | E | Q | A | R | A | R | A | A | R | E | Q | A | K* | T | N | Aib | E | I | L | A | E | Aib | 9.4 | 2.2 |
| 227 | MeI | V | L | S | L | D | V | P | I | G | L | L | Q | I | L | L | E | Q | A | R | A | R | A | A | R | E | Q | A | K* | T | N | Aib | E | I | Nle | A | E | Aib | 9.7 | 5.5 |

Table 3

| | Amino acid positions | | | | | | | | | | | | | | | | | | |
|---|---|---|---|---|---|---|---|---|---|---|---|---|---|---|---|---|---|---|---|
| SEQ ID NO: | $X^4$ | $X^5$ | $X^6$ | $X^7$ | $X^8$ | $X^9$ | $X^{10}$ | $X^{11}$ | $X^{12}$ | $X^{13}$ | $X^{14}$ | $X^{15}$ | $X^{16}$ | $X^{17}$ | $X^{18}$ | $X^{19}$ | $X^{20}$ | $X^{21}$ | $X^{22}$ |
| Exemplified amino acids in table 3 | I, MeI | V | L | S | L | D | V, Cle | P, Hyp, c4NHPro, c4FPro | I | G | L | L | Q | I | L | L | E, Y, W, T, S, P, F, I, H, E, G, Q, D, R, A, S, V | Q | A |

| | Amino acid positions | | | | | | | | | | | | | | | | | | | Potency | |
|---|---|---|---|---|---|---|---|---|---|---|---|---|---|---|---|---|---|---|---|---|---|
| SEQ ID NO: | $X^{23}$ | $X^{24}$ | $X^{25}$ | $X^{26}$ | $X^{27}$ | $X^{28}$ | $X^{29}$ | $X^{10}$ | $X^{31}$ | $X^{32}$ | $X^{33}$ | $X^{34}$ | $X^{35}$ | $X^{36}$ | $X^{37}$ | $X^{38}$ | $X^{39}$ | $X^{40}$ | $X^{41}$ | hCRHR2 $pEC_{50}$ | hCRHR1 $pEC_{50}$ |
| Exemplified amino acids in table 3 | R | A | R | A, I, N, V, Q, S | A, Q, L | R | E, Q | Q, E, R | A | K* | T, V, Y, W, S, P, F, L, I, H, G, Q, A, Aib | N | A, Aib, W, T, S, F, K, L, H, G, Q, D, N, R | E | I | Nle, L | A | E | I, V, L, Aib | - | - |

**Example 5: Improvement of hCRHR2 potency while retaining high selectivity, chemical stability and solubility**

In order to identify additional substitutions in position $X^{20}$ capable of restoring the high selectivity for hCRHR2, a library of 74 peptides were synthesised. 18 amino acid substitutions were introduced in position $X^{20}$ in the chemically stable UCN2 analogues being lipidated in position $X^{32}$, substituted with a glutamic acid (E) in position $X^{36}$ and $X^{40}$, and substituted with Aib in $X^{33}$ or $X^{35}$ (i.e. reference 1 or 2 in Table 4).

The $EC_{50}$ values on hCRHR2 and hCRHR1 were determined for each library and SHAP values calculated from a random forest model, where $pEC_{50}$ values were fitted to the peptide amino acid sequences. Delta mean SHAP values were used to determine the level of contribution of each amino acid substitution relative to the corresponding native UCN2 residue on hCRHR2 and hCRHR1 potency (Breiman, L. (2001), Random Forests, Machine Learning 45(1), 5-32.; Lundberg, S. M., & Lee, S. I. (2017). A unified approach to interpreting model predictions. Advances in neural information processing systems, 30.). Substitutions with positive delta mean SHAP values increased the end-point relative to the native UCN2 residue, while negative delta mean SHAP values decreased the end-point relative to the native UCN2 residue. The contribution of each amino acid is summarized in Fig. 4.

The results in Fig. 4 demonstrates the potency improving effects of $X^{38}$ = Nle and the selectivity improving effect of substituting position $X^{20}$ when $X^{38}$ = Nle. In $X^{20}$, the following amino acids had negative effect on hCRHR1 while not significantly negatively affecting hCRHR2 $pEC_{50}$: A, F, G, H, I, K, L, N, Q, R, S, T, or V. Thus, when $X^{38}$ = Nle, the selectivity ratio for hCRHR2 over hCRHR1 (i.e. hCRHR1 $EC_{50}$/hCRHR2 $EC_{50}$), can be improved by substituting the glutamate (E) present in $X^{20}$ with A, F, G, H, I, K, L, N, Q, R, S, T, or V. In a preferred embodiment, when $X^{38}$ = Nle, to improve potency, $X^{20}$ is selected as A, F, G, H, I, K, L, N, Q, R, S, T, or V to restore a high selectivity for hCRHR2. In a more preferred embodiment, when $X^{38}$ = Nle, to improve potency, $X^{20}$ is selected as N, F, G, K, Q, S, or T to restore a high selectivity for hCRHR2. In the most preferred embodiment, when $X^{38}$ = Nle, to improve potency, $X^{20}$ is selected as S.

**Table 4. Improvement of selectivity in peptides having $X^{38}$ = Nle**

| | Amino acid positions | | | | | | | | | | | | | | | | | | | | | | | | | | | | | | | | | | | | | | Potency | | Selectivity |
|---|---|---|---|---|---|---|---|---|---|---|---|---|---|---|---|---|---|---|---|---|---|---|---|---|---|---|---|---|---|---|---|---|---|---|---|---|---|---|---|---|---|
| SEQ ID NO: | $X^{4}$ | $X^{5}$ | $X^{6}$ | $X^{7}$ | $X^{8}$ | $X^{9}$ | $X^{10}$ | $X^{11}$ | $X^{12}$ | $X^{13}$ | $X^{14}$ | $X^{15}$ | $X^{16}$ | $X^{17}$ | $X^{18}$ | $X^{19}$ | $X^{20}$ | $X^{21}$ | $X^{22}$ | $X^{23}$ | $X^{24}$ | $X^{25}$ | $X^{26}$ | $X^{27}$ | $X^{28}$ | $X^{29}$ | $X^{30}$ | $X^{31}$ | $X^{32}$ | $X^{33}$ | $X^{34}$ | $X^{35}$ | $X^{36}$ | $X^{37}$ | $X^{38}$ | $X^{39}$ | $X^{40}$ | $X^{41}$ | hCRHR2 $pEC_{50}$ | hCRHR1 $pEC_{50}$ | hCRHR1 EC50/hCRHR2 EC50 |
| 205 (Ref. 1) | MeI | V | L | S | L | D | V | P | I | G | L | L | Q | I | L | L | E | Q | A | R | A | R | A | A | R | E | Q | A | K* | T | N | Aib | E | I | Nle | A | E | V | 10.2 | 5.9 | 20689 |
| 196 | MeI | V | L | S | L | D | V | P | I | G | L | L | Q | I | L | L | A | Q | A | R | A | R | A | A | R | E | Q | A | K* | T | N | Aib | E | I | Nle | A | E | V | 10.5 | 6.0 | > Ref. 1 |
| 198 | MeI | V | L | S | L | D | V | P | I | G | L | L | Q | I | L | L | R | Q | A | R | A | R | A | A | R | E | Q | A | K* | T | N | Aib | E | I | Nle | A | E | V | 10.5 | 4.2 | > Ref. 1 |
| 202 | MeI | V | L | S | L | D | V | P | I | G | L | L | Q | I | L | L | Q | Q | A | R | A | R | A | A | R | E | Q | A | K* | T | N | Aib | E | I | Nle | A | E | V | 10.6 | 6.1 | > Ref. 1 |
| 204 | MeI | V | L | S | L | D | V | P | I | G | L | L | Q | I | L | L | G | Q | A | R | A | R | A | A | R | E | Q | A | K* | T | N | Aib | E | I | Nle | A | E | V | 10.6 | 6.0 | > Ref. 1 |
| 207 | MeI | V | L | S | L | D | V | P | I | G | L | L | Q | I | L | L | H | Q | A | R | A | R | A | A | R | E | Q | A | K* | T | N | Aib | E | I | Nle | A | E | V | 10.4 | 6.0 | > Ref. 1 |
| 209 | MeI | V | L | S | L | D | V | P | I | G | L | L | Q | I | L | L | I | Q | A | R | A | R | A | A | R | E | Q | A | K* | T | N | Aib | E | I | Nle | A | E | V | 10.7 | 5.5 | > Ref. 1 |
| 229 | MeI | V | L | S | L | D | V | P | I | G | L | L | Q | I | L | L | L | Q | A | R | A | R | A | A | R | E | Q | A | K* | T | N | Aib | E | I | Nle | A | E | V | 10.1 | 5.2 | > Ref. 1 |
| 230 | MeI | V | L | S | L | D | V | P | I | G | L | L | Q | I | L | L | K | Q | A | R | A | R | A | A | R | E | Q | A | K* | T | N | Aib | E | I | Nle | A | E | V | 10.3 | 4.5 | > Ref. 1 |
| 211 | MeI | V | L | S | L | D | V | P | I | G | L | L | Q | I | L | L | F | Q | A | R | A | R | A | A | R | E | Q | A | K* | T | N | Aib | E | I | Nle | A | E | V | 10.6 | 4.1 | > Ref. 1 |
| 215 | MeI | V | L | S | L | D | V | P | I | G | L | L | Q | I | L | L | S | Q | A | R | A | R | A | A | R | E | Q | A | K* | T | N | Aib | E | I | Nle | A | E | V | 10.6 | 5.5 | > Ref. 1 |
| 217 | MeI | V | L | S | L | D | V | P | I | G | L | L | Q | I | L | L | T | Q | A | R | A | R | A | A | R | E | Q | A | K* | T | N | Aib | E | I | Nle | A | E | V | 10.4 | 6.0 | > Ref. 1 |
| 231 | MeI | V | L | S | L | D | V | P | I | G | L | L | Q | I | L | L | V | Q | A | R | A | R | A | A | R | E | Q | A | K* | T | N | Aib | E | I | Nle | A | E | V | 10.2 | 5.6 | > Ref. 1 |
| 140 | MeI | V | L | S | L | D | V | P | I | G | L | L | Q | I | L | L | E | Q | A | R | A | R | A | A | R | E | Q | A | K* | T | N | Aib | E | I | Nle | A | E | I | 11.9 | 6.4 | > Ref. 1 |
| 124 (Ref. 2) | MeI | V | L | S | L | D | V | P | I | G | L | L | Q | I | L | L | E | Q | A | R | A | R | A | A | R | E | Q | A | K* | Aib | N | A | E | I | Nle | A | E | V | 11.2 | 6.6 | 40378 |
| 119 | MeI | V | L | S | L | D | V | P | I | G | L | L | Q | I | L | L | A | Q | A | R | A | R | A | A | R | E | Q | A | K* | Aib | N | A | E | I | Nle | A | E | V | 10.7 | 6.0 | >Ref. 2 |
| 232 | MeI | V | L | S | L | D | V | P | I | G | L | L | Q | I | L | L | R | Q | A | R | A | R | A | A | R | E | Q | A | K* | Aib | N | A | E | I | Nle | A | E | V | 9.8 | 4.3 | >Ref. 2 |
| 123 | MeI | V | L | S | L | D | V | P | I | G | L | L | Q | I | L | L | G | Q | A | R | A | R | A | A | R | E | Q | A | K* | Aib | N | A | E | I | Nle | A | E | V | 11.3 | 6.1 | >Ref. 2 |
| 126 | MeI | V | L | S | L | D | V | P | I | G | L | L | Q | I | L | L | H | Q | A | R | A | R | A | A | R | E | Q | A | K* | Aib | N | A | E | I | Nle | A | E | V | 11.0 | 6.1 | >Ref. 2 |
| 235 | MeI | V | L | S | L | D | V | P | I | G | L | L | Q | I | L | L | L | Q | A | R | A | R | A | A | R | E | Q | A | K* | Aib | N | A | E | I | Nle | A | E | V | 9.9 | 3.8 | >Ref. 2 |
| 233 | MeI | V | L | S | L | D | V | P | I | G | L | L | Q | I | L | L | K | Q | A | R | A | R | A | A | R | E | Q | A | K* | Aib | N | A | E | I | Nle | A | E | V | 10.4 | 5.5 | >Ref. 2 |
| 128 | MeI | V | L | S | L | D | V | P | I | G | L | L | Q | I | L | L | F | Q | A | R | A | R | A | A | R | E | Q | A | K* | Aib | N | A | E | I | Nle | A | E | V | 11.3 | 4.0 | >Ref. 2 |
| 132 | MeI | V | L | S | L | D | V | P | I | G | L | L | Q | I | L | L | S | Q | A | R | A | R | A | A | R | E | Q | A | K* | Aib | N | A | E | I | Nle | A | E | V | 10.7 | 6.1 | >Ref. 2 |
| 134 | MeI | V | L | S | L | D | V | P | I | G | L | L | Q | I | L | L | T | Q | A | R | A | R | A | A | R | E | Q | A | K* | Aib | N | A | E | I | Nle | A | E | V | 11.1 | 6.1 | >Ref. 2 |
| 138 | MeI | V | L | S | L | D | V | P | I | G | L | L | Q | I | L | L | V | Q | A | R | A | R | A | A | R | E | Q | A | K* | Aib | N | A | E | I | Nle | A | E | V | 10.8 | 6.0 | >Ref. 2 |

The results in Table 5 confirm the potency improving effects of $X^{38}$ = Nle and the selectivity improving effect of substituting position $X^{20}$ when $X^{38}$ = Nle. Also, the selectivity retaining effect of substituting position $X^{41}$ is shown. The data further demonstrates that high chemical stability (i.e. obtained by position $X^{33}$ or $X^{35}$), high physical stability (i.e. no fibrillation) and high solubility (obtained by positions $X^{36}$ and $X^{40}$) are preserved when introducing these substitutions.

**Table 5**

| SEQ ID NO: | Substitutions relative to comparator | hCRHR2 $EC_{50}$ (nM) | hCRHR1 $EC_{50}$ (nM) | Selectivity ratio (hCRHR1/hCRHR2) | Chemical stability at pH 7.5 after 14 days / 28 days at 40°C (% degradation) | Fibrillation (pH 7.5) | Solubility pH 7.5, target conc. =10 mg/mL (measured mg/mL) |
|---|---|---|---|---|---|---|---|
| 3 (Comparator) | - | 1.1 | >5000 | >4546 | -6.1 / -15 | No | 10 |
| 45 | $X^{38}$ = Nle | 0.52 | 56 | 107 | ND | ND | ND |
| 83 | $X^{20}$ = S; $X^{38}$ = Nle | 0.55 | >5000 | >9091 | -3.2 / -10 | No | 9.5 |
| 69 | $X^{38}$ = Nle; $X^{41}$ = I($NH_2$) | 0.54 | >5000 | >9259 | -0.5 / -8.8 | No | 10 |
| 89 | $X^{20}$ =S; $X^{38}$ =Nle; $X^{41}$ = I($NH_2$) | 0.55 | >5000 | >9091 | ND | ND | ND |
| 234 | $X^{20}$ = S; $X^{35}$ = Aib; $X^{38}$ = Nle | 0.8 | >5000 | >6250 | -0.43 / -1.1 | No | 11 |
| 100 | $X^{35}$ = $X^{41}$ = I | 0.77 | >5000 | >6494 | -0.43 / -0.8 | No | 11 |
| 236 | $X^{33}$ = Aib; $X^{38}$ = Nle; $X^{41}$ = I | 0.67 | >5000 | >7463 | -1/-3 | No | 10 |
| 237 | $X^{20}$ = S; $X^{33}$ = Aib; $X^{38}$ = Nle | 0.77 | >5000 | >6494 | -1.1 /-2.8 | No | 10 |
| 239 | $X^{20}$ = S; $X^{33}$ = E; $X^{38}$ = Nle | 0.9 | >5000 | >5556 | -1.9 / -2.2 | No | 11 |
| 132 | $X^{4}$ = MeI; $X^{20}$ = S; $X^{33}$ = Aib; $X^{38}$ = Nle | 0.77 | >5000 | >6494 | -1.6 / -1.3 | No | 11 |
| 238 | $X^{4}$ = P; $X^{20}$ = S; $X^{33}$ = Aib; $X^{38}$ = Nle | 0.79 | >5000 | >6329 | -1.6 / -4.6 | No | 11 |
| 215 | $X^{4}$ = MeI; $X^{20}$ = S; $X^{35}$ = Aib; $X^{38}$ = Nle | 0.87 | >5000 | >5747 | -0.92 / 0.31 | No | 12 |
| 101 | $X^{4}$ = MeI; $X^{20}$ = S; $X^{33}$ = E; $X^{38}$ = Nle | 0.92 | >5000 | 5435 | -2.2 / -4.3 | No | 11 |

## Example 6 - Extended biophysical characterization of selected compounds

*Solubility of selected peptides at varying pH*

The solubility of selected compounds with improved chemical stability was tested up to 20 mg/mL at varying pH with/without preservative, here phenol was used at preservative. Compounds were dissolved to a nominal concentration of 4000 μM and analysed as described above. Samples with phenol contained 5.0 mg/ml phenol. The measured concentration of peptides in solution are shown in the Table 6.

**Table 6. Solubility of selected peptides**

| SEQ ID NO: | pH 6.5 (mg/ml) | pH 7.0 (mg/mL) | pH 7.5 (mg/mL) | pH 7.5 + phenol (mg/mL) |
|---|---|---|---|---|
| 6 | 21.1 | 20.0 | 18.7 | 18.1 |
| 238 | 20.4 | 20.6 | 18.0 | 17.9 |
| 16 | 20.6 | 19.8 | 17.9 | 18.0 |
| 215 | 19.6 | 20.6 | 17.7 | 18.0 |

In conclusion, all peptides were soluble up to 20 mg/mL in the pH range pH 6.5 - 7.5. At pH 7.5, all peptides were soluble up to at least 20 mg/mL in the presence of 5.0 mg/mL phenol.

*Chemical stability of selected peptides*

The chemical stability of selected peptides was evaluated at varying pH's at 40° C over 28 days. Samples were prepared and analysed as described in procedure with the addition of pH adjusting samples to the targets indicated below. As a comparator,r commercially available semaglutide drug product (Ozempic®, Novo Nordisk) was included in the sample set. The obtained results are shown in the Table 7 below.

**Table 7. Chemical stability of selected peptides**

| SEQ ID NO: | pH | Purity loss (RP-UPLC analysis) | | HMWP formation (SEC analysis) | |
|---|---|---|---|---|---|
| | | T14-T0 (%) | T28-T0 (%) | T14-T0 (%) | T28-T0 (%) |
| 6 | 7.0 | -1.8 | -2.0 | -0.1 | 0.1 |
| 6 | 7.5 | -1.1 | -2.6 | -0.4 | -0.4 |
| 6 | 8.0 | -3.1 | -7.1 | -0.2 | 0.0 |
| 238 | 7.0 | -1.0 | -1.2 | 0.3 | 0.5 |
| 238 | 7.5 | -3.1 | -4.8 | 0.4 | 0.4 |
| 238 | 8.0 | -5.1 | -9.8 | -0.1 | 0.2 |
| 16 | 7.0 | -0.6 | -1.1 | -0.3 | -0.3 |
| 16 | 7.5 | -0.1 | -0.3 | 0.0 | 0.0 |
| 16 | 8.0 | -0.5 | -1.1 | -0.2 | -0.3 |
| 215 | 7.0 | 0.2 | -0.3 | -0.3 | -0.3 |
| 215 | 7.5 | 0.1 | -0.1 | 0.1 | 0.3 |
| 215 | 8.0 | -0.4 | -0.7 | 0.0 | -0.1 |
| Semaqlutide | 7.4 | -2.2 | -2.8 | 0.7 | 0.9 |

All peptides showed a chemical degradation below 10% after 28 days at 40°C. No HMWP formation was observed for all peptides.

*Dynamic light scattering (DLS) measurements of particles size*

Samples were prepared and measured as described in procedure. The hydrodynamic radii (Rh) and associated polydispersity (PD) resulting from cumulant analysis (assuming one species) are shown in the Table 8 as averages of three replica. T0 indicates the measurement at time zero after sample preparation; T5 indicates the measurements of five days of incubation as described above.

**Table 8**

| SEQ ID NO | Timepoint | Rh (nm) | PD (%) |
|---|---|---|---|
| 6 | T0 | 5.8 | 38.5 |
| 6 | T5 | 30.6 | Multimodal |
| 238 | T0 | 4.8 | 5.3 |
| 238 | T5 | 4.8 | 8.1 |
| 16 | T0 | 4.7 | 10.5 |
| 16 | T5 | 4.7 | 11.6 |
| 215 | T0 | 5.6 | 10.8 |
| 215 | T5 | 5.9 | 15.1 |
| Liraglutide pH 8.2 | T0 | 2.1 | 1.4 |
| Liraglutide pH 8.2 | T5 | 2.1 | 3.8 |
| Liraglutide pH 6.7 | T0 | 2.5 | 6.0 |
| Liraglutide pH 6.7 | T5 | 5.6 | Multimodal |

In conclusion, SEQ ID NO: 6 and liraglutide pH 6.7 formed larger particles and several species (multimodal) during the five days incubation at 40°C with continuously shaking, whereas all the other compounds showed no or only minor increases in hydrodynamic radii indicating high physical/colloidal stability during the incubation.

*Amyloid fibril formation assessed using a ThT assay*

Compounds were dissolved to 200 μM (ca 1.0 mg/ml) in 50 mM phosphate buffer with preparation and ThT assay performed as described in the *"General procedure for determination of fibril formation of peptides."* above. Furthermore, samples were adjusted to pH values as indicated below. Additional co-formulations of compounds with semaglutide were prepared by dissolving compounds to 200 μM in commercially available semaglutide drug product (Ozempic®); these co-formulations were adjusted to pH 7.4. Amyloid fibril formation detected by an increase in ThT fluorescence was denoted with "Yes" whereas samples without increasing ThT fluorescence hence not forming amyloid fibrils were denoted "No". The results are summarized in Table 9.

**Table 9**

| SEQ ID NO: | pH 7.0 | pH 7.5 | pH 8.0 | + semaglutide (Ozempic®), pH 7.4 |
|---|---|---|---|---|
| 6 | No | No | No | No |
| 238 | No | No | No | No |
| 16 | No | No | No | No |
| 215 | No | No | No | No |
| None | n.a. | n.a | n.a. | No |

In conclusion, none of the tested compounds formed amyloid fibrils in the pH range pH 7.0 - 8.0 in 50 mM phosphate. All compounds could be co-formulated with semaglutide without any observation of immediate precipitation or amyloid fibril formation.

**Example 7 - Potency characterization of selected compounds**

*Different species*

Selected peptides were tested for potency on different species (mouse, rat, porcine) using transiently transfected CHO-K1 cells using the general procedure.

Table 10 shows $EC_{50}$ values (average, n=3) of selected peptides tested against human, mouse, rat and porcine CRH 1 and 2 receptors using transiently transfected CHO-K1 cell systems (pcDNA3.1(+)-N or C-DYK from Genscript, rCRHR2 NM_022714.1 (rat), rCRHR1 NM_030999.4 (rat), mCRHR2 NM_001288618.1 (mouse), mCRHR1 NM_007762.5 (mouse), pCRHR1 (pig), pCRHR2 (pig), hCRHR1 NM_004382.5 (human), hCRHR2a NM_001883.5 (human), hCRHR2b NM_001202475.1 (human)).

**Table 10**

| SEQ ID NO: | Substitutions relative to SEQ ID NO: 3 | Stable EC50 (nM) | Transient cell system EC50 (nM) | | | | Stable EC50 (nM) | Transient cell system EC50 (nM) | | | | |
|---|---|---|---|---|---|---|---|---|---|---|---|---|
| | | hCRHR 1 | hCR HR1 | mCRH R1 | rCRH R1 | pCRHR 1 | hCRHR 2a | hCRHR 2a | hCRHR 2b | mCRH R2 | rCRHR 2 | pCR HR2 |
| 3 | - | >5000 | > 1000 | 44 | 163 | >5000 | 1.1 | 0.016 | 0.0081 | 0.0054 | 0.070 | 0.027 |
| 6 | $X^4$ = Mel; $X^{32}$ = T; $X^{33}$ = K(Lip) | >5000 | > 1000 | 71 | 275 | >5000 | 3.0 | 0.023 | 0.015 | 0.0043 | 0.13 | 0.030 |
| 238 | $X^4$ = P;$X^{20}$ =S;$X^{33}$=Aib; $X^{38}$=Nle | >5000 | >5000 | 323 | > 1000 | >5000 | 0.79 | 0.018 | 0.016 | 0.0047 | 0.067 | 0.015 |
| 16 | $X^4$=Mel; $X^{35}$=Aib | >5000 | > 1000 | 76 | 256 | >5000 | 2.7 | 0.025 | 0.012 | 0.0049 | 0.19 | 0.028 |
| 215 | $X^4$=Mel; $X^{20}$=S; $X^{35}$=Aib; $X^{38}$=Nle | >5000 | 826 | 50 | 175 | > 1000 | 0.87 | 0.013 | 0.016 | 0.0040 | 0.072 | 0.017 |

Overall ranking of the peptides was the same for the four species. Less than 10-fold differences in $EC_{50}$-values between human and tested species were obtained for the transient transfected cell systems. High level of correlation ($R^2$ = 0.81) was seen for the two isoforms of the human CRHR2 receptor (hCRHR2a vs hCRHR2b).

**Example 8** - **Pharmacokinetic characterization of selected peptides**

The pharmacokinetic properties of selected peptides were assessed in mice, rats, and pigs according to the general procedures described above. The results of these studies are presented in Tables 11, 12, 13 and 14 below.

**Table 11**

| | Rat | | Mouse | | Pig | |
|---|---|---|---|---|---|---|
| SEQ ID NO: | T½ | MRT | T½ | MRT | T½ | MRT |
| 3 | 7.7 | 9.2 | 13.5 | 17.5 | 156 | 235 |
| 5 | 8.6 | 7.9 | 14.3 | 21 | 162 | 218 |
| 4 | 5.3 | 6.4 | 14.8 | 18.2 | NA | NA |
| 6 | 6.1 | 7.4 | 14.7 | 16 | 145 | 195 |
| 14 | 5.1 | 6.1 | NA | NA | NA | NA |
| 15 | 7.7 | 8 | NA | NA | NA | NA |
| 19 | 5.9 | 7.4 | NA | NA | NA | NA |
| 17 | 7.1 | 7.6 | NA | NA | NA | NA |
| 234 | 4.1 | 5.1 | NA | NA | NA | NA |
| 237 | 4.1 | 5.1 | NA | NA | NA | NA |
| 132 | 4.8 | 6.2 | 11.2 | 16 | 122 | 162 |
| 238 | 10.2 | 14.2 | 19.6 | 21.4 | 212 | 293 |
| 16 | 7.8 | 9.4 | 15.9 | 21.5 | 208 | 278 |
| 215 | 5.7 | 6.7 | 12.6 | 17.4 | 156 | 211 |
| 101 | 6.5 | 6.8 | NA | NA | NA | NA |

**Table 12**

| SEQ ID NO: | Species | Treatment | T½ (h) | Cl (L/h/kg) | F (%) | Cmax (nmol/L) | tmax (h) |
|---|---|---|---|---|---|---|---|
| 3 | Mouse | 50 nmol/kg i.v. | 13.5 | <0.01 | - | - | - |
| | | 50 nmol/kg s.c. | 11.4 | - | 43.1 | 141 | 24 |
| 6 | Mouse | 15 nmol/kg i.v. | 14.7 | 0.0033 | - | - | - |
| | | 15 nmol/kg s.c. | 16 | | 78 | 90 | 24 |
| 238 | Mouse | 15 nmol/kg i.v. | 19.6 | 0.0044 | - | - | - |
| | | 15 nmol/kg s.c. | 25.2 | - | 60 | 48 | 24 |
| 16 | Mouse | 15 nmol/kg i.v. | 15.9 | 0.0037 | - | - | - |
| | | 15 nmol/kg s.c. | 14.8 | - | 62 | 60 | 6 |
| 215 | Mouse | 15 nmol/kg i.v. | 12.6 | 0.004 | - | - | - |
| | | 15 nmol/kg s.c. | 14.1 | - | 56 | 58 | 6 |

| Table 13 | | | | | | | |
|---|---|---|---|---|---|---|---|
| SEQ ID NO: | Species | Treatment | T½ (h) | Cl (L/h/kg) | F (%) | Cmax (nmol/L) | tmax (h) |
| 3 | Rat | 50 nmol/kq i.v. | 7.7 | 0.005 | - | - | - |
| | | 50 nmol/kg s.c. | 7.9 | - | 27 | 90 | 24 |
| 6 | Rat | 50 nmol/kg i.v. | 6.1 | 0.009 | - | - | - |
| | | 50 nmol/kg s.c. | 8.4 | - | 35 | 70 | 24 |
| 238 | Rat | 50 nmol/kg i.v. | 10.2 | 0.007 | - | - | - |
| | | 50 nmol/kg s.c. | 11 | - | 43 | 91 | 24 |
| 16 | Rat | 50 nmol/kg i.v. | 7.8 | 0.007 | - | - | - |
| | | 50 nmol/kg s.c. | 9.8 | - | 31 | 87 | 24 |
| 215 | Rat | 50 nmol/kq i.v. | 5.7 | 0.012 | - | - | - |
| | | 50 nmol/kg s.c. | 6.1 | - | 28 | 42 | 24 |

| Table 14 | | | | | | | |
|---|---|---|---|---|---|---|---|
| SEQ ID NO: | Species | Treatment | T½ (h) | Cl (L/h/kg ) | F (%) | Cmax(nmol/L) | tmax (h) |
| 3 | Pig | 6.0 nmol/kg i.v. | 156 | 0.0003 0 | - | - | - |
| | | 6.0 nmol/kg s.c | - | - | 55 | 60 | 24 |
| 6 | Pig | 6.0 nmol/kg i.v. | 145 | 0.0003 4 | - | - | - |
| | | | | | | | |
| 238 | Pig | 5.8 nmol/kg i.v. | 212 | 0.0002 8 | - | - | - |
| | | 6.0 nmol/kg s.c | - | - | 86 | 44 | 48 |
| 16 | Pig | 4.6 nmol/kq i.v. | 208 | 0.0002 8 | - | - | - |
| | | 6.0 nmol/kg s.c | - | - | 66 | 35 | 48 |
| 215 | Pig | 4.2 nmol/kg i.v. | 156 | 0.0003 | - | - | - |
| | | 6.0 nmol/kg s.c | - | - | 68 | 38 | 24 |

In conclusion, all evaluated peptides showed favourable pharmacokinetic profiles compatible with once weekly dosing in humans.

**Example 9 - Effect of selected peptides on acute food intake in mice**

Selected compounds were tested according to the general procedure on acute food intake in mice to determine the effect of a single subcutaneous dose on food intake in mice over 3 days. The results are shown in Table 15 below.

| Table 15 - Acute food intake relative to vehicle 24 hours post dosing | | | |
|---|---|---|---|
| | % FI compared to vehicle, 24 h post-dosing | | |
| SEQ ID NO: | 30 nmol/kg | 100 nmol/kg | 300 nmol/kg |
| 3 | 67.63*** | 30.75*** | 30.66*** |
| 5 | 45.67*** | 18.68*** | 15.93*** |
| 4 | 63.41*** | 19.33*** | 18.14*** |
| 6 | 51.61*** | 25.76*** | 12.69*** |
| 19 | 56.47*** | 29.68*** | 21.66*** |
| 17 | 78.97* | 48.64*** | 19.52*** |

(continued)

| Table 15 - Acute food intake relative to vehicle 24 hours post dosing | | | |
|---|---|---|---|
| | % FI compared to vehicle, 24 h post-dosing | | |
| SEQ ID NO: | 30 nmol/kg | 100 nmol/kg | 300 nmol/kg |
| 237 | 51.49*** | 16.66*** | 13.2*** |
| 132 | 46.64*** | 18.5*** | 20.04*** |
| 238 | 29.73*** | 12.92*** | 7.94*** |
| 16 | 44.62*** | 18.21*** | 12.36*** |
| 215 | 38.67*** | 20.26*** | 10.31*** |
| 101 | 30.85*** | 21.44*** | 8.09*** |
| Dunnett's test one-factor linear mode, compared to Vehicle: *: p < 0.05 , **: p < 0.01, ***: p < 0.001 | | | |

In conclusion, all selected peptides demonstrated dose dependent reduction in food intake compared to vehicle treated animals.

**Example 10: Assessment of effect on body weight and body composition in DIO mice**

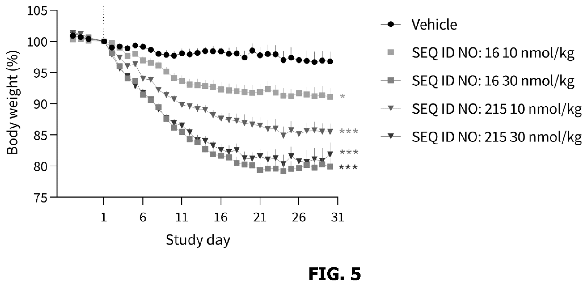

The effect of selected peptides on body composition were assessed in DIO mice. The results of the two DIO mouse model studies are presented in Tables 16 and 17 and in Fig. 5.

Treatment with SEQ ID NO: 3, SEQ ID NO: 6, SEQ ID NO: 238, SEQ ID NO: 16 and SEQ ID NO: 215 for 28 days resulted in a prolonged dose-dependent reduction in relative body weight when compared to vehicle treated animals. For groups dosed with 30 nmol/kg, bodyweight was reduced with approximately 20 % of the initial body weight.

| Table 16 | | | | | |
|---|---|---|---|---|---|
| | Vehicle | SEQ ID NO: 3 | | | |
| Dose [nmol/kg] | NA | 10 | 30 | 60 | 100 |
| BW at termination [g ± SEM] | 47.7 ± 1.6 | 41.9 ± 1.5** | 38.2 ± 1.0*** | 38.5 ± 0.6*** | 37.3 ± 0.6*** |
| Relative body weight change at termination [% ± SEM] | 100.6 ± 1.0 | 94.3 ± 1.6* | 89.0 ± 2.2*** | 89.9 ± 1.9*** | 87.7 ± 1.3*** |
| Fat mass change from baseline [g ± SEM] | -0.9 ± 0.6 | -7.7 ± 0.7*** | -10.2 ± 0.6*** | -10.8 ± 0.6*** | -11.2 ± 0.6*** |
| Lean mass change from baseline [g ± SEM] | -0.8 ± 0.3 | 1.4 ± 0.3*** | 0.4 ± 0.4* | 0.8 ± 0.3*** | 0.8 ± 0.2*** |
| M. Soleus [g] | 0.009 ± 0.0004 | 0.011 ± 0.0005 | 0.012 ± 0.0005** | 0.012 ± 0.0004** | 0.011 ± 0.0006* |
| M. Gastrocnemius [g] | 0.15 ± 0.003 | 0.17 ± 0.005*** | 0.17 ± 0.003** | 0.16 ±0.004* | 0.17 ± 0.003*** |
| Blood HbA1c [%] | 4.5 ± 0.04 | 4.3 ± 0.1 | 4.1 ± 0.1*** | 4.15 ± 0.1** | 4.1 ± 0.1** |
| Dunnett's test one-factor linear model compared to DIO Vehicle: *: p < 0.05, **: p < 0.01, ***: p < 0.001 | | | | | |

| Table 17 | | | | | | | | | | |
|---|---|---|---|---|---|---|---|---|---|---|
| | | SEQ ID NO: | | | | | | | | |
| | Vehicle | 3 | 6 | | 238 | | 16 | | 215 | |
| Dose [nmol/kg] | NA | 30 | 10 | 30 | 10 | 30 | 10 | 30 | 10 | 30 |

(continued)

| Table 17 | | | | | | | | | | |
|---|---|---|---|---|---|---|---|---|---|---|
| | | SEQ ID NO: | | | | | | | | |
| | Vehicle | 3 | 6 | | 238 | | 16 | | 215 | |
| Body weight at termination [g ± SEM] | 46.3 ± 1.1 | 39.3 ± 0.9*** | 42.6 ± 1.3 | 38 ± 0.5*** | 39.1 ± 0.7*** | 37.4 ± 0.9*** | 44 ± 1.1 | 38.3 ± 0.9*** | 40.5 ± 1.2*** | 38.9 ± 1.1*** |
| Relative body weight change at termination [% ± SEM] | 96.8 ± 1.5 | 82.4 ± 1.6*** | 89.1 ± 2.0** | 79.3 ± 1.2*** | 82.3 ± 1.2*** | 77.9 ± 0.7*** | 91.1 ± 1.2* | 79.9 ± 1.4*** | 85.5 ± 1.3*** | 81.8 ± 1.9*** |
| Fat mass change from baseline [g ± SEM] | -1.5 ± 0.6 | -9.5 ± 0.6*** | -7.0 ± 0.9*** | -10.7 ± 0.5*** | -9.5 ± 0.6*** | -10.7 ± 0.3*** | -5.6 ± 0.5*** | -10.5 ± 0.6*** | -8.7 ± 0.5*** | -9.9 ± 0.7*** |
| Lean mass change from baseline [g ± SEM] | -0.9 ± 0.6 | -0.2 ± 0.2 | 0.1 ± 0.3 | -0.1 ± 0.3 | -0.4 ± 0.2 | -0.7 ± 0.3 | 0.6 ± 0.3* | -0.6 ± 0.3 | 0.1 ± 0.5 | -0.5 ± 0.3 |
| M. Soleus [g] | 0.011 ± 0.0009 | 0.013 ± 0.0006 | 0.012 ± 0.0008 | 0.013 ± 0.0004 | 0.012 ± 0.0003 | 0.011 ± 0.0007 | 0.013 ± 0.0004 | 0.013 ± 0.0009 | 0.012 ± 0.0005 | 0.012 ± 0.0005 |
| M. Gastrocne-mius [g] | 0.18 ± 0.003 | 0.19 ± 0.003** | 0.18 ± 0.004 | 0.18 ± 0.001 | 0.18 ± 0.001 | 0.18 ± 0.004 | 0.19 ± 0.003* | 0.19 ± 0.002* | 0.19 ± 0.002* ** | 0.18 ± 0.003 |
| Blood HbA1c [%] | 4.7 ± 0.03 | 4.2 ± 0.1*** | 4.0 ± 0.1*** | 4.1 ± 0.1*** | 4.3 ± 0.04** | 4.4 ± 0.1** | 4.4 ± 0.1 | 4.1 ± 0.1*** | 4.3 ± 0.1*** | 4.4 ± 0.03* |
| Dunnett's test one-factor linear model compared to DIO Vehicle: *: p < 0.05, **: p < 0.01, ***: p < 0.001 | | | | | | | | | | |

The data demonstrated a nice correlation between in vitro potencies and in vivo effects. Fig. 5 shows SEQ ID NO: 215 compared to GSEQ ID NO: 16, where SEQ ID NO: 215 gives a relative higher body weight loss when dosed at 10 nmol/kg. The significant reduction in body weight after 4 weeks of treatment correlated to loss of fat tissue mass while absolute lean tissue mass was maintained. In line with the MRI results, treatment with the selected peptides resulted in maintained or increased weight of M. Soleus and M. Gastrocnemius. A reduction in blood HbA1c level was observed in groups treated with selected peptides indicating an improved glucose homeostasis.

In conclusion, treatment with the selected peptides resulted in a dose-dependent reduction in body weight and fat tissue mass while lean tissue mass was maintained.

**Example 11: Telemetry in mice**

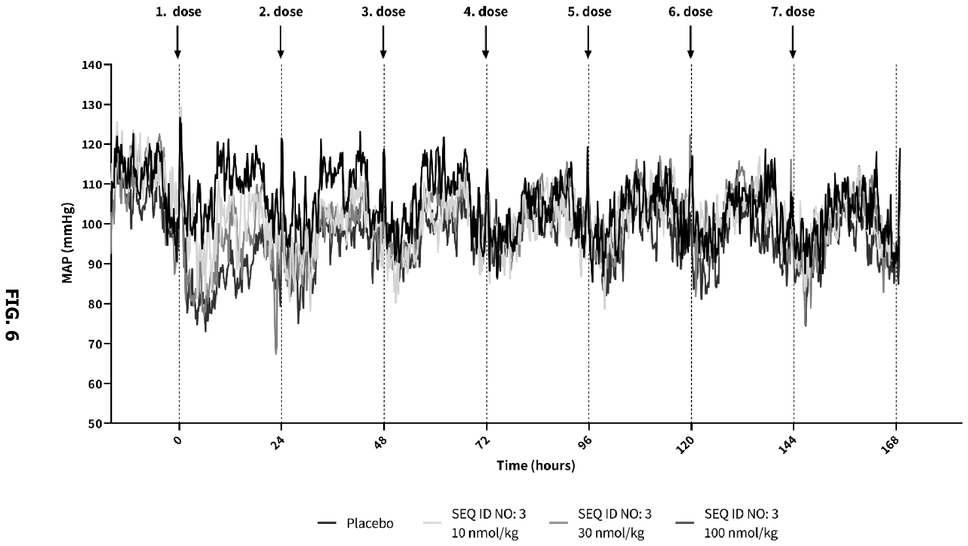

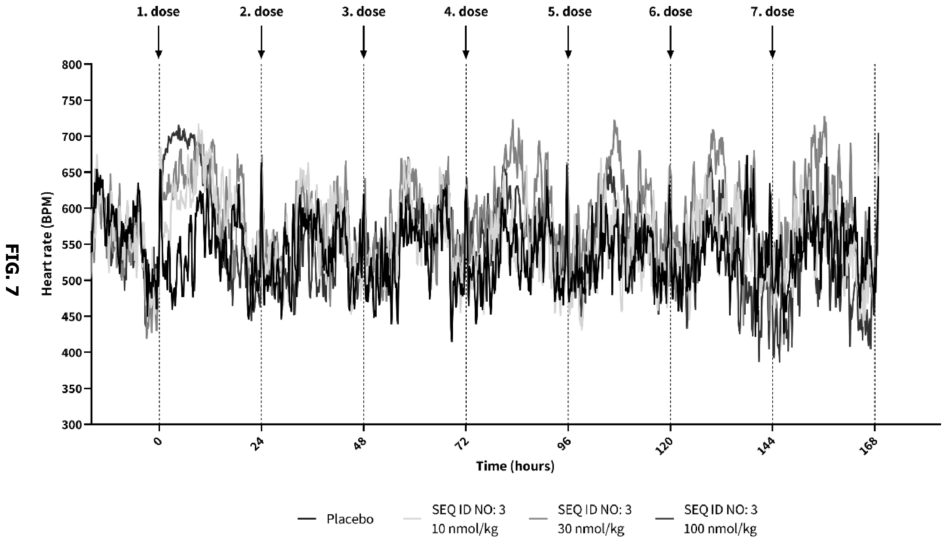

Results from assessment of blood pressure and heart frequency in mice treated with SEQ ID NO: 3 can be seen in Fig. 6 and Table 18 and Fig. 7 and Table 19, respectively. Treatment with SEQ ID NO: 3 resulted in a transient and dose-dependent effect on mean arterial blood pressure and a permanent increase of heart rate.

| Table 18. Mean arterial pressure [mmHg] | | | | |
|---|---|---|---|---|
| | Placebo | SEQ ID NO: 3 | | |
| Dose [nmol/kg] | NA | 10 | 30 | 100 |
| Baseline dark period | 113.22 ± 4.9 | 117.13 ± 6.7 | 111.32 ± 3.0 | 108.55 ± 1.2 |
| 1. dose | 103.26 ± 5.6 | 97.23 ± 3.7 | 83.81 ± 3.9 | 83.22 ± 5.6 |
| 1. dark period | 113.17 ± 6.0 | 102.19 ± 4.0 | 97.14 ± 2.8 | 86.82 ± 2.2* |

(continued)

| Table 18. Mean arterial pressure [mmHg] | | | | |
|---|---|---|---|---|
| | Placebo | SEQ ID NO: 3 | | |
| Dose [nmol/kg] | NA | 10 | 30 | 100 |
| 2. dose | 101.11 ± 5.8 | 89.86 ± 2.5 | 89.68 ± 5.3 | 89.63 ± 4.9 |
| 2. dark period | 112.49 ± 4.8 | 102.50 ± 2.9 | 102.00 ± 4.4 | 98.30 ± 4.8 |
| 3. dose | 97.77 ± 4.5 | 89.07 ± 1.9 | 93.98 ± 4.5 | 92.77 ± 4.1 |
| 3. dark period | 112.94 ± 6.1 | 101.92 ± 1.5 | 106.02 ± 3.9 | 101.88 ± 5.1 |
| 4. dose | 96.31 ± 1.5 | 96.25 ± 1.4 | 94.91 ± 5.0 | 94.17 ± 3.4 |
| 4. dark period | 104.23 ± 1.1 | 103.17 ± 1.7 | 106.67 ± 4.8 | 103.60 ± 5.3 |
| 5. dose | 97.71 ± 3.1 | 93.27 ± 1.8 | 94.31 ± 5.2 | 91.26 ± 2.0 |
| 5. dark period | 105.70 ± 1.2 | 105.59 ± 1.5 | 107.74 ± 5.7 | 103.68 ± 4.4 |
| 6. dose | 96.59 ± 2.0 | 99.01 ± 3.0 | 97.05 ± 6.6 | 88.76 ± 1.3* |
| 6. dark period | 105.54 ± 1.0 | 104.54 ± 1.8 | 109.48 ± 6.3 | 103.45 ± 3.1 |
| Dunnett's test two-way ANOVA compared to Placebo: *: $p < 0.05$ | | | | |

| Table 19. Heart rate [BPM] | | | | |
|---|---|---|---|---|
| | Placebo | SEQ ID NO: 3 | | |
| Dose [nmol/kg] | NA | 10 | 30 | 100 |
| Baseline dark period | 587.74 ± 19.3 | 573.24 ± 24.3 | 573.65 ± 26.2 | 581.49 ± 7.7 |
| 1. dose | 513.32 ± 17.3 | 590.34 ± 26.4 | 637.60 ± 39.0 | 692.31 ± 7.0*** |
| 1. dark period | 569.85 ± 14.6 | 617.82 ± 17.8 | 636.57 ± 28.2 | 593.28 ± 23.1 |
| 2. dose | 517.13 ± 18.7 | 526.42 ± 18.4 | 534.13 ± 15.4 | 507.39 ± 8.1 |
| 2. dark period | 573.19 ± 16.1 | 576.21 ± 15.5 | 579.68 ± 23.9 | 517.97 ± 16.2 |
| 3. dose | 501.98 ± 24.8 | 517.24 ± 12.1 | 524.21 ± 17.2 | 536.49 ± 10.4 |
| 3. dark period | 565.64 ± 14.8 | 570.38 ± 9.8 | 621.30 ± 9.5* | 591.77 ± 7.0 |
| 4. dose | 498.38 ± 22.6 | 521.88 ± 17.1 | 576.95 ± 21.0 | 560.41 ± 16.0 |
| 4. dark period | 534.03 ± 16.2 | 557.65 ± 11.8 | 650.03 ± 9.0*** | 606.21 ± 10.1* |
| 5. dose | 516.13 ± 24.7 | 515.46 ± 3.8 | 552.59 ± 12.9 | 554.35 ± 15.3 |
| 5. dark period | 533.44 ± 16.7 | 548.23 ± 8.3 | 628.77 ± 14.9** | 619.17 ± 11.0** |
| 6. dose | 510.36 ± 30.8 | 538.37 ± 13.6 | 558.58 ± 15.1 | 550.11 ± 16.2 |
| 6. dark period | 538.80 ± 14.2 | 571.18 ± 17.4 | 625.92 ± 11.6** | 612.05 ± 11.8** |
| Dunnett's test two-way ANOVA compared to Placebo: *: $p < 0.05$, **: $p < 0.01$, ***: $p < 0.001$ | | | | |

**Example 12: Telemetry in rats**

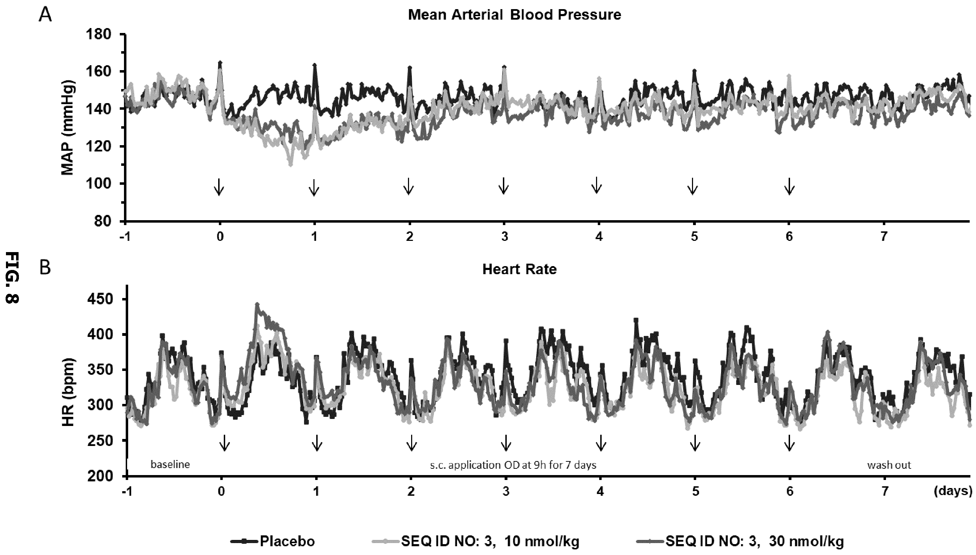

Results from telemetry in rats can be seen in Fig. 8 and in Tables 20 and 21. Treatment with SEQ ID NO: 3 resulted in a transient and dose-dependent decrease in blood pressure accompanied by a transient and dose-dependent increase in HR.

| Table 20. Mean arterial pressure [mmHg] | | | |
|---|---|---|---|
| | Placebo | SEQ ID NO: 3 | |
| Dose [nmol/kg] | NA | 10 | 30 |
| Day -1 | 146.17 ± 1.2 | 145.33 ± 3.1 | 148.88 ± 3.1 |
| Day 0 | 145.67 ± 1.7 | 130.83 ± 2.1*** | 126.25 ± 2.5**** |
| Day 1 | 146.00 ± 1.4 | 130.33 ± 1.8**** | 130.00 ± 2.4**** |
| Day 2 | 146.00 ± 1.6 | 136.17 ± 2.0* | 141.00 ± 3.1 |
| Day 3 | 138.50 ± 1.9 | 139.17 ± 2.3 | 142.50 ± 2.7 |
| Day 4 | 145.33 ± 1.6 | 137.67 ± 2.4 | 141.00 ± 3.1 |
| Day 5 | 147.00 ± 2.0 | 139.00 ± 2.9 | 141.75 ± 2.6 |
| Day 6 | 146.50 ± 1.7 | 138.50 ± 2.4 | 141.75 ± 2.7 |
| Dunnett's test two-way ANOVA compared to Placebo: *: p < 0.05, **: p < 0.01, ***: p < 0.001, ****: p < 0.0001 | | | |

| Table 21. Heart rate [BPM] | | | |
|---|---|---|---|
| | Placebo | SEQ ID NO: 3 | |
| Dose [nmol/kg] | NA | 10 | 30 |
| Day -1 | 331.00 ± 6.5 | 314.50 ± 4.8 | 322.38 ± 5.9 |
| Day 0 | 332.17 ± 6.7 | 345.67 ± 5.3 | 364.50 ± 5.4*** |
| Day 1 | 340.50 ± 6.5 | 330.67 ± 6.2 | 334.00 ± 4.8 |
| Day 2 | 335.50 ± 5.6 | 324.00 ± 5.2 | 327.50 ± 2.7 |
| Day 3 | 352.67 ± 7.1 | 324.00 ± 5.2** | 325.88 ± 4.2** |
| Day 4 | 345.50 ± 4.7 | 323.33 ± 7.4* | 325.38 ± 6.4* |
| Day 5 | 340.00 ± 5.8 | 320.50 ± 7.0 | 323.50 ± 5.5 |
| Day 6 | 331.67 ± 6.9 | 315.67 ± 7.2 | 328.88 ± 5.8 |
| Dunnett's test two-way ANOVA compared to Placebo: *: p < 0.05, **: p < 0.01, ***: p < 0.001 | | | |

**Example 13: Telemetry in pigs**

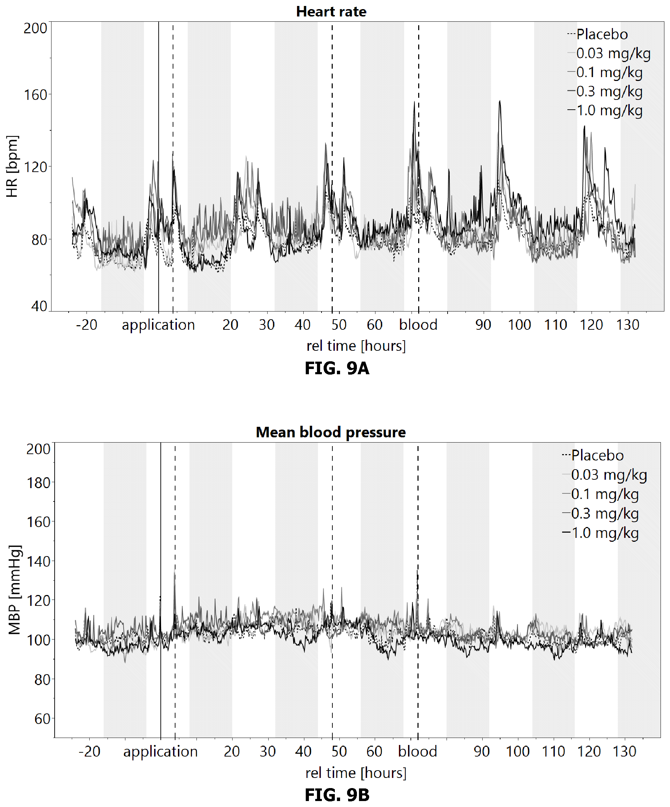

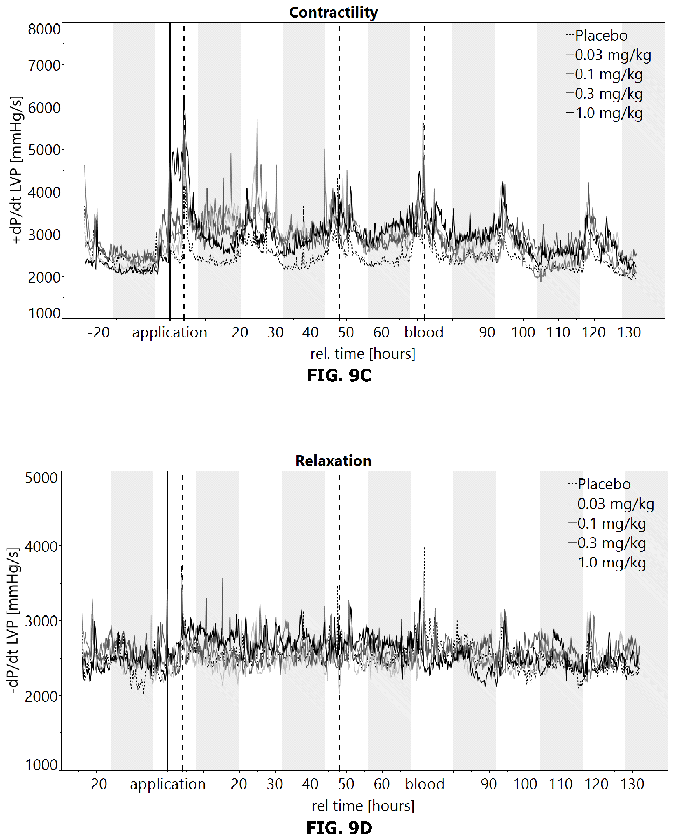

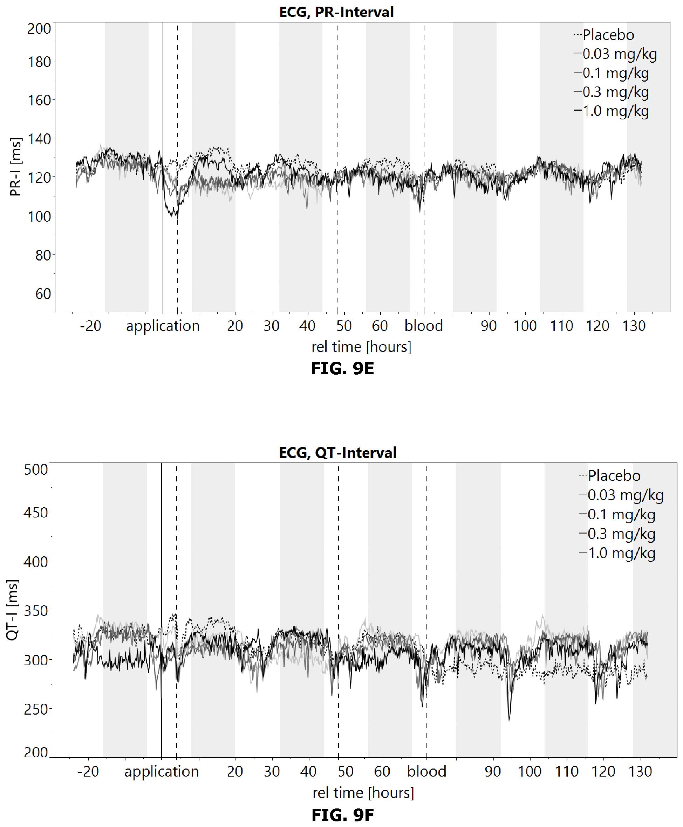

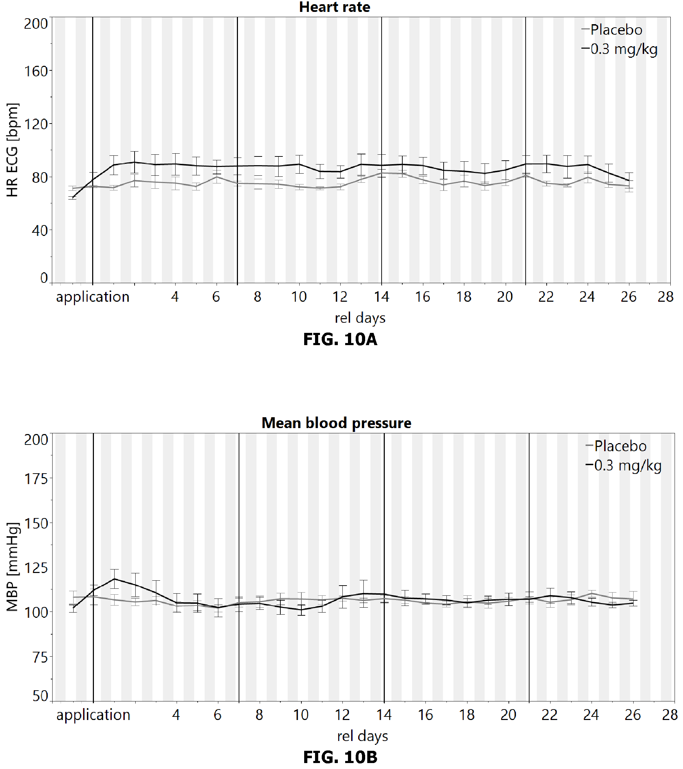

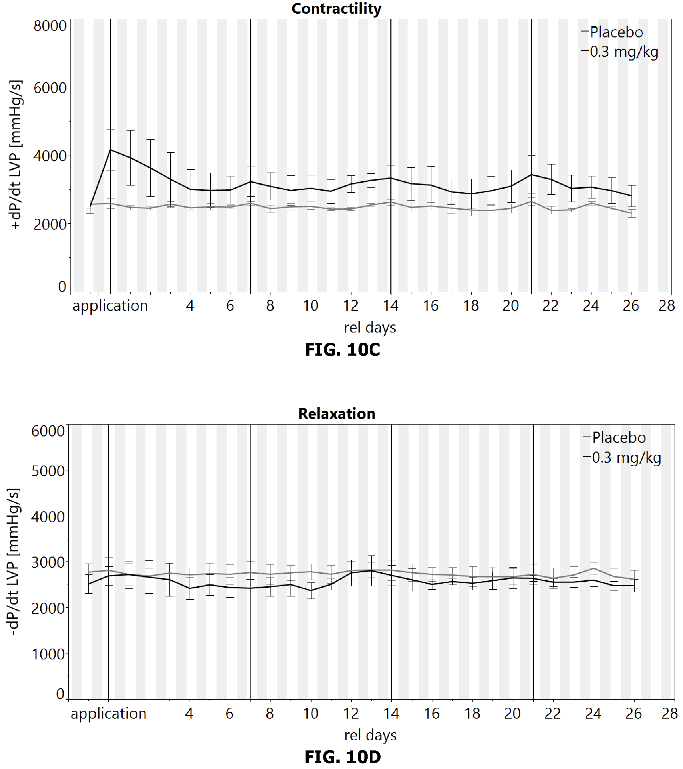

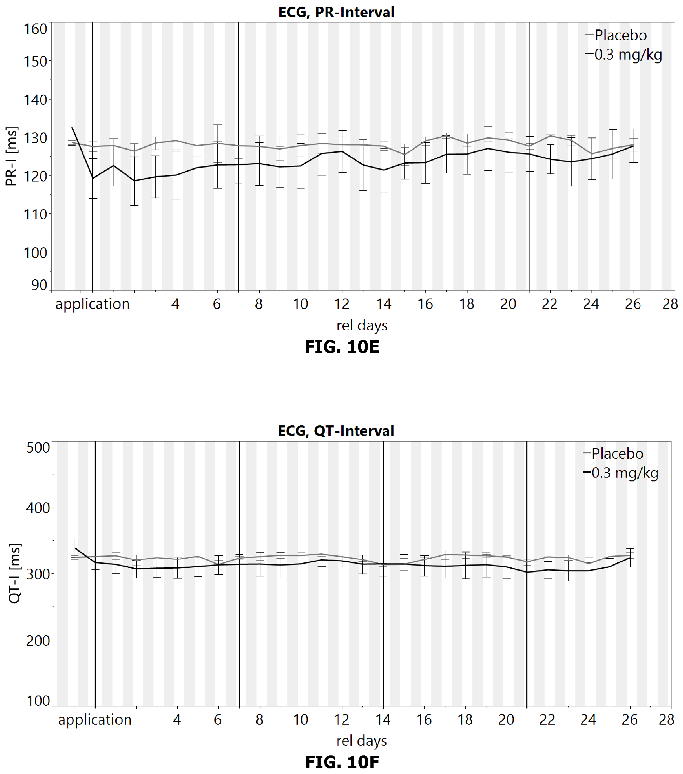

Hemodynamic effects of treatment with either an ascending dose regime (study A, Figs. 9A-F) or repeated dosing with a single dose (study B, Fig. 10A-F) of SEQ ID NO: 3 were assessed as described in the general procedure. Only those animals that showed accurate phasic signals were included in the analysis.

In study A, treatment with SEQ ID NO: 3 resulted in long-lasting positive ionotropic effects whereas relaxation of the left ventricle was not affected. Heart rate and mean arterial blood pressure remained unaffected. A slight decrease in PR-Interval was observed, but QT-Interval were unaffected. No relevant findings on systemic or cardiac hemodynamics were observed as measured by LV end diastolic pressure, time constant of relaxation, baroreceptor sensitivity and heart rate variability. Similar findings were observed in study B. In conclusion, SEQ ID NO: 3 induced long-lasting positive inotropic effects without safety relevant findings.

**Example 14: acute effects of selected peptide on hemodynamics in anesthetized pigs**

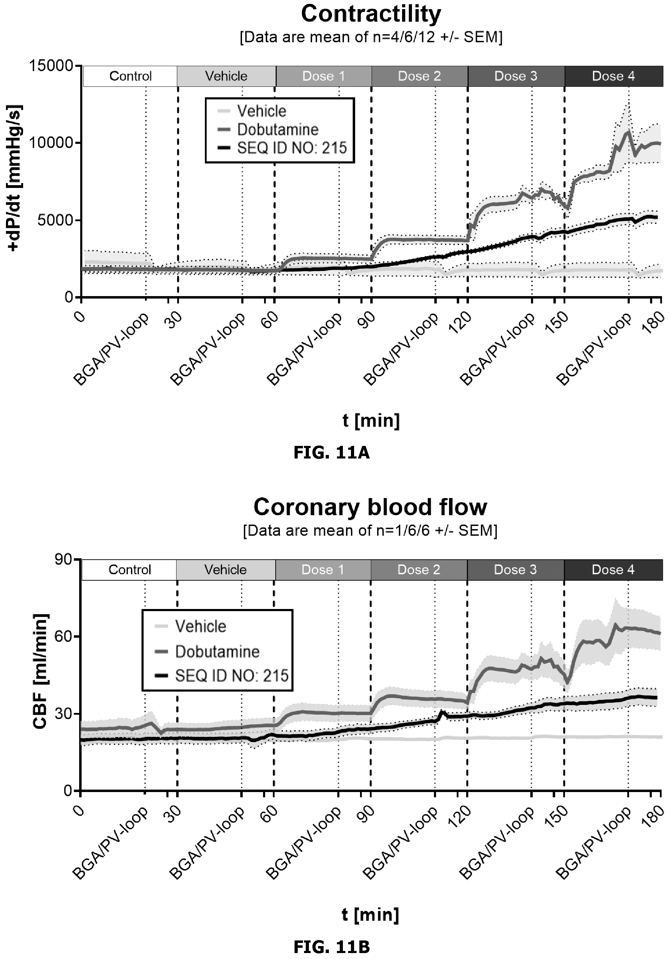

The acute effects of ascending doses of SEQ ID NO: 215 on hemodynamics in anesthetized pigs can be seen in Figs. 11A-D.

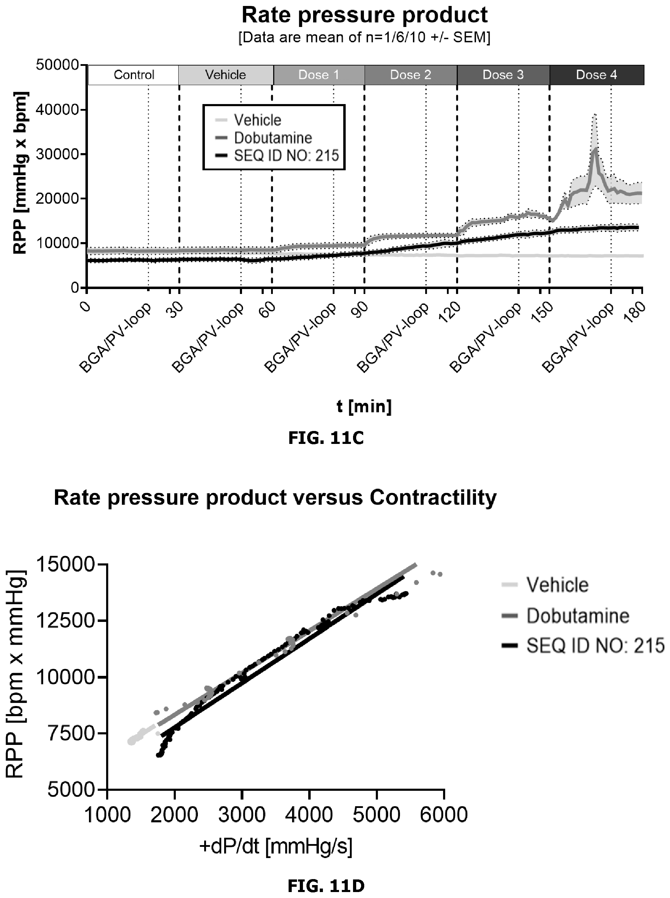

As seen from the telemetry studies, treatment with SEQ ID NO: 215 results in positive inotropic effects (Fig. 11A). This is associated with increasing coronary blood flow (Fig. 11B) and $O_2$ consumption (Figs. 11C and 11D) as seen with treatment with Dobutamine.

In conclusion, SEQ ID NO: 215 showed robust inotropic effects.

**Example 15: Myocardial ischemia model in rats**

The effect of SEQ ID NO: 3 on myocardial infarction was assessed according to the general procedure. Results of 2 months of treatment can be seen in Table 22.

3 months post-MI, 2 months post induction of treatment, the MI-vehicle group displayed left ventricular dysfunction with a reduction in ejection fraction and fractional shortening. In addition, MI-Vehicle animals presented a significant decrease in stroke volume and cardiac output compared to sham operated animals. This was associated with a significant increase in the diastolic parameter; isovolumic relaxation time. Rats treated with SEQ ID NO: 3 showed improved cardiac function compared to vehicle treated MI animals, with significant improvements in ejection fraction, fractional shortening, stroke volume and cardiac output. In comparison, the control treatment (A+L+S) only improved ejection fraction and fractional shortening when compared to vehicle treated MI animals.

Mean blood pressure and heart rate as measured in anesthetized rats prior to termination were reduced in MI animals when compared to sham operated animals. Treatment did not influence these hemodynamic parameters.

At termination, animals treated with SEQ ID NO: 3 weighed significantly more than MI-Vehicle animals. This was associated with a significant increase in muscle mass.

**Table 22**

| | Sham | MI -Vehicle | MI - A+L+S | MI - SEQ ID NO: 3 | |
|---|---|---|---|---|---|
| Dose | NA | NA | 1-1-10 mg/kg | 30 nmol/kg | 100 nmol/kg |
| EF B-mode [% ± SEM] | 80.93 ± 0.9#### | 38.74 ± 1.9 | 43.78 ± 0.9* | 45.52 ± 1.2*** | 45.55 ± 0.7*** |
| EF M-mode [% ± SEM] | 84.15 ± 1.2#### | 39.24 ±1.5 | 44.46 ± 0.9** | 46.42 ± 1.2**** | 46.22 ± 0.7*** |
| FS [% ± SEM] | 48.46 ± 1.4#### | 16.89 ± 0.7 | 19.55 ± 0.5** | 20.63 ± 0.5**** | 20.46 ± 0.4*** |
| SV [ml ± SEM] | 0.41 ± 0.02### | 0.32 ± 0.02 | 0.33 ± 0.01 | 0.41 ± 0.01**** | 0.40 ± 0.02**** |
| CO [ml/min ± SEM] | 144.8 ± 6.3#### | 112.6 ± 3.4 | 115.8 ± 4.6 | 129.0 ± 5.1* | 125.4 ± 3.8 |
| IVRT [ms ± SEM] | 19.25 ± 0.5#### | 24.8 ± 0.5 | 22.67 ± 0.5 | 21.81 ± 0.5* | 20.87 ± 0.3**** |
| LVIDd [mm ± SEM] | 8.03 ± 0.2#### | 10.99 ± 0.2 | 10.43 ± 0.1 | 10.86 ± 0.2 | 10.94 ± 0.1 |
| LVIDs [mm ± SEM] | 4.12 ± 0.2#### | 9.15 ± 0.2 | 8.40 ± 0.1* | 8.63 ± 0.2 | 8.71 ± 0.1 |
| LVPWd [mm ± SEM] | 1.83 ± 0.1# | 1.60 ± 0.1 | 1.53 ± 0.04 | 1.84 ± 0.04** | 1.63 ± 0.1 |
| LVPWs [mm ± SEM] | 3.15 ± 0.05#### | 2.54 ± 0.1 | 2.55 ± 0.06 | 2.94 ± 0.05**** | 2.73 ± 0.05 |
| Mean blood pressure [mmHg] | 127 ± 4.2## | 103.0 ± 5.4 | 107.1 ± 3.0 | 109.9 ± 4.0 | 112.6 ± 4.3 |
| Heart rate [bpm] | 370.2 ± 8.7# | 339.2 ± 8.3 | 355.0 ± 7.3 | 348.8 ± 6.7 | 353.2 ± 9.1 |
| Body weight at termination [g ± SEM] | 534.0 ± 10.2 | 505.5 ± 9.2 | 487.9 ± 11.3 | 550.1 ± 10.6** | 551.6 ± 6.7** |
| Left soleus mass [mg ± SEM] | 239.3 ± 15.5 | 233.5 ± 5.4 | 228.0 ± 7.4 | 274.9 ± 9.9** | 275.2 ± 6.8** |
| T-test: #p<0.05, ##p<0.01, ###p<0.001, ####p<0.0001 compared to MI-Vehicle. ANOVA: *p<0.05, **p<0.01, ***p<0.001, ****p<0.0001 compared to MI-Vehicle. Abbreviations: CO; cardiac output, EF; ejection fraction, FS; fractional shortening, IVRT; isovolumic relaxation time, LVID(d/s); left ventricular internal diameter (end-diastole, end-systole), LVPW(d/s); Left ventricular posterior wall dimension (end-diastole, end-systole), SV; stroke volume. | | | | | |

In conclusion, treatment with SEQ ID NO: 3 improved cardiac function in a rat model of myocardial infarction.

**Example 16: Subchronic IRI in mice**

Results from treatment with SEQ ID NO: 3 in uIRI model in mice can be seen in Table 23. uIRI induction increased serum levels of creatinine, and kidney tubular injury (KIM-1), inflammation (F4/80), and fibrosis (Col1a1) when compared to sham operated animals. Treatment with SEQ ID NO: 3 (100 nmol/kg) reduced serum levels of creatinine and kidney fibrosis and inflammation when comparing to uIRI animals treated with vehicle.

**Table 23**

| | Sham - Vehicle | uIRI - Vehicle | uIRI - SEQ ID NO: 3 | |
|---|---|---|---|---|
| Dose [nmol/kg] | NA | NA | 30 | 100 |
| BW at termination [g ± SEM] | 22.3 ± 0.5 | 22.9 ± 0.5 | 24.4 ± 0.5 | 21.8 ± 0.4 |
| Kidney weight at termination [g ± SEM] | 0.14 ± 0.005* | 0.17 ± 0.008 | 0.16 ± 0.005 | 0.16 ± 0.006 |
| Serum creatinine [mg/dl] | 0.17 ± 0.001*** | 1.97 ± 0.06 | 1.83 ± 0.004 | 1.17 ± 0.13*** |
| Serum urea [mg/dl] | 138 ± 2.8*** | 484.0 ± 11.6 | 463.0 ± 10.5 | 433 ± 22.5 |
| Renal fibrosis, Col1a1 [fractional area %] | 7.41 ± 0.6*** | 37.7 ± 2.2 | 34.3 ± 2.3 | 26.4 ± 1.4*** |
| Renal inflammation, F4/80 [fractional area %] | 0.05 ± 0.6*** | 6.6 ± 0.6 | 7.3 ± 0.6 | 2.5 ± 0.6*** |
| Renal injury, KIM-1 [fractional area %] | 0.0425 ± 0.04*** | 7.47 ± 0.51 | 7.48 ± 0.49 | 6.00 ± 0.50 |
| Dunnett's test one-factor linear model compared to DIO Vehicle: *: p < 0.05, **: p < 0.01, ***: p < 0.001 | | | | |

In conclusion, treatment with SEQ ID NO: 3 improved evaluated parameters of kidney function in a mouse model of uIRI.

ITEMS

1. A polypeptide or a pharmaceutically acceptable salt thereof comprising the structure of Formula (I)

$X^4$-V-L-S-L-D-V-P-1-G-L-L-Q-I-L-L-$X^{20}$-$X^{21}$-A-R-A-R-A-A-R-E-Q-A-$X^{32}$-$X^{33}$-N-$X^{35}$-E-I-$X^{38}$-A-E-$X^{41}$-$NH_2$ (I)

wherein

$X^4$ is selected as I, MeI or P;
$X^{20}$ is selected as E, A, F, G, H, I, K, L, N, Q, R, S, T, or V;
$X^{21}$ is selected as Q or L;
$X^{32}$ is selected as T or K;
$X^{33}$ is selected as T, E, K, V, Y, W, S, P, F, L, I, H, G, Q, A or Aib;
$X^{35}$ is selected as A, E, W, T, S, F, K, L, H, G, Q, D, N, R, Aib, L, I, or V;
$X^{38}$ is selected as L or Nle;
$X^{41}$ is V or I;
and wherein only one of $X^{32}$ or $X^{33}$ is selected as K, and wherein the K is lipidated, optionally through a linker/spacer and further wherein the hCRHR1-$EC_{50}$/hCRHR2-$EC_{50}$ ratio is at least 1000.

2. The polypeptide or a pharmaceutically acceptable salt thereof according to item 1, wherein $X^{33}$ is selected as T, E, K, or Aib; $X^{35}$ is selected as A, Aib, L, I, or V, and wherein $X^{35}$ is selected as Aib, L, I, or V if $X^{33}$ is T, or wherein $X^{33}$ is selected as E, K, or Aib, wherein the K is lipidated, optionally through a linker/spacer if $X^{35}$ is A.

3. The polypeptide or a pharmaceutically acceptable salt thereof according to any one of the preceding items, wherein $X^{38}$ is selected as Nle.

4. The polypeptide or a pharmaceutically acceptable salt thereof according to item 3, wherein $X^{20}$ is selected as A, F, G, H, I, K, L, N, Q, R, S, T, or V.

5. The polypeptide or a pharmaceutically acceptable salt thereof according to item 3, wherein $X^{20}$ is selected as N, F, G, K, Q, S, or T.

6. The polypeptide or a pharmaceutically acceptable salt thereof according to item 3, wherein $X^{20}$ is selected as S.

7. The polypeptide or a pharmaceutically acceptable salt thereof according to any one of the items 1-3, wherein $X^{20}$ is selected as S or E.

8. The polypeptide or a pharmaceutically acceptable salt thereof according to any one of the preceding items, wherein $X^{21}$ is selected as Q.

9. The polypeptide or a pharmaceutically acceptable salt thereof according to any one of the preceding items, wherein $X^{33}$ is selected T.

10. The polypeptide or a pharmaceutically acceptable salt thereof according to according to any one of the preceding items, wherein $X^{41}$ is selected as V.

11. The polypeptide or a pharmaceutically acceptable salt thereof according to any one of the preceding items, wherein $X^4$ is selected as MeI or P.

12. The polypeptide or a pharmaceutically acceptable salt thereof according to item 1, comprising the structure of Formula (I)

$X^4$-V-L-S-L-D-V-P-I-G-L-L-Q-I-L-L-$X^{20}$-Q-A-R-A-R-A-A-R-E-Q-A-[K*]-$X^{33}$-N-$X^{35}$-E-I-[Nle]-A-E-V-$NH_2$ (Ia)

wherein $X^4$ is selected as MeI or P; $X^{20}$ is selected as N, F, G, K, Q, S, or T, most preferably $X^{20}$ is selected as S; $X^{33}$ is selected as T, or Aib; $X^{35}$ is selected as A or Aib; and further wherein $X^{35}$ is selected as Aib if $X^{33}$ is T, or wherein $X^{33}$ is selected as Aib if $X^{35}$ is A; * denotes a covalent attachment of a lipid, optionally through a linker/spacer, to the ε-amino group of the lysine side-chain.

13. The polypeptide or a pharmaceutically acceptable salt thereof according to item 1, wherein the polypeptide comprises the sequence selected from the list consisting of [MeI]VLSLDVPIGLLQILLSQARARAAREQA[K*]TN[Aib]EI[Nle]AEV($NH_2$) (SEQ ID NO: 215), PVLSLDVPIGLLQILLSQARARAAREQA[K*][Aib]NAEI[Nle]AEV($NH_2$) (SEQ ID NO: 238), [MeI]VLSLDVPIGLLQILLEQARARAAREQAT[K*]NAEILAEV($NH_2$) (SEQ ID NO: 6), or [MeI]VLSLDVPIGLLQILLEQARARAAREQA[K*]TN[Aib]EILAEV($NH_2$) (SEQ ID NO: 16), wherein the * denotes a covalent attachment of a lipid, optionally through a linker/spacer, to the ε-amino group of the lysine side-chain.

14. The polypeptide or a pharmaceutically acceptable salt thereof according to any one of the preceding items, wherein the hCRHR1-$EC_{50}$/hCRHR2-$EC_{50}$ ratio is at least 2000.

15. The polypeptide or a pharmaceutically acceptable salt thereof according to any one of the preceding items, for use as a medicament in the treatment of a metabolic disorder, preferably obesity.

16. A pharmaceutical composition comprising a polypeptide or a pharmaceutically acceptable salt thereof according to any one of the items 1-14.

## Claims

**1.** A polypeptide or a pharmaceutically acceptable salt thereof, wherein the polypeptide comprises the amino acid sequence [MeI]VLSLDVPIGLLQILLSQARARAAREQA[K*]TN[Aib]EI[Nle]AEV($NH_2$) (SEQ ID NO: 215), wherein the * denotes a covalent attachment of a lipid, optionally through a linker/spacer, to the ε-amino group of the lysine side-chain.

**2.** The polypeptide or a pharmaceutically acceptable salt thereof according to claim 1, wherein the lipid and linker/spacer is C20DA[γE][γE][OEG][OEG]-.

3. The polypeptide or a pharmaceutically acceptable salt thereof according to claim 1, wherein the polypeptide consists of the amino acid sequence [MeI]VLSLDVPIGLLQILLSQARARAAREQA[K*]TN[Aib]EI[Nle]AEV($NH_2$) (SEQ ID NO: 215), wherein the * denotes a covalent attachment of a lipid, to the ε-amino group of the lysine sidechain, wherein the lipid and linker/spacer is C20DA[γE][γE][OEG][OEG]-.

4. The polypeptide or a pharmaceutically acceptable salt thereof according to any one of claims 1-3, for use as a medicament in the treatment of a metabolic disorder.

5. The polypeptide or a pharmaceutically acceptable salt thereof according to claim 4, wherein the metabolic disorder is obesity.

6. A pharmaceutical composition comprising a polypeptide or a pharmaceutically acceptable salt thereof according to any one of claims 1-3.

P21
P22
P23
P24
P25
P26
P27
P28
P33
P35
P36
P37
P39
P40
P41
Delta mean SHAP value
0.25
0.00
-0.25
-0.50
E
hCRHR1
hCRHR2

FIG. 1

SEQ ID NO: 228
hCRHR2 pEC50 [M]
hCRHR1 pEC50 [M]

**FIG. 2A**

Selectivity ratio [CRHR1 EC50/CRHR2 EC50]
Lipidation position
None

**FIG. 2B**

Delta mean SHAP value
1.0
0.5
0.0
-0.5
hCRHR1
hCRHR2
P6
hAla
hSer
S
Sar
T
P8
F
Nle
V
P10
Aib
Chg
Cle
F
hAla
L
Nle
P11
c4FPro
c4NHPro
Hyptrans
P12
4FPhe
Chg
dF
F
hPhe
L
Nle
S
Y
P20
N
S
T
P21
I
L
Nle
P22
E
P23
K
P24
Q
T
P25
E
K
Q
P26
I
K
L
N
Q
S
V
P27
E
I
L
Q
P29
Q
P30
E
R
P38
F
Nle
P41
I


**FIG. 3**

P20
P33
P35
P38
Delta mean SHAP value
0.0
-0.2
-0.4
-0.6
A
D
F
G
H
I
K
L
N
P
Q
R
S
T
V
W
Y
Aib
Aib
Nle
hCRHR1
hCRHR2

**FIG. 4**

105
100
95
90
85
80
75
Body weight (%)
1
6
11
16
21
26
31
Study day
*
***
***
***
Vehicle
SEQ ID NO: 16 10 nmol/kg
SEQ ID NO: 16 30 nmol/kg
SEQ ID NO: 215 10 nmol/kg
SEQ ID NO: 215 30 nmol/kg

**FIG. 5**

1. dose
2. dose
3. dose
4. dose
5. dose
6. dose
7. dose
MAP (mmHg)
140
130
120
110
100
90
80
70
60
50
0
24
48
72
96
120
144
168
Time (hours)
Placebo
SEQ ID NO: 3
10 nmol/kg
SEQ ID NO: 3
30 nmol/kg
SEQ ID NO: 3
100 nmol/kg

**FIG. 6**

1. dose
2. dose
3. dose
4. dose
5. dose
6. dose
7. dose
Heart rate (BPM)
800
750
700
650
600
550
500
450
400
350
300
0
24
48
72
96
120
144
168
Time (hours)
Placebo
SEQ ID NO: 3 10 nmol/kg
SEQ ID NO: 3 30 nmol/kg
SEQ ID NO: 3 100 nmol/kg

FIG. 7

A
Mean Arterial Blood Pressure
MAP (mmHg)
180
160
140
120
100
80
-1
0
1
2
3
4
5
6
7
B
Heart Rate
HR (bpm)
450
400
350
300
250
200
baseline
s.c. application OD at 9h for 7 days
wash out
(days)
Placebo
SEQ ID NO: 3, 10 nmol/kg
SEQ ID NO: 3, 30 nmol/kg

**FIG. 8**

Heart rate
HR [bpm]
200
160
120
80
40
Placebo
0.03 mg/kg
0.1 mg/kg
0.3 mg/kg
1.0 mg/kg
-20
application
20
30
40
50
60
blood
90
100
110
120
130
rel time [hours]

**FIG. 9A**

Mean blood pressure
MBP [mmHg]
200
180
160
140
120
100
80
60
Placebo
0.03 mg/kg
0.1 mg/kg
0.3 mg/kg
1.0 mg/kg
-20
application
20
30
40
50
60
blood
90
100
110
120
130
rel time [hours]

**FIG. 9B**

Contractility
+dP/dt LVP [mmHg/s]
8000
7000
6000
5000
4000
3000
2000
1000
Placebo
0.03 mg/kg
0.1 mg/kg
0.3 mg/kg
1.0 mg/kg
-20
application
20
30
40
50
60
blood
90
100
110
120
130
rel. time [hours]

**FIG. 9C**

Relaxation
-dP/dt LVP [mmHg/s]
5000
4000
3000
2000
1000
Placebo
0.03 mg/kg
0.1 mg/kg
0.3 mg/kg
1.0 mg/kg
-20
application
20
30
40
50
60
blood
90
100
110
120
130
rel time [hours]

**FIG. 9D**

ECG, PR-Interval
200
180
160
140
120
100
80
60
PR-I [ms]
Placebo
0.03 mg/kg
0.1 mg/kg
0.3 mg/kg
1.0 mg/kg
-20
application
20
30
40
50
60
blood
90
100
110
120
130
rel time [hours]

**FIG. 9E**

ECG, QT-Interval
500
450
400
350
300
250
200
QT-I [ms]
Placebo
0.03 mg/kg
0.1 mg/kg
0.3 mg/kg
1.0 mg/kg
-20
application
20
30
40
50
60
blood
90
100
110
120
130
rel time [hours]

**FIG. 9F**

Heart rate
Placebo
0.3 mg/kg
HR ECG [bpm]
200
160
120
80
40
0
application
4
6
8
10
12
14
16
18
20
22
24
26
28
rel days

**FIG. 10A**

Mean blood pressure
Placebo
0.3 mg/kg
MBP [mmHg]
200
175
150
125
100
75
50
application
4
6
8
10
12
14
16
18
20
22
24
26
28
rel days

**FIG. 10B**

Contractility
8000
6000
4000
2000
0
+dP/dt LVP [mmHg/s]
Placebo
0.3 mg/kg
application
4
6
8
10
12
14
16
18
20
22
24
26
28
rel days

**FIG. 10C**

Relaxation
6000
5000
4000
3000
2000
1000
0
-dP/dt LVP [mmHg/s]
Placebo
0.3 mg/kg
application
4
6
8
10
12
14
16
18
20
22
24
26
28
rel days

**FIG. 10D**

ECG, PR-Interval
Placebo
0.3 mg/kg
PR-I [ms]
160
150
140
130
120
110
100
90
application
4
6
8
10
12
14
16
18
20
22
24
26
28
rel days

**FIG. 10E**

ECG, QT-Interval
Placebo
0.3 mg/kg
QT-I [ms]
500
400
300
200
100
application
4
6
8
10
12
14
16
18
20
22
24
26
28
rel days

**FIG. 10F**

Contractility
[Data are mean of n=4/6/12 +/- SEM]
15000
10000
5000
0
+dP/dt [mmHg/s]
Control
Vehicle
Dose 1
Dose 2
Dose 3
Dose 4
Vehicle
Dobutamine
SEQ ID NO: 215
0
30
60
90
120
150
180
BGA/PV-loop
BGA/PV-loop
BGA/PV-loop
BGA/PV-loop
BGA/PV-loop
BGA/PV-loop
t [min]


FIG. 11A

Coronary blood flow
[Data are mean of n=1/6/6 +/- SEM]
90
60
30
0
CBF [ml/min]
Control
Vehicle
Dose 1
Dose 2
Dose 3
Dose 4
Vehicle
Dobutamine
SEQ ID NO: 215
0
30
60
90
120
150
180
BGA/PV-loop
BGA/PV-loop
BGA/PV-loop
BGA/PV-loop
BGA/PV-loop
BGA/PV-loop
t [min]


FIG. 11B

Rate pressure product
[Data are mean of n=1/6/10 +/- SEM]
Control
Vehicle
Dose 1
Dose 2
Dose 3
Dose 4
Vehicle
Dobutamine
SEQ ID NO: 215
RPP [mmHg x bpm]
50000
40000
30000
20000
10000
0
0
30
60
90
120
150
180
BGA/PV-loop
t [min]

FIG. 11C

Rate pressure product versus Contractility
RPP [bpm x mmHg]
15000
12500
10000
7500
5000
1000
2000
3000
4000
5000
6000
+dP/dt [mmHg/s]
Vehicle
Dobutamine
SEQ ID NO: 215

FIG. 11D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2022038179 A
- WO 2023285334 A
- WO 2018013803 A
- WO 2023285347 A
- WO 2018013803 A1
- WO 2023285334 A1
- WO 2022038179 A1


### Non-patent literature cited in the description


- Remington's Pharmaceutical Sciences
- **BREIMAN, L.** Random Forests. *Machine Learning*, 2001, vol. 45 (1), 5-32
- **LUNDBERG, S. M.** ; **LEE, S. I.** A unified approach to interpreting model predictions. *Advances in neural information processing systems*, 2017, 30